# EUROPEAN PATENT APPLICATION

(11) **EP 4 653 439 A1**
(43) Date of publication of application: **26.11.2025**
(21) Application number: 24744321.1
(22) Date of filing: 17.01.2024
(51) Int. Cl.: C07D 487/04, A61K 31/519, A61P 27/06, A61P 35/00, A61P 11/06, A61P 5/48, A61P 13/12, A61P 19/10, A61P 25/28

(54) **AZACYCLOALKANE COMPOUND AND PHARMACEUTICAL COMPOSITION, AND USE THEREOF**

(30) Priority: 18.01.2023 CN 202310086563
(71) Applicant: Prime Gene Therapeutics Co., Ltd., Beijing 100070 (CN)
(72) Inventor: ZHU, Li, Beijing 100070 (CN); HU, Wei, Beijing 100070 (CN); ZHANG, Hui, Beijing 100070 (CN); DU, Daniel Yunlong, Beijing 100070 (CN)
(74) Representative: Winter, Brandl - Partnerschaft mbB
(86) International application number: PCT/CN2024/072856
(87) International publication number: WO 2024/153147

(57) **Abstract**

The present application relates to an azacycloalkane compound, a pharmaceutical composition, and use thereof. The compound has a structural formula as shown in formula (I). The azacycloalkane compound and the pharmaceutical composition thereof provided in the present application have significant Rho kinase inhibitory activity, the enzymatic activity and cell activity of the azacycloalkane compound and the pharmaceutical composition thereof are better than those of existing ROCK inhibitors such as Ripasudil, Netarsudil, and Belumosudil, and thus the azacycloalkane compound and the pharmaceutical composition thereof have great potential for application.

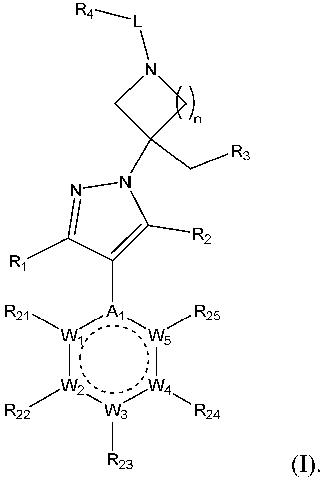

## Description

### Technical Field

The present disclosure provides an azacycloalkane compound, a stereoisomer and a pharmaceutical composition comprising the compound, and use of the compound and the pharmaceutical composition. The azacycloalkane compound is an inhibitor targeting ROCK that can be used to regulate Rho kinase mediated diseases.

### Background

Rho kinase/ROCK (Rho associated kinase) signaling pathway induces cytoskeleton reorganization, cell migration, adhesion and stress fiber formation, which are related to various physiological functions. ROCK family includes ROCK1 and ROCK2. ROCK1 is highly expressed in lung, liver, spleen, kidney and testis, while ROCK2 is highly expressed in brain and heart. ROCK1 indirectly acts on an epithelial cadherin compound by binding to the scaffold protein P120 catenin of E-cadherin; ROCK1 can be concentrated in the microtubule tissue center and the pseudopodia edge of motor cells, which is related to cell migration. ROCK2 is mainly concentrated in cytoplasm and is located on plasma membrane through C-terminal region, which is related to vimentin and actin tension fibers.

ROCK mediates many pathological and physiological signals, which are related to various physiological functions such as endothelial permeability, tissue contraction, and growth. ROCK inhibitors have potential applications in diseases comprsing asthma, cancer, glaucoma, insulin resistance, kidney failure, neuronal degeneration, and osteoporosis. Two ROCK1/ROCK2 inhibitors Ripasudil and Netarsudil eye drops have been approved for the treatment of glaucoma, and one ROCK2 inhibitor belumosudil has been approved for the treatment of chronic graft-versus-host disease (cGVHD). However, the existing ROCK inhibitors on the market have weak activity, and the function and clinical application value of ROCK have not been fully developed. Therefore, it is urgent to develop a pharmaceutical targeting ROCK to address the unmet clinical needs.

### Summary of the Invention

The present application developes a highly active azacycloalkane compound targeting ROCK with a novel framework and a pharmaceutical composition thereof for regulating Rho kinase mediated diseases.

The present disclosure provides an azacycloalkane compound shown in formula I, or pharmaceutically acceptable salts, solvates, active metabolites, polymorphs, isotopic markers, isomers or prodrugs thereof:

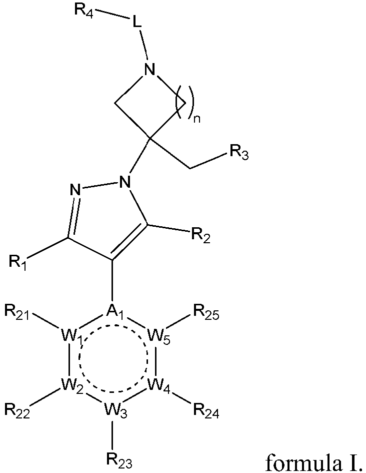

Another aspect of the present disclosure is to provide a method for manufacturing the compound as shown in formula I, isotope isomers or pharmaceutically acceptable salts, solvates, active metabolites, polymorphs, isotopic markers, isomers or prodrugs thereof, and use thereof for treating Rho kinase mediated diseases.

### Brief Description of the Drawings

Figure 1 shows the weight changes of animals in each group during the experimental period;
Figure 2 shows the 24-hour intraocular pressure change curves of animals in each group at different times after D1 administration; and
Figure 3 shows the results of RGCs of animals in each group at the endpoint of the experiment.

### Detailed Description

The present application will be further explained below in detail through embodiments. Through these descriptions, the features and advantages of the present application will become more clearly defined.

The term "exemplary" used here means "serving as an example, embodiment, or illustration." Any embodiment described here as "exemplary" should not be interpreted as being superior or better than other embodiments.

Furthermore, the technical features in different embodiments of the present application described below can be combined with each other if they do not conflict.

The following embodiments are provided to illustrate the purpose and should not limit the present disclosure in any way. One of skill in the art should easily recognize that various noncritical parameters can be changed or modified to obtain essentially the same results.

### Definitions

Unless defined otherwise, all technical and scientific terms used herein have the same meaning as that commonly understood by one of skill in the art to which the claimed subject matter belongs. All patents, patent applications, published materials referred to throughout the entire disclosure herein, unless noted otherwise, are incorporated by reference in their entirety. When a trade name appears herein, it is intended to refer to its corresponding product or its active ingredient.

It is to be understood that the foregoing general description and the following detailed description are exemplary and explanatory only, and are not restrictive of any subject matter claimed. In the present application, it must be noted that unless otherwise explicitly stated in the text, the singular used in the present description and claims includes the plural of the referred object. It should also be noted that "or" used herein means "and/or" unless stated otherwise. Furthermore, the term "comprise(s)" used herein as well as other forms, such as "comprising", "contain(s)", and "include(s)" is not limiting.

Definitions of standard chemical terms may be found in the literature, including Carey and Sundberg's "Advanced Organic Chemistry 4th Ed, Vol A (2000) and B (2001), Plenum Press, New York. Unless otherwise specified, conventional methods within the technical field are applied, such as mass spectrometry, NMR, HPLC, protein chemistry, biochemistry, recombinant DNA technology, and pharmacological methods. Unless specific definitions are provided, those skilled in the art know the related terms and laboratory operations and techniques in analytical chemistry, synthetic organic chemistry, and medical and pharmaceutical chemistry used herein. Standard techniques may be used for chemical synthesis, chemical analysis, drug preparation, formulation, drug delivery and patient treatment. Standard techniques may be used for recombinant DNA, oligonucleotide synthesis, and tissue culture and transformation (such as electroporation, lipid infection). For example, a kit with instructions provided by the manufacturer may be used, or the reaction and purification techniques may be carried out according to methods known in the art, or according to the method described in the present disclosure. Generally speaking, the aforementioned techniques and steps may be implemented by conventional methods well-known in the art and described in various general documents or more specific documents. These documents are described and cited in the present disclosure.

When a substituent is described by a conventional chemical formula written from left to right, the substituent also includes chemically equivalent substituents obtained when the structural formula is written from right to left. For example, CH₂O is equivalent to OCH₂.

The term "substituted or unsubstituted" comprsies two situations, i.e. "substituted" and "unsubstituted", wherein "substituted" means that any one or more hydrogen atoms on a specific atom are replaced by a substituent, as long as the valence of the specific atom is normal and the compound after substitution is stable; "unsubstituted" means that the hydrogen atom on a specific atom has not been replaced by a substituent. For example, "substituted or unsubstituted ethyl" (such as in the case of halogen substituents) comprsies unsubstituted (-CH₂CH₃), monosubstituted (such as -CH₂CH₂F), multi-substituted (such as -CHFCH₂F, -CH₂CHF₂, etc.), or completely substituted (-CF₂CF₃). Those skilled in the art can understand that for any group containing one or more substituents, no substitution or substitution pattern that is spatially impossible and/or cannot be synthesized will be introduced. When the substituent is oxo (i.e. =O), it means that two hydrogen atoms on the same atom are replaced.

When any variable (such as R) occurs more than once in the composition or structure of a compound, its definition in each case is independent. Thus, for example, if a group is substituted with 0-2 R, the group may optionally be substituted with up to two R, and R has independent options in each case. In addition, combinations of substituents and/or variants thereof are only permitted if such combinations result in stable compounds. The term "optional" or "optionally" means that the subsequently described event or circumstance may or may not occur, and that the description includes instances wherein said event or circumstance occurs and instances in which it does not.

As used herein, C_{m~n} refers to the part having m~n carbon atoms. For example, the "C_{1~8}" group means that the part has 1-8 carbon atoms, that is, the group contains 1 carbon atom, 2 carbon atoms, 3 carbon atoms... 8 carbon atoms. Therefore, for example, "C_{1~8} alkyl" refers to an alkyl containing 1-8 carbon atoms, that is, the alkyl is selected from the group consisting of methyl, ethyl, propyl, isopropyl, n-butyl, isobutyl, sec-butyl, tert-butyl... octyl, etc. Numerical ranges herein, for example "1-8" refers to each integer within the given range. For example, "1-8 carbon atoms" means that the group may have 1 carbon atom, 2 carbon atoms, 3 carbon atoms, 4 carbon atoms, 5 carbon atoms, 6 carbon atoms, 7 carbon atoms, or 8 carbon atoms.

The term "alkyl" refers to an optionally substituted straight-chain or optionally substituted branched-chain saturated aliphatic hydrocarbon radical, which is connected to the rest of the molecule through a single bond. The "alkyl" herein may have from 1 to about 8 carbon atoms, for example, from 1 to 6 carbon atoms, or from 1 to 4 carbon atoms, or from 1 to 3 carbon atoms. Examples of the alkyl herein include but not limited to methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, n-butyl, isobutyl, sec-butyl, tert-butyl, n-pentyl, isopentyl, neopentyl, tert-amyl, hexyl, etc, and longer alkyl, such as heptyl, octyl and the like. The group defined herein, when the number range of "alkyl" appears, for example, "C₁₋₈ alkyl" refers to the alkyl which consists of 1 carbon atom, 2 carbon atoms, 3 carbon atoms, 4 carbon atoms, 5 carbon atoms, 6 carbon atoms, 7 carbon atoms, or 8 carbon atoms. As another example, "C₁₋₄ alkyl" refers to the alkyl which consists of 1 carbon atom, 2 carbon atoms, 3 carbon atoms, or 4 carbon atoms. The alkyl herein also includes cases where the numerical range is not specified.

The term "alkenyl" refers to an optionally substituted straight-chain or optionally substituted branched-chain monovalent hydrocarbon group which contains at least one carbon-carbon double bond. The alkylene has, but not limited to, from 2 to about 8 carbon atoms, for example, from 2 to about 6 carbon atoms, or from 2 to about 4 carbon atoms. The group may be in either cis or trans conformation about the double bond(s), and should be understood to include both isomers. Examples of alkylene include, but are not limited to ethenyl (CH=CH₂), 1-propenyl (CH₂CH=CH₂), isopropenyl (C(CH₃)=CH₂), butenyl, 1,3-butadienyl and the like. When the number range of the alkenyl defined herein appears, for example, "C₂₋₈ alkenyl" refers to the alkenyl which may consist of 2 carbon atoms, 3 carbon atoms, 4 carbon atoms, 5 carbon atoms, 6 carbon atoms, 7 carbon atoms, or 8 carbon atoms. The alkenyl herein also includes cases where the numerical range is not specified.

The term "alkynyl" refers to an optionally substituted straight- or branched-chain monovalent hydrocarbon group which has at least one carbon-carbon triple-bond. The alkynyl has from 2 to about 8 carbon atoms, for example, from 2 to about 6 carbon atoms, or from 2 to about 4 carbon atoms. Examples of the alkynyl herein include, but are not limited to ethynyl, 2-propynyl, 2-butynyl, 1,3-butadiynyl, and the like. When the number range of the alkynyl defined herein appears, for example, "C₂₋₈ alkynyl" refers to the alkynyl which may consist of 2 carbon atoms, 3 carbon atoms, 4 carbon atoms, 5 carbon atoms, 6 carbon atoms, 7 carbon atoms, or 8 carbon atoms. The alkynyl herein also includes cases where the numerical range is not specified.

The term "cycloalkyl" refers to non-aromatic carbon containing rings, which comprise saturated carbon rings (such as cycloalkyl) or unsaturated carbon rings (such as cycloalkenyl). Carbocyclic ring comprsies mono-carbocyclic ring (having one ring), such as monocyclic cycloalkyl; bi-carbocyclic ring (having two rings), such as bicyclic cycloalkyl; poly-carbocyclic ring (having two or more rings). The rings can be bridged or sprio rings. Carbocyclic ring (such as cycloalkyl or cycloalkenyl) can have from 3 to 8 carbon atoms, such as from 3 to about 6 ring-forming carbon atoms, or form 3 to about 5 ring-forming carbon atoms.

The term "aryl" refers to an optionally substituted aromatic hydrocarbon group which has 6 to about 20, for example, 6 to 12 or 6 to 10 ring-forming carbon atoms, which may be monocyclic aryl, bicyclic aryl or polycyclic aryl. The bicyclic aryl or polycyclic aryl may be fused through a monocyclic aryl with other independent rings, such as alicyclic, heterocyclic, aromatic, heteroaromatic rings. Non-limiting examples of the monocyclic aryl group comprise monocyclic aryl having from 6 to about 12, from 6 to about 10, or from 6 to about 8 ring-forming carbon atoms, for example, phenyl; bicyclic aryl, for example, naphthyl; and polycyclic aryl, for example, phenanthrenyl, anthracenyl, and azulenyl.

The term "heteroaryl" refers to an optionally substituted heteroaryl containing from about 5 to about 20, for example, from 5 to 12, or from 5 to 10 skeletal ring-forming atoms, wherein at least one (for example, 1 to 4, 1 to 3, or 1 to 2) ring-forming atom is a heteroatom which is independently selected from the group consisting of oxygen, nitrogen, sulfur, phosphorous, silicon, selenium and tin, but not limited to these atoms. Heteroaryl comprises monocyclic heteroaryl (containing one ring), bicyclic heteroaryl (containing two rings) or polycyclic heteroaryl (containg more than two rings). In embodiments in which two or more heteroatoms are present in the ring, the two or more heteroatoms can be identical to each another, or some or all of the two or more heteroatoms can each be different from the others. The bicyclic heteroaryl or polycyclic heteroaryl may be fused through a monocyclic heteroaryl with other independent rings such as alicyclic, heterocyclic, aromatic, heteroaromatic rings (which may be collectively known as fused ring heteroaryl). Non-limiting examples of heteroaryl comprise but are not limited to pyrrolyl, furyl, thienyl, imidazolyl, oxazolyl, pyrazolyl, pyridinyl, pyrimidyl, pyrazinyl, quinolyl, isoquinolyl, tetrazolyl, triazolyl, triazinyl, benzofuranyl, benzothienyl, indolyl, isoindolyl, and the like.

The term "heterocyclyl" refers to a non-aromatic heterocycle, which comprises a saturated heterocycle or an unsaturated heterocycle (containing an unsaturated bond), does not have a completed conjugated π-electron system and can be divided into non-aromatic monocyclic, fused polycyclic, bridged or spirocyclic systems, wherein one or more (e.g., 1 to 4, 1 to 3, or 1 to 2) ring-forming atoms are heteroatoms such as oxygen, nitrogen or sulfur atoms. The heterocycle may comprise mono-heterocycle (containing one ring) or bis-heterocycle (containing two bridged rings) or poly-heterocycle (containing more than two bridged rings); and also comprise sprio rings. Heterocyclyl may have from 3 to about 20, for example, from 3 to about 10, from 3 to about 8, from 4 to 8, from 4 to 7, from 5 to about 8, or from 5 to about 6 ring atoms. Non-limiting examples of "heterocyclyl" comprise oxiranyl, thiiranyl, aziranyl, azetidinyl, oxetanyl, thietanyl, tetrahydrofuranyl, pyrrolidinyl, oxazolidinyl, tetrahydropyrazolyl, pyrrolinyl, dihydrofuranyl, dihydrothienyl, piperidinyl, tetrahydropyranyl, tetrahydrothiapyranyl, morpholinyl, piperazinyl, dihydropyridyl, tetrahydropyridyl, dihydropyranyl, tetrahydropyranyl, dihydrothiapyranyl, azacycloheptyl, oxacycloheptyl, thiacycloheptyl, oxa-azabicyclo[2.2.1]heptyl, azaspiro[3.3]heptyl, and the like.

The term "halo" or "halogen" refers to an optionally substituted group (such as alkyl, alkenyl, alkynyl, etc.), in which at least one hydrogen atom is replaced with a halogen atom (such as fluorine, chlorine, bromine, iodine, or a combination thereof). In some embodiments, two or more hydrogen atoms are replaced with halogen atoms that are identical to each other (e.g., difluoromethyl, trifluoromethyl); in other embodiments, two or more hydrogens are replaced with halogen atoms that are not completely identical to each other (e.g., 1-chloro-1-fluoro-1-iodo-ethyl).

The term "alkoxy" refers to an alkyl ether group (O-alkyl). Non-limiting examples of alkoxy radicals comprise methoxy, ethoxy, n-propoxy, isopropoxy, n-butoxy, isobutoxy, sec-butoxy, tert-butoxy and the like.

The term "alkylacyl" refers to a group formed by connecting an alkyl with -CO-, and non-limiting examples thereof include formyl, acetyl, propionyl, butyryl, and the like. For example, the term "C₁₋₆ alkylacyl" refers to a group formed by connecting C₁₋₆ alkyl with -CO-. As another example, the term "C₁₋₄ alkylacyl" refers to a group formed by connecting C₁₋₄ alkyl with -CO-.

The term "alkylsulfonyl" refers to a group formed by connecting an alkyl with -SO₂-, and non-limiting examples thereof comprise methylsulfonyl, ethylsulfonyl, propylsulfonyl, butylsulfonyl, and the like. For example, the term "C₁₋₆ alkylsulfonyl" refers to a group formed by connecting C₁₋₆ alkyl with -SO₂-. As another example, the term " C₁₋₄ alkylsulfonyl" refers to a group formed by connecting C₁₋₄ alkyl with -SO₂-.

The term "heteroarylacyl" refers to a group formed by connecting a heteroaryl with-CO-. For example, the term "C₅₋₂₀ heteroarylacyl" refers to a group formed by connecting C₅₋₂₀ heteroaryl with -CO-. The "heteroaryl" and "C₅₋₂₀ heteroaryl" are defined as above.

Other terms for group herein comprise: "hydroxyl" refers to -OH group, "thiol" refers to - SH group, "cyano" refers to -CN group, and "carboxyl" refers to -COOH group.

The term "member" refers to the number of skeletal atoms constituting the ring. For example, pyridine is a six-membered ring and pyrrole is a five-membered ring.

The term "pharmaceutically acceptable" refers to those compounds, materials, compositions and/or dosage forms that are within the scope of reliable medical judgment and are suitable for use in contact with human and animal tissues without excessive toxicity, irritation, allergic reactions or other problems or complications of the disease, and are commensurate with a reasonable benefit/risk ratio.

The term "pharmaceutical composition" refers to a biologically active compound optionally mixed with at least one pharmaceutically acceptable chemical component or agent. The pharmaceutically acceptable chemical component or agent is the "carrier", which helps for introducing the compound into cells or tissues. It comprises, but is not limited to, stabilizers, diluents, suspending agents, thickeners, and/or excipients.

The term "pharmaceutically acceptable salt" refers to a salt that retains the biological efficacy of the free acid and free base of the specified compound and has no adverse effects in biology or other aspects. Unless otherwise specified, the salts in the present disclosure may comprise metal salts, ammonium salts, salts formed with organic bases, salts formed with inorganic acids, salts formed with organic acids, salts formed with basic or acidic amino acids, etc. Non-limiting examples of metal salts comprise, but are not limited to, alkali metal salts, such as sodium salt, potassium salt, etc.; alkaline earth metal salts, such as calcium salt, magnesium salt, barium salt, etc.; aluminum salt, and the like. Non-limiting examples of salts formed with organic bases comprise, but are not limited to, the salts formed with trimethylamine, triethylamine, pyridine, picoline, 2,6-lutidine, ethanolamine, diethanolamine, triethanolamine, cyclohexylamine, dicyclohexylamine and the like. Non-limiting examples of salts formed with inorganic acids comprise, but are not limited to, salts formed with hydrochloric acid, hydrobromic acid, nitric acid, sulfuric acid, phosphoric acid, and the like. Non-limiting examples of salts formed with organic acids comprise, but are not limited to, salts formed with formic acid, acetic acid, trifluoroacetic acid, fumaric acid, oxalic acid, malic acid, maleic acid, tartaric acid, citric acid, succinic acid, methanesulfonic acid, benzene sulfonic acid, p-toluenesulfonic acid, etc. Non-limiting examples of salts formed with basic amino acids comprise, but are not limited to, salts formed with arginine, lysine, ornithine, and the like. Non-limiting examples of salts formed with acidic amino acids comprise, but are not limited to, salts formed with aspartic acid, glutamic acid, and the like.

Pharmaceutically acceptable salts may be synthesized from parent compounds containing acid radicals or bases by conventional chemical methods. Generally, such salts are prepared by reacting these compounds in free acid or base form with a stoichiometric amount of appropriate base or acid in water or organic solvent or a mixture of both. Generally, non-aqueous media such as ether, ethyl acetate, ethanol, isopropanol or acetonitrile are preferred.

The term "solvate" refers to a physical aggregate formed by a compound of the present imvention and one or more solvent molecules. This physical aggregate comprises varying degrees of ions and covalent bonds, such as hydrogen bonds. It has been shown that this solvate may be separated, for example, when one or more solvent molecules are mixed in the crystal lattice. "solvate" comprises both solvent phase and separable solvate. There are many examples of corresponding solvates, including ethanol solvates, methanol solvates and the like. "Hydrate" is a solvate that uses water (H₂O) molecules as a solvent. One or more compounds in the present disclosure may be prepared as solvates at will. The preparation of solvates is well known. For example, M. Caira et al, J. Pharmaceutical Sci., 93(3), 601-611 (2004 ) describe the preparation of a solvate of the antifungal drug fluconazole, that is, preparation with ethyl acetate and water. E.C. van Tonder et al, AAPS PharmSciTech., 5(1), article 12 (2004); and AL Bingham et al, Chem. Commun., 603-604 (2001) also describes the similar methods for preparing solvates and hydrates. A typical, non-limiting preparation process is to dissolve the compound of the present disclosure in an ideal solvent (organic solvent or water or their mixed solvent) at a temperature higher than normal temperature, to cool down, and to leave to crystallize. Then, the crystals are separated by using standard methods. I. R. spectroscopy analysis technique may confirm the existence of the solvent (water) that forms the solvate (hydrate) in the crystal.

The term "active metabolite" refers to an active derivative of the compound formed when the compound is metabolized.

The term "polymorphs" refers to compounds of the present disclosure that exist in different crystal lattice forms.

The term "isotopic marker" refers to an isotopically marked compound of the present disclosure. For example, the isotopes in the compound of the present disclosure may comprise various isotopes of elements such as H, C, N, O, P, F, S, such as ²H, ³H, ¹³C, ¹⁴C, ¹⁵N, ¹⁸O, ¹⁷O, ³¹P, ³²P, ³⁵S, ¹⁸F and ³⁶S.

The term "pharmaceutically acceptable prodrug" or "prodrug" refers to any pharmaceutically acceptable salt, ester, ester salt or other derivative of the compound of the present disclosure, which may directly or indirectly provide the compound of the present disclosure or its pharmaceutically active metabolite or residue after administration to a recipient. Particularly preferred derivatives or prodrugs are those compounds that may improve the bioavailability of the compounds of the present disclosure when administered to patients (for example, may make oral compounds more easily absorbed into the blood), or promote the parent compound delivering to biological organs or the site of action, such as the brain or lymphatic system. The functional groups in the compound may be modified by conventional operations or in vivo in a modification manner that may be decomposed into the parent compound to prepare a prodrug. Various prodrug forms are well known in the art. See, Pro-drugs as Novel Delivery Systems (1987) Vol. 14 of the ACS Symposium Series, Bioreversible Carriers in Drug Design, (1987) Edward B. Roche, ed., American Pharmaceutical Association by T. Higuchi and V. Stella and Pergamon Press provide discussions on prodrugs. Design of Prodrugs, Bundgaard, A. Ed., Elseview, 1985 and Method in Enzymology, Widder, K. et al., Ed.; Academic, 1985, vol. 42, p. 309-396 ; Bundgaard, H. "Design and Application of Prodrugs" in A Textbook of Drug Design and Development, Krosgaard-Larsen and H. Bundgaard, Ed., 1991, Chapter 5, pp. 113-191 ; and Bundgaard, H., Advanced Drug Delivery Review, 1992, 8, 1-38 , the above documents are incorporated herein by reference.

The term "stereoisomers" refers to isomers produced by different arrangements of atoms in the molecule in space. The compounds of the present disclosure contain structures such as asymmetric or chiral centers and double bonds. Therefore, the compounds of the present disclosure may comprise optical isomers, geometric isomers, tautomers, atropisomers and other isomers. These isomers and their single isomers, racemates, etc. are all included in the scope of the present disclosure. For example, for optical isomers, optically active (R)- and (S)-isomers and D and L isomers may be prepared by chiral resolution, chiral synthesis or chiral reagents or other conventional techniques. For example, it may be converted into diastereomers by reacting with appropriate optically active substances (such as chiral alcohols or Mosher's Mohsyl chloride), separated and converted (such as hydrolyzed) into the corresponding single isomer. As another example, it may also be separated by a chromatographic column.

The "pharmaceutical compositions" herein may be prepared in a manner well known in the pharmaceutical field, and they may be administered or applied by various routes, depending on whether local or systemic treatment is required and the area to be treated. It may be topically administered (for example, transdermal, skin, eye and mucous membranes including intranasal, vaginal and rectal delivery), pulmonarily administered (for example, by inhalation or insufflation of powder or aerosol, including through sprayers; intratracheal, intranasal), orally or parenterally administered. Parenteral administration includes intravenous, intraarterial, subcutaneous, intraperitoneal or intramuscular injection or infusion; or intracranial, such as intrathecal or intracerebroventricular administration. It may be administered parenterally in a single bolus dose, or it may be administered by, for example, a continuous infusion pump. The pharmaceutical composition herein comprises but is not limited to the following forms: tablets, pills, powders, lozenges, sachets, cachets, elixirs, suspensions, emulsions, solutions, syrups, aerosols (solid or dissolved in a liquid vehicle); for example, ointments, soft and hard gelatin capsules, suppositories, sterile injection solutions and sterile packaged powders containing up to 10% by weight of the active compound.

The pharmaceutical composition herein may be formulated in a unit dosage form, and each dosage may contain about 0.1 to 1000 mg, usually about 5 to 1000 mg of active ingredient, and more usually about 100 to 500 mg of active ingredient. The term "unit dosage form" refers to a physically separated single dosage unit suitable for use in human patients and other mammals, each unit containing a predetermined amount of active substance mixed with a suitable pharmaceutical carrier calculated to produce the desired therapeutic effect.

The term "individual" refers to an individual suffering from a disease, disorder, or condition, and comprises mammals and non-mammals. Examples of mammals comprise, but are not limited to, any member of the class Mammals: humans, non-human primates (such as chimpanzees and other apes and monkeys); domestic animals, such as cows, horses, sheep, goats, and pigs; domesticated animals, such as rabbits, dogs, and cats; laboratory animals, comprising rodents, such as rats, mice, and guinea pigs.

The term "treatment" and other similar synonyms comprise alleviation, reduction or amelioration of symptoms of a disease or condition, prevention of other symptoms, amelioration or prevention of the underlying metabolic cause of the symptoms, inhibition of the disease or condition, such as preventing the development of the disease or condition, alleviating the disease or condition, making a disease or condition better, alleviating the symptoms caused by the disease or condition, or stoping the symptoms of the disease or condition. In addition, the term may also comprise the purpose of prevention. The term also comprises obtaining therapeutic effects and/or preventive effects. The therapeutic effect refers to curing or improving the underlying disease being treated. In addition, the cure or improvement of one or more physiological symptoms associated with the underlying disease is also a therapeutic effect. For example, although the patient may still be affected by the underlying disease, an improvement in the patient's condition is observed. In terms of preventive effects, the composition or compound may be administered to patients who are at risk of suffering from a specific disease, or even if a disease diagnosis has not been made, the composition or compound may be administered to patients who have one or more physiological symptoms of the disease.

The term "amount to obtain the necessary therapeutic effect" or "therapeutically effective amount" refers to the amount of at least one agent or compound sufficient to relieve one or more symptoms of the disease or condition being treated after administration. The result may be a reduction and/or alleviation of signs, symptoms or causes, or any other desired changes in the biological system. Techniques such as dose escalation tests may be used to determine the effective amount suitable for any individual case. The actual amount of compound, pharmaceutical composition or agent to be administered is usually determined by the physician according to the relevant circumstances, comprising the condition being treated, the route of administration selected, the actual compound administered; the age, weight and response of the individual patient; and the severity of the patient's symptoms, etc.

The ratio or concentration of the compound of the present disclosure in the pharmaceutical composition may not be fixed, depending on various factors, comprising dosage, chemical properties (for example, hydrophobicity), route of administration, and the like. For example, the compound of the present disclosure may be provided by a physiologically buffered aqueous solution containing about 0.1-10% w/v of the compound for parenteral administration. Some typical dosage ranges are from about 1 µg/kg to about 1 g/kg body weight/day. In certain embodiments, the dosage range is from about 0.01 mg/kg to about 100 mg/kg body weight/day. The dosage is likely to depend on such variables, such as the type and degree of development of the disease or condition, the general health status of the specific patient, the relative biological efficacy of the selected compound, the excipient formulation and its route of administration.

The term "administration" refers to a method capable of delivering a compound or composition to a desired site for biological action. These methods comprise, but are not limited to, oral routes, transduodenal routes, parenteral injections (comprising intravenous, subcutaneous, intraperitoneal, intramuscular, intraarterial injection or infusion), topical, and rectal administration. Those skilled in the art are familiar with administration techniques that may be used for the compounds and methods described herein, for example, those discussed in Goodman and Gilman, The Pharmacological Basis of Therapeutics, currented.; Pergamon; and Remington's, Pharmaceutical Sciences (current edition), Mack Publishing Co., Easton, Pa.

The term "IC50" refers to 50% inhibition of the maximum effect obtained in an analysis measuring such an effect.

### Compounds

On the one hand, the present application provides an azacycloalkane compound shown in formula I, or pharmaceutically acceptable salts, solvates, active metabolites, polymorphs, isotopic markers, isomers or prodrugs thereof: 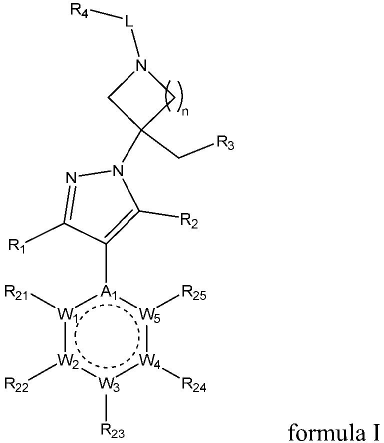 wherein R₁ is -NHR₁₁,-OR₁₁, -SR₁₁ or -C(=O)NHR₁₁; wherein R₁₁ is each independently selected from the group consisting of hydrogen, substituted or unsubstituted C_{1~6} alkyl, substituted or unsubstituted C₂₋₆ alkenyl, substituted or unsubstituted C₂₋₆ alkynyl, substituted or unsubstituted C_{3~6} cycloalkyl, substituted or unsubstituted C_{3~8} heterocyclyl, substituted or unsubstituted C_{6~20} aryl, substituted or unsubstituted C_{5~20} heteroaryl, substituted or unsubstituted C_{1~6} alkylacyl, substituted or unsubstituted C_{1~6} alkylsulfonyl, substituted or unsubstituted C_{5~20} heteroarylacyl, and substituted or unsubstituted C_{1~6} alkoxy; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, -C(=O)R₃₃, hydroxyl, mercapto, substituted C_{3~8} cycloalkyl, substituted C_{3~8} heterocyclyl, substituted C_{6~20} aryl, and substituted C_{5~20} heteroaryl;
R₂ is selected from the group consisting of hydrogen, halogen, substituted or unsubstituted C_{1~6} alkyl, substituted or unsubstituted C₂₋₆ alkenyl, substituted or unsubstituted C₂₋₆ alkynyl, substituted or unsubstituted C_{1~6} haloalkyl, substituted or unsubstituted C_{1~6} alkoxy, cyano,-NR₃₁R₃₂, hydroxyl, carboxyl, and mercapto; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₃ is selected from the group consisting of cyano, -CONH₂, and carboxyl;
L is selected from the group consisting of -S(=O)₂-, -C(=O)-, -CH₂-, and -S(=O)(=N)R_{L}-, wherein R_{L} is selected from the group consisting of hydrogen, and substituted or unsubstituted C_{1~8} alkyl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano,-NR₃₁R₃₂, hydroxyl, carboxyl, and mercapto;
R₄ is selected from the group consisting of halogen, substituted or unsubstituted C_{1~8} alkyl, -NR₃₁R₃₂, substituted or unsubstituted C_{1~8} alkoxy, substituted or unsubstituted C₂₋₈ alkenyl, substituted or unsubstituted C₂₋₈ alkynyl, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, hydrazide, C_{1~8} alkylsulfonyl, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, -COR₃₃, carboxyl, and mercapto;
A₁ is selected from C atom;
W₁, W₂, W₃, W₄ and W₅ are each independently selected from the group consisting of C atom and N atom, and one, two, or three of W₁, W₂, W₃, W₄ and W₅ are N atom; the dashed circle represents the bonds that form aromatic ring;
R₂₁ is selected from the group consisting of hydrogen, halogen, -NR₃₁R₃₂, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₂₂ is selected from the group consisting of hydrogen, halogen, -NR₃₁R₃₂, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₂₃ is selected from the group consisting of hydrogen, halogen, -NR₃₁R₃₂, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₂₄ is selected from the group consisting of hydrogen, halogen, -NR₃₁R₃₂, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₂₅ is selected from the group consisting of hydrogen, halogen, -NR₃₁R₃₂, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
or, R₂₄, and W₄ connected to R₂₄ together with R₂₅ and W₅ connected to R₂₅ form a 5-membered aryl, cycloalkyl, heteroaryl, or heterocycloalkyl ring fused with a 6-membered ring formed by A₁, W₁, W₂, W₃, W₄ and W₅;
R₃₁ and R₃₂ are each independently selected from the group consisting of hydrogen, sulfonamido, substituted or unsubstituted C_{1~6} alkyl, substituted or unsubstituted C₂₋₆ alkenyl, substituted or unsubstituted C₂₋₆ alkynyl, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, -C(=O)OR₃₄, and mercapto;
R₃₃ is selected from the group consisting of hydrogen, substituted or unsubstituted C_{1~8} alkyl, substituted or unsubstituted C_{1~8} alkoxy, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, or mercapto;
R₃₄ is selected from the group consisting of hydrogen, substituted or unsubstituted C_{1~8} alkyl, substituted or unsubstituted C_{1~8} haloalkyl, substituted or unsubstituted C_{1~8} alkoxy, and substituted or unsubstituted C_{3~8} cycloalkyl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, carboxyl, and mercapto;
n is 1, 2 or 3;
and, the compound is not the following compounds:

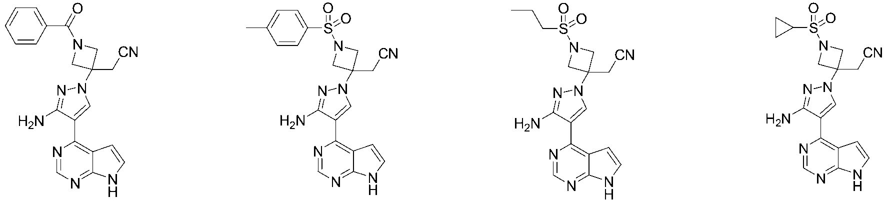

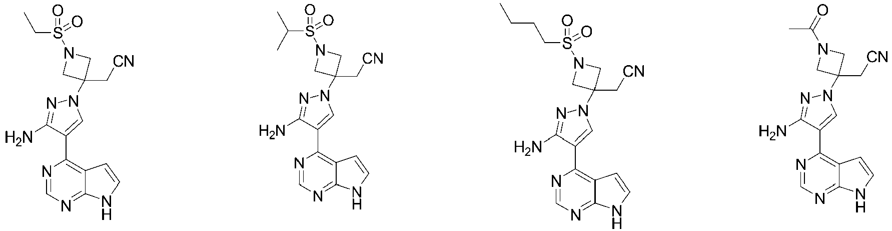

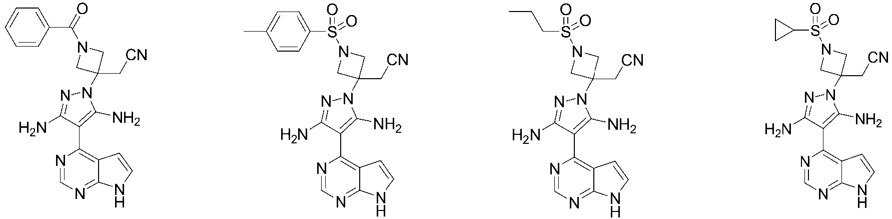

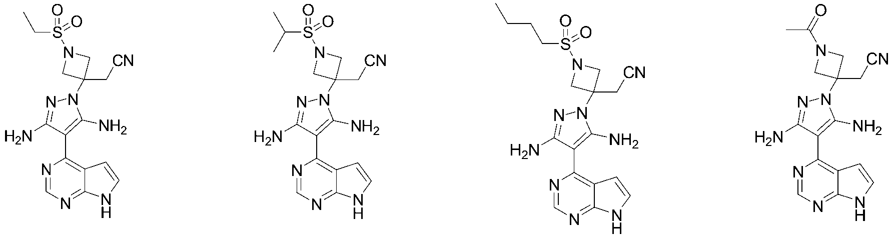

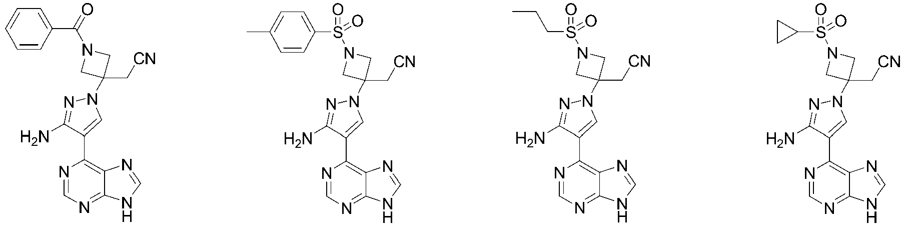

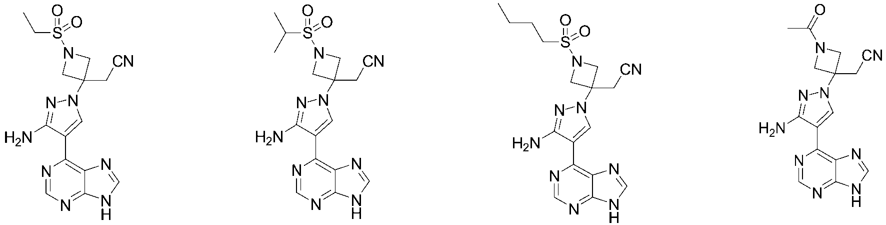

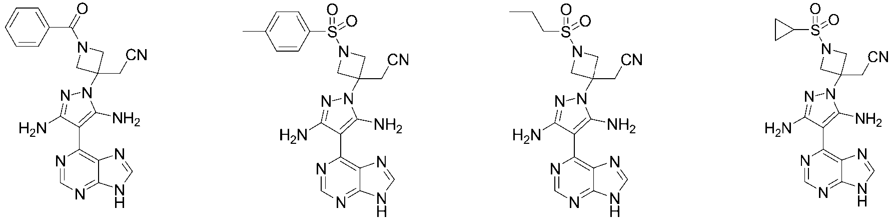

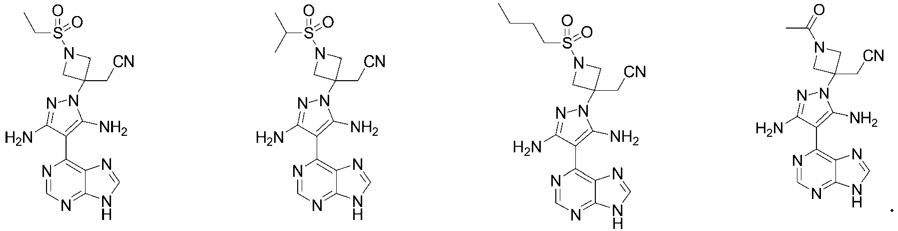

The inventor found through research that the compounds of the present disclosure have good Rho kinase inhibitory activity and can be used as inhibitors targeting ROCK, which can be used to regulate Rho kinase mediated diseases.

In one embodiment, R₁ is selected from -NHR₁₁, wherein R₁₁ is each independently selected from the group consisting of hydrogen, substituted or unsubstituted C_{1~6} alkyl, substituted or unsubstituted C₂₋₆ alkenyl, substituted or unsubstituted C₂₋₆ alkynyl, substituted or unsubstituted C_{3~6} cycloalkyl, substituted or unsubstituted C_{3~8} heterocyclyl, substituted or unsubstituted C_{6~20} aryl, substituted or unsubstituted C_{5~20} heteroaryl, substituted or unsubstituted C_{1~6} alkylacyl, substituted or unsubstituted C_{1~6} alkylsulfonyl, substituted or unsubstituted C_{5~20} heteroarylacyl, and substituted or unsubstituted C_{1~6} alkoxy; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, -C(=O)R₃₃, hydroxyl, mercapto, substituted C_{3~8} cycloalkyl, substituted C_{3~8} heterocyclyl, substituted C_{6~20} aryl, and substituted C_{5~20} heteroaryl;
wherein R₃₁ and R₃₂ are each independently selected from the group consisting of hydrogen, substituted or unsubstituted C_{1~6} alkyl, substituted or unsubstituted C_{1~6} alkyl, substituted or unsubstituted C₂₋₆ alkenyl, substituted or unsubstituted C₂₋₆ alkynyl, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, -C(=O)OR₃₄, and mercapto;
R₃₃ is selected from the group consisting of hydrogen, substituted or unsubstituted C_{1~8} alkyl, substituted or unsubstituted C_{1~8} alkoxy, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₃₄ is selected from the group consisting of hydrogen, substituted or unsubstituted C_{1~8} alkyl, substituted or unsubstituted C_{1~8} haloalkyl, substituted or unsubstituted C_{1~8} alkoxy, and substituted or unsubstituted C_{3~8} cycloalkyl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, carboxyl, and mercapto.

In one embodiment, R₁ is selected from -NHR₁₁, wherein R₁₁ is selected from the group consisting of hydrogen and substituted or unsubstituted C_{1~6} alkyl, and the substituent is selected from the group consisting of -NR₃₁R₃₂, -C(=O)R₃₃ and substituted or unsubstituted C_{3~8} heterocyclyl. For example, R₁₁ can be selected from the group consisting of hydrogen, optionally substituted methyl, optionally substituted ethyl, optionally substituted propyl, optionally substituted isopropyl, optionally substituted butyl, optionally substituted tert-butyl, optionally substituted pentyl, optionally substituted hexyl, etc. The substituent can be selected from the group consisting of -NR₃₁R₃₂, -C(=O)R₃₃, and substituted or unsubstituted C_{3~8} heterocyclyl (the substituent can be C_{1~6} alkyl, such as methyl, ethyl, propyl, isopropyl, and the like), etc. Preferably, R₁₁ is selected from substituted or unsubstituted C_{1~6} alkyl, and the substituent is selected from the group consisting of -NR₃₁R₃₂, wherein R₃₁ and R₃₂ are each independently hydrogen, substituted or unsubstituted C_{1~6} alkyl, or substituted or unsubstituted C_{3~8} heterocyclyl (the substituent can be C_{1~6} alkyl, such as methyl, ethyl, propyl, isopropyl, and the like). Preferably, R₁₁ is selected from substituted or unsubstituted C_{1~6} alkyl, and the substituent is selected from the group consisting of -C(=O)R₃₃, wherein R₃₃ is independently substituted or unsubstituted C_{1~6} alkyl, or substituted or unsubstituted C_{3~8} heterocyclyl (the substituent can be C_{1~6} alkyl, such as methyl, ethyl, propyl, isopropyl, and the like).

In one embodiment, R₁ is selected from the group consisting of -NH₂, -NHC(=O)CH₃,

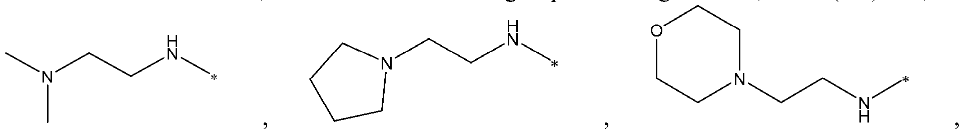

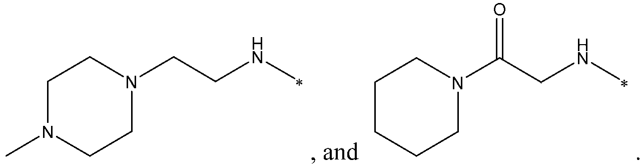

In one embodiment, R₁ is selected from the group consisting of -OR₁₁, wherein R₁₁ is each independently selected from the group consisting of hydrogen, substituted or unsubstituted C_{1~6} alkyl, substituted or unsubstituted C₂₋₆ alkenyl, substituted or unsubstituted C₂₋₆ alkynyl, substituted or unsubstituted C_{3~6} cycloalkyl, substituted or unsubstituted C_{3~8} heterocyclyl, substituted or unsubstituted C_{6~20} aryl, substituted or unsubstituted C_{5~20} heteroaryl, substituted or unsubstituted C_{1~6} alkylacyl, substituted or unsubstituted C_{1~6} alkylsulfonyl, substituted or unsubstituted C_{5~20} heteroarylacyl, and substituted or unsubstituted C_{1~6} alkoxy; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, -C(=O)R₃₃, hydroxyl, and mercapto ;
preferably, R₁₁ is selected from the group consisting of hydrogen and substituted or unsubstituted C_{1~6} alkyl, and the substituent is selected from the group consisting of -NR₃₁R₃₂,-C(=O)R₃₃, and substituted or unsubstituted C_{3~8} heterocyclyl;
wherein R₃₁ and R₃₂ are each independently selected from the group consisting of hydrogen, substituted or unsubstituted C_{1~6} alkyl, substituted or unsubstituted C_{1~6} alkyl, substituted or unsubstituted C₂₋₆ alkenyl, substituted or unsubstituted C₂₋₆ alkynyl, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, -C(=O)OR₃₄, and mercapto;
R₃₃ is selected from the group consisting of hydrogen, substituted or unsubstituted C_{1~8} alkyl, substituted or unsubstituted C_{1~8} alkoxy, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₃₄ is selected from the group consisting of hydrogen, substituted or unsubstituted C_{1~8} alkyl, substituted or unsubstituted C_{1~8} haloalkyl, substituted or unsubstituted C_{1~8} alkoxy, and substituted or unsubstituted C_{3~8} cycloalkyl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, carboxyl, and mercapto.

In one embodiment, R₁ is selected from the group consisting of -OR₁₁, wherein R₁₁ is hydrogen, or C_{1~6} alkyl (for example C₁₋₃ alkyl, such as methyl, ethyl, propyl, isopropyl).

In one embodiment, R₁ is selected from -C(=O)NHR₁₁, wherein R₁₁ is each independently selected from the group consisting of hydrogen, substituted or unsubstituted C_{1~6} alkyl, substituted or unsubstituted C₂₋₆ alkenyl, substituted or unsubstituted C₂₋₆ alkynyl, substituted or unsubstituted C_{3~6} cycloalkyl, substituted or unsubstituted C_{3~8} heterocyclyl, substituted or unsubstituted C_{6~20} aryl, substituted or unsubstituted C_{5~20} heteroaryl, substituted or unsubstituted C_{1~6} alkylacyl, substituted or unsubstituted C_{1~6} alkylsulfonyl, substituted or unsubstituted C_{5~20} heteroarylacyl, and substituted or unsubstituted C_{1~6} alkoxy; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, -C(=O)R₃₃, hydroxyl, and mercapto;
preferably, R₁₁ is selected from the group consisting of hydrogen and substituted or unsubstituted C_{1~6} alkyl, and the substituent is selected from the group consisting of -NR₃₁R₃₂, - C(=O)R₃₃, and substituted or unsubstituted C_{3~8} heterocyclyl;
wherein R₃₁ and R₃₂ are each independently selected from the group consisting of hydrogen, substituted or unsubstituted C_{1~6} alkyl, substituted or unsubstituted C_{1~6} alkyl, substituted or unsubstituted C₂₋₆ alkenyl, substituted or unsubstituted C₂₋₆ alkynyl, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, -C(=O)OR₃₄, and mercapto;
R₃₃ is selected from the group consisting of hydrogen, substituted or unsubstituted C_{1~8} alkyl, substituted or unsubstituted C_{1~8} alkoxy, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₃₄ is selected from the group consisting of hydrogen, substituted or unsubstituted C_{1~8} alkyl, substituted or unsubstituted C_{1~8} haloalkyl, substituted or unsubstituted C_{1~8} alkoxy, and substituted or unsubstituted C_{3~8} cycloalkyl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, carboxyl, and mercapto.

In one embodiment, R₁ is selected from -C(=O)NHR₁₁, wherein R₁₁ is hydrogen, or C_{1~6} alkyl (for example C₁₋₃ alkyl, such as methyl, ethyl, propyl, isopropyl).

In the above embodiments involving R₁, R₃₁ and R₃₂ can each independently be hydrogen, substituted or unsubstituted C_{1~6} alkyl, or substituted or unsubstituted C_{3~8} heterocyclyl (the substituent can be C_{1~6} alkyl, such as methyl, ethyl, propyl, isopropyl, and the like). R₃₃ can independently be substituted or unsubstituted C_{1~6} alkyl, or substituted or unsubstituted C_{3~8} heterocyclyl (the substituent can be C_{1~6} alkyl, such as methyl, ethyl, propyl, isopropyl, and the like).

In one embodiment, R₂ is selected from hydrogen, and R₃ is selected from cyano.

In formula I, the dashed circle represents the bonds that form aromatic ring. In one embodiment, W₁, W₂, W₃, W₄ and W₅ are each independently selected from the group consisting of C atom and N atom, and one, two, or three of W₁, W₂, W₃, W₄ and W₅ are N atom. In one embodiment, two of W₁, W₂, W₃, W₄ and W₅ are N atom. In one embodiment, three of W₁, W₂, W₃, W₄ and W₅ are N atom. In one embodiment, W₃ is N atom, and one or two of W₁, W₂, W₄ and W₅ are N atom.

In one embodiment, 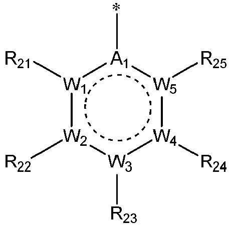 in formula I can be selected from the group consisting of the following structures A1-A7: 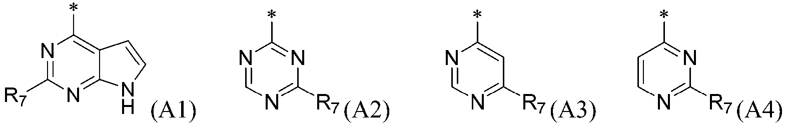 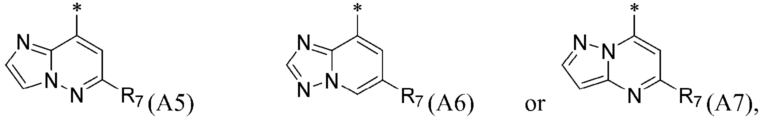
wherein, in the structures A1-A7, R₇ is each independently selected from the group consisting of hydrogen, halogen, -NR₃₁R₃₂, and substituted or unsubstituted C_{5~20} heteroaryl,
wherein R₃₁ and R₃₂ are each independently selected from the group consisting of hydrogen, substituted or unsubstituted C_{1~6} alkyl, substituted or unsubstituted C_{6~20} aryl, substituted or unsubstituted C_{5~20} heteroaryl;
wherein the substituent is selected from the group consisting of halogen, C_{1∼8} alkyl, C_{1∼8} haloalkyl, C_{1~8} alkoxy, C_{3~8} cycloalkyl, C_{3~8} heterocyclyl, C_{6~20} aryl, C_{5~20} heteroaryl, cyano, hydroxyl, carboxyl, and mercapto.

In the above structural formulas, * represents the position connected to the pyrazole ring structure.

In one embodiment, R₇ is each independently selected from the group consisting of hydrogen, -NR₃₁R₃₂, and substituted or unsubstituted C_{5~20} heteroaryl,
wherein R₃₁ and R₃₂ are each independently selected from the group consisting of hydrogen, substituted or unsubstituted C_{1~6} alkyl, substituted or unsubstituted C_{5~20} heteroaryl, and substituted or unsubstituted C_{3~8} heterocyclyl;
wherein the substituent is selected from C_{1~8} alkyl.

In one embodiment, 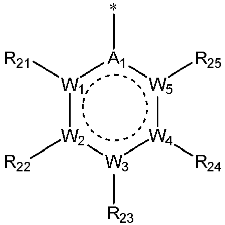 in formula I can be the following structure A1: 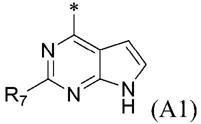 wherein, in the structure A1, R₇ is hydrogen.

In one embodiment, 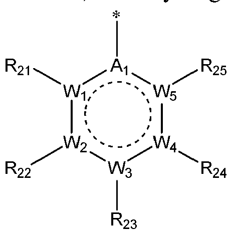 in formula I is the following structure A2: 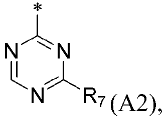
wherein, in the structure A2, R₇ is -NR₃₁R₃₂, or substituted or unsubstituted C_{5~20} heteroaryl,
wherein R₃₁ and R₃₂ are each independently selected from the group consisting of hydrogen, substituted or unsubstituted C_{1~6} alkyl, and substituted or unsubstituted C_{5~20} heteroaryl;
wherein the substituent is selected from the group consisting of C_{1∼8} alkyl and hydroxyl.

In one embodiment, 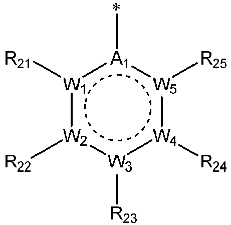 in formula I is the following structure A3: 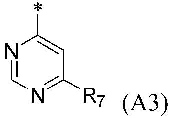
wherein, in the structure A3, R₇ is -NR₃₁R₃₂, or substituted or unsubstituted C_{5~20} heteroaryl,
wherein R₃₁ and R₃₂ are each independently selected from the group consisting of hydrogen, substituted or unsubstituted C_{1~6} alkyl, and substituted or unsubstituted C_{5~20} heteroaryl;
wherein the substituent is selected from the group consisting of C_{1∼8} alkyl, and hydroxyl.

In one embodiment, 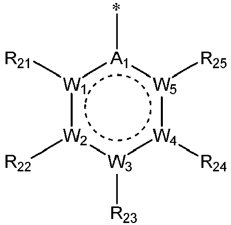 in formula I is the following structure A4: 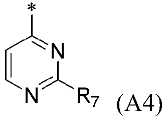
wherein, in the structure A4, R₇ is -NR₃₁R₃₂, or substituted or unsubstituted C_{5~20} heteroaryl,
wherein R₃₁ and R₃₂ are each independently selected from the group consisting of hydrogen, substituted or unsubstituted C_{1~6} alkyl, and substituted or unsubstituted C_{5~20} heteroaryl;
wherein the substituent is selected from the group consisting of C_{1∼8} alkyl, and hydroxyl.

In one embodiment, 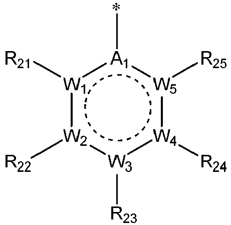 in formula I is the following structure A5: 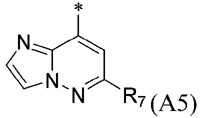
wherein, in the structure A5, R₇ is -NR₃₁R₃₂, and substituted or unsubstituted C_{5~20} heteroaryl,
wherein R₃₁ and R₃₂ are each independently selected from the group consisting of hydrogen, substituted or unsubstituted C_{1~6} alkyl, and substituted or unsubstituted C_{5~20} heteroaryl;
wherein the substituent is selected from the group consisting of C_{1∼8} alkyl, and hydroxyl.

In one embodiment, 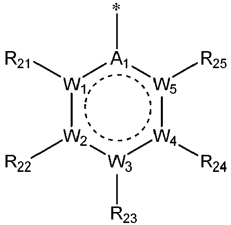 in formula I is the following structure A6: 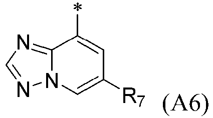
wherein, in the structure A6, R₇ is -NR₃₁R₃₂, or substituted or unsubstituted C_{5~20} heteroaryl,
wherein R₃₁ and R₃₂ are each independently selected from the group consisting of hydrogen, substituted or unsubstituted C_{1~6} alkyl, and substituted or unsubstituted C_{5~20} heteroaryl;
wherein the substituent is selected from the group consisting of C_{1∼8} alkyl, and hydroxyl.

In one embodiment, 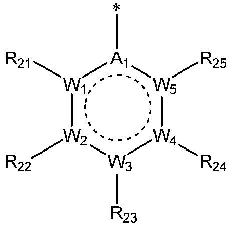 in formula I is the following structure A7: 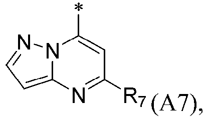
wherein, in the structure A7, R₇ is -NR₃₁R₃₂, or substituted or unsubstituted C_{5~20} heteroaryl,
wherein R₃₁ and R₃₂ are each independently selected from the group consisting of hydrogen, substituted or unsubstituted C_{1~6} alkyl, and substituted or unsubstituted C_{5~20} heteroaryl;
wherein the substituent is selected from the group consisting of C_{1∼8} alkyl, and hydroxyl.

In the above embodiments, R₇ can be selected from the group consisting of -NHCH₃,-NHCH₂CH₂OH, Cl,

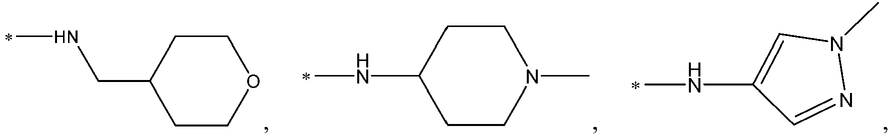

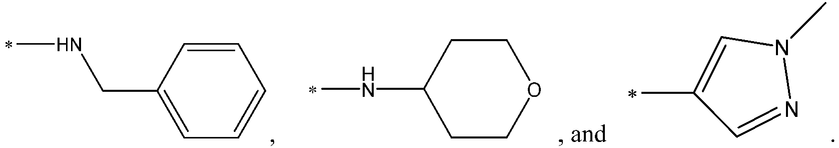

In one embodiment, L is selected from the group consisting of -S(=O)₂-, -C(=O)-, and-S(=O)(=N)R_{L}-, wherein R_{L} is selected from the group consisting of hydrogen, and C_{1~6} alkyl. In one embodiment, L is selected from the group consisting of -S(=O)₂-. In one embodiment, L is selected from the group consisting of -C(=O)-. In one embodiment, L is selected from the group consisting of -S(=O)(=N)R_{L}-, wherein R_{L} is selected from the group consisting of hydrogen, and C_{1~6} alkyl, for example methyl, ethyl, propyl, isopropyl, butyl, tert-butyl, pentyl or hexyl, etc.

In one embodiment, R₄ is selected from the group consisting of substituted or unsubstituted C_{1∼8} alkyl, -NR₃₁R₃₂, substituted or unsubstituted C_{1∼8} alkoxy, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, hydrazide, C_{1∼8} alkylsulfonyl, C_{1~8} alkyl, C_{1∼8} haloalkyl, C_{1∼8} alkoxy, C_{3~8} cycloalkyl, C_{3~8} heterocyclyl, C_{6~20} aryl, C_{5~20} heteroaryl, cyano, -NR₃₁R₃₂, hydroxyl, -COR₃₃, carboxyl, and mercapto;
wherein R₃₁ and R₃₂ are each independently selected from the group consisting of hydrogen, sulfonamido, substituted or unsubstituted C_{1~6} alkyl, substituted or unsubstituted C_{1~6} alkyl, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1∼8} alkyl, C_{1∼8} alkoxy, C_{3~8} cycloalkyl, cyano, -NR₃₁R₃₂, hydroxyl, -C(=O)OR₃₄, and mercapto.

In one embodiment, R₄ is selected from the group consisting of substituted or unsubstituted C_{1∼8} alkyl (for example methyl, ethyl, propyl, etc), wherein the substituent can be halogen, hydroxyl, cyano, C_{1∼8} alkylsulfonyl, etc. For example, R₄ can be methyl, ethyl, trifluoroethyl, trifluoromethyl, methylsulfonylmethyl, etc.

In one embodiment, R₄ is selected from the group consisting of substituted or unsubstituted C_{3~8} cycloalkyl (for example cyclopropyl, cyclobutyl, etc), wherein the substituent can be halogen, hydroxyl, cyano, etc. For example, R₄ can be 2,2-difluorocyclopropyl, 2-cyanocyclopropyl, etc.

In one embodiment, R₄ is selected from the group consisting of -NR₃₁R₃₂, wherein R₃₁ and R₃₂ are each independently selected from the group consisting of hydrogen, sulfonamido, substituted or unsubstituted C_{1~6} alkyl, substituted or unsubstituted C_{1~6} alkyl, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1∼8} alkoxy, C_{3~8} cycloalkyl, cyano,-NR₃₁R₃₂, hydroxyl, -C(=O)OR₃₃, and mercapto; R₃₃ is selected from the group consisting of hydrogen and C_{1~6} alkyl (for example methyl, ethyl, propyl, etc). In one embodiment, R₄ is selected from the group consisting of -NR₃₁R₃₂, wherein R₃₁ is hydrogen, and R₃₂ is selected from the group consisting of substituted or unsubstituted C_{1∼8} alkyl (for example methyl, ethyl, propyl, etc), substituted or unsubstituted C_{3~8} cycloalkyl (for example cyclopropyl, etc), and substituted or unsubstituted C_{5~20} heteroaryl (for example thiadiazolyl, etc), wherein the substituent can be halogen, hydroxyl, cyano, etc. In one embodiment, R₄ is selected from the group consisting of -NR₃₁R₃₂, wherein R₃₁ is hydrogen, and R₃₂ is independently selected from substituted or unsubstituted C_{1~6} alkyl (for example methyl, ethyl, propyl, etc), and the substituent is selected from the group consisting of -C(=O)OR₃₄, wherein R₃₄ is selected from the group consisting of hydrogen, and C_{1~6} alkyl (for example methyl, ethyl, propyl, etc).

In one embodiment, n is 1, 2, or 3, particularly 1 or 2. When n is 1, the azacycloalkane ring is azacyclobutyl; when n is 2, the azacycloalkane ring is azacyclopentyl; when n is 3, the azacycloalkane ring is azacyclohexyl.

In one embodiment, the azacycloalkane compound is selected from the following compounds:

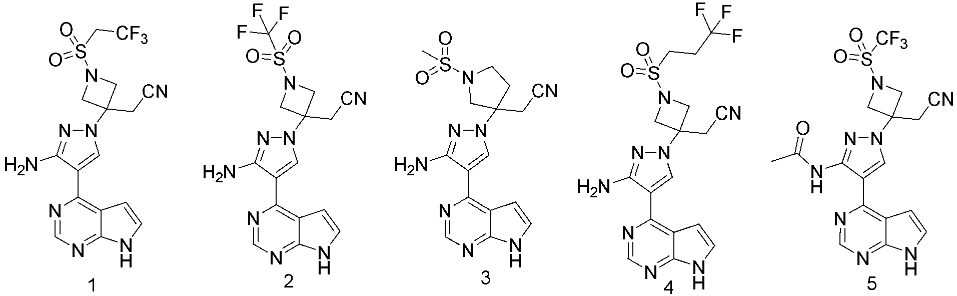

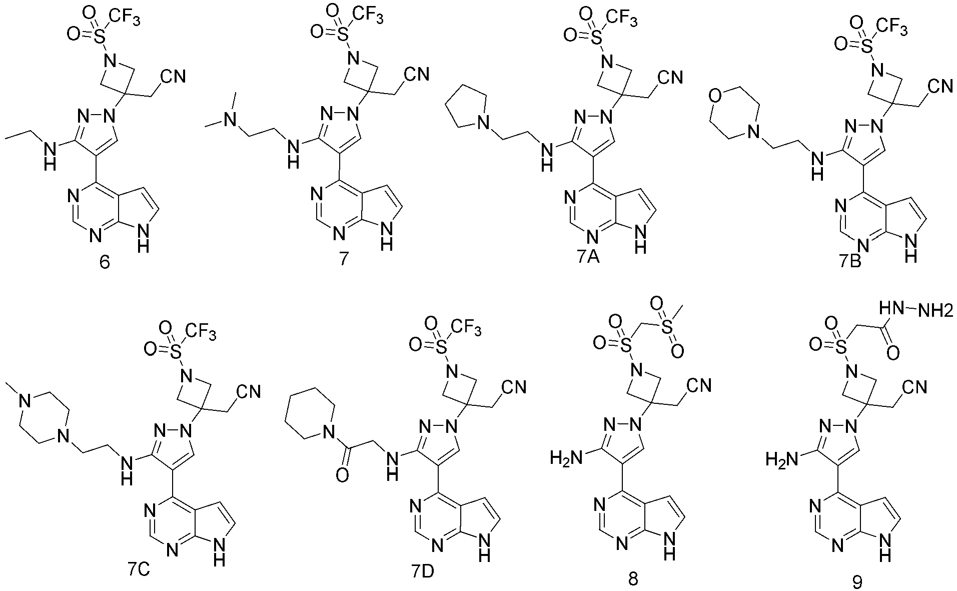

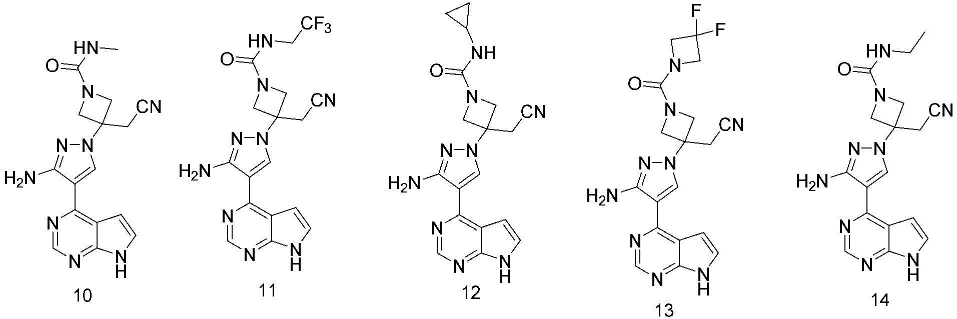

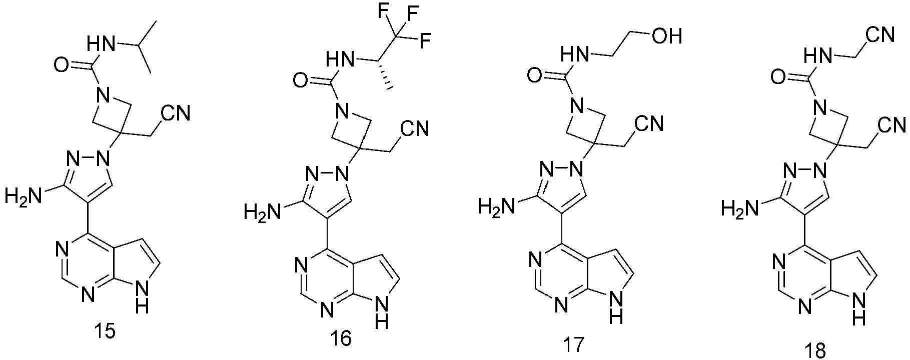

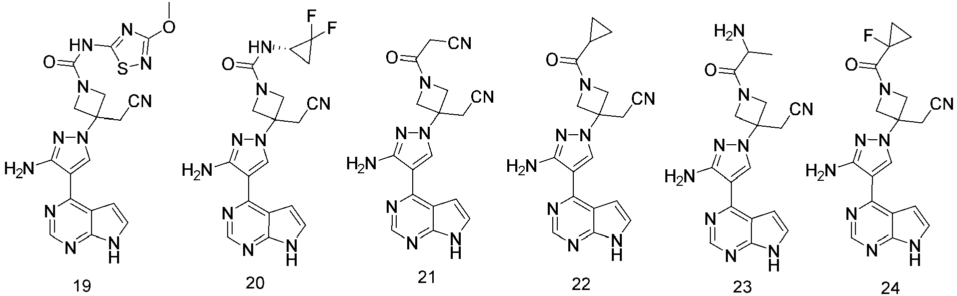

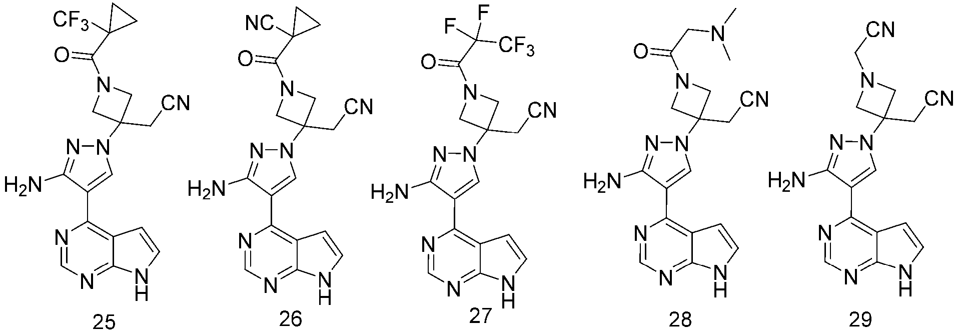

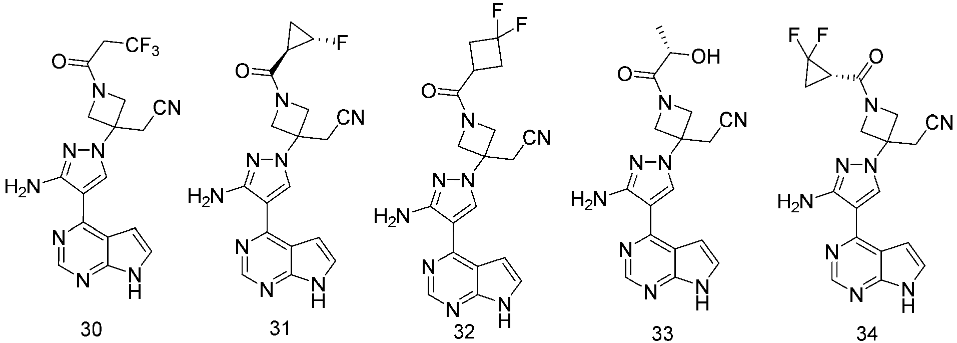

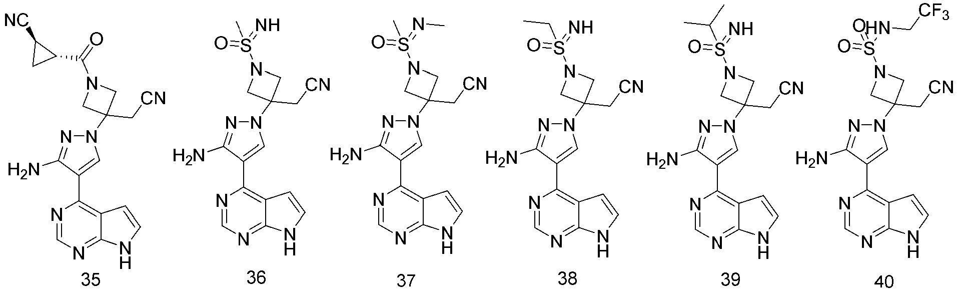

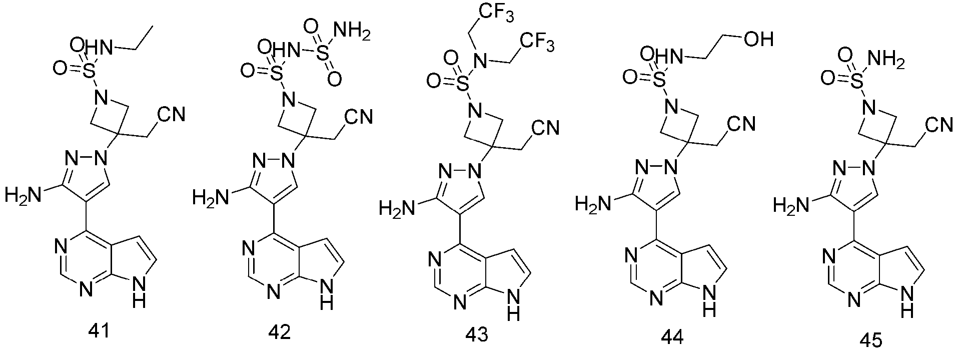

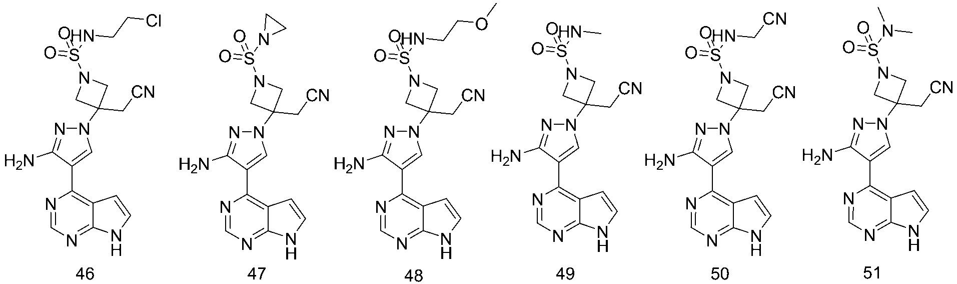

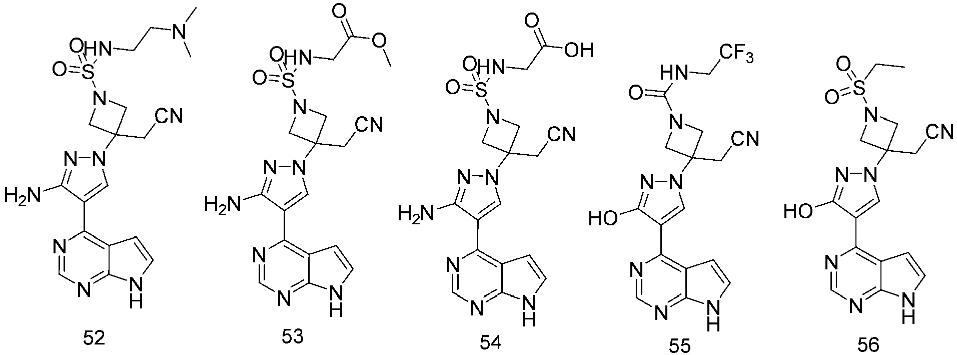

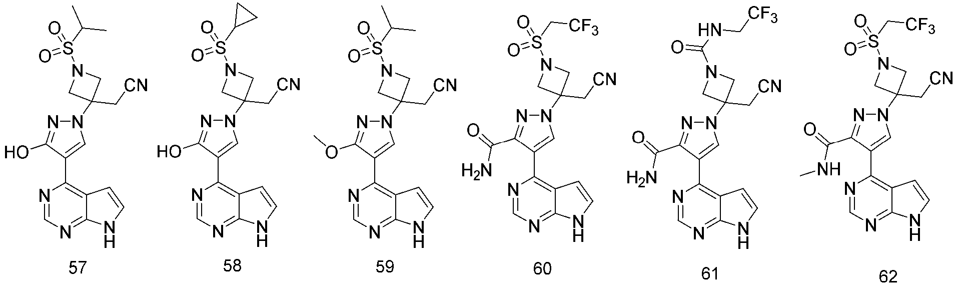

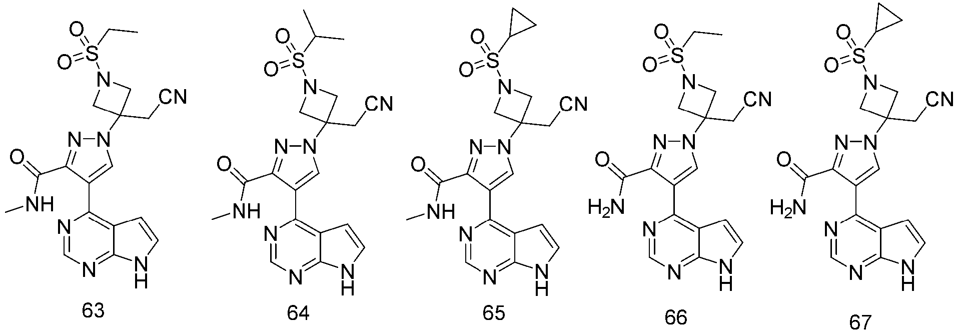

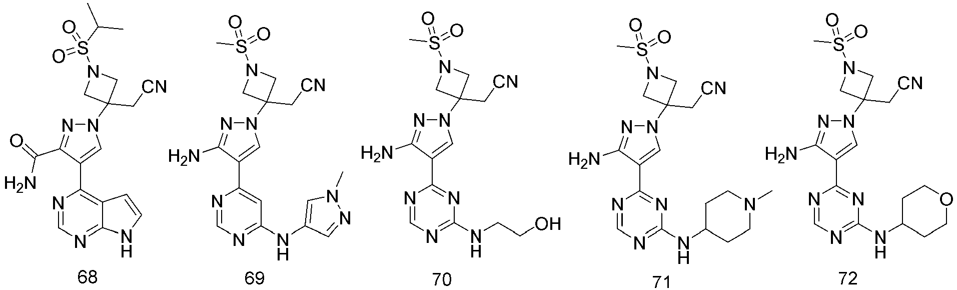

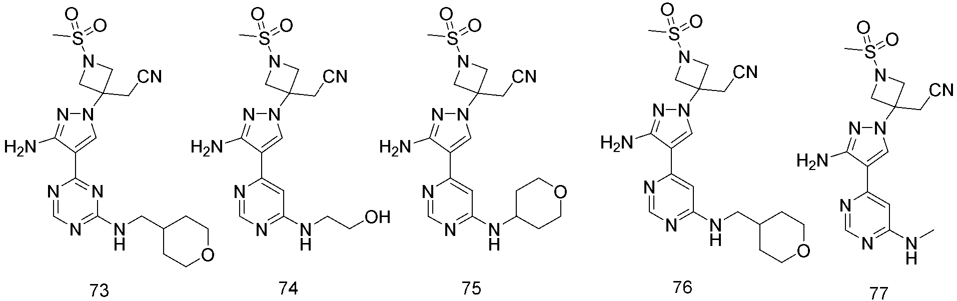

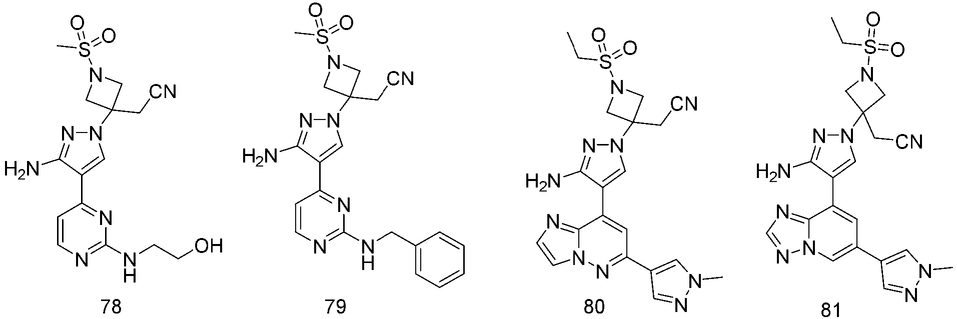

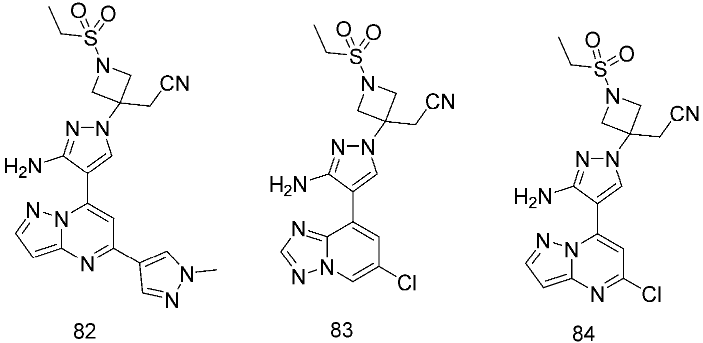

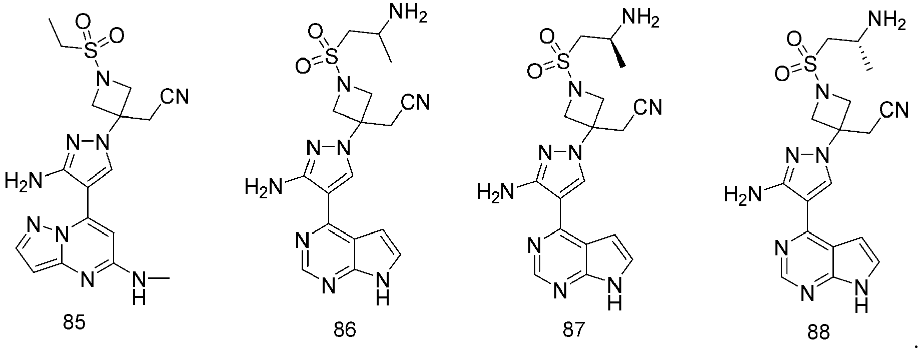

In a referred embodiment, the azacycloalkane compound is selected from the following compounds:

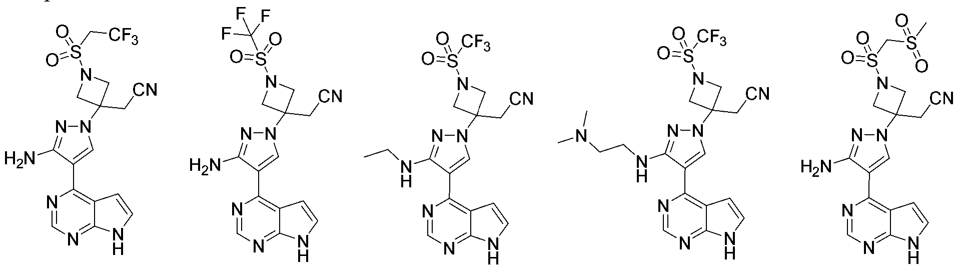

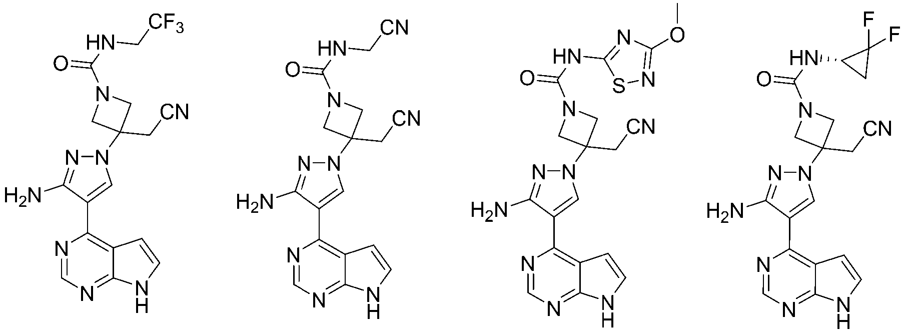

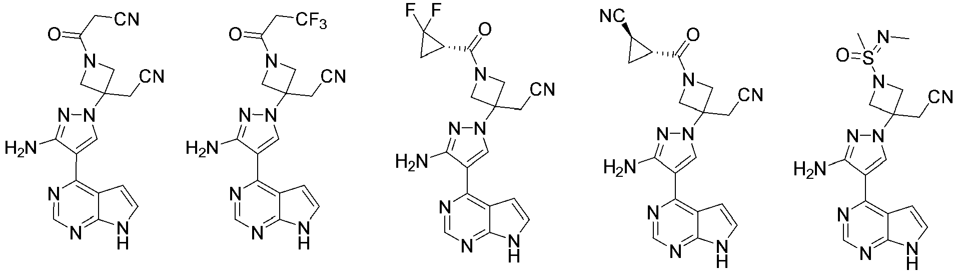

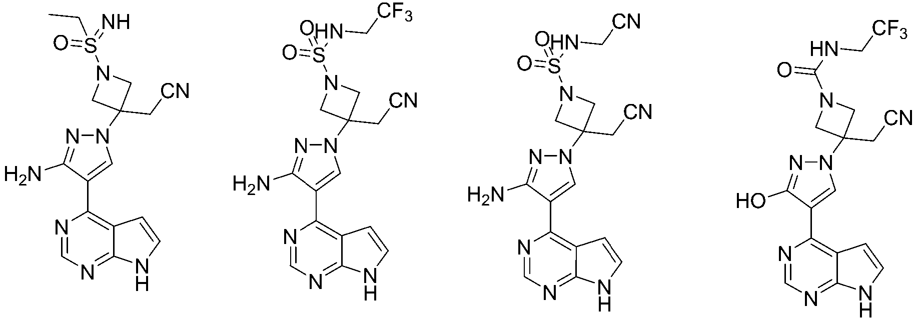

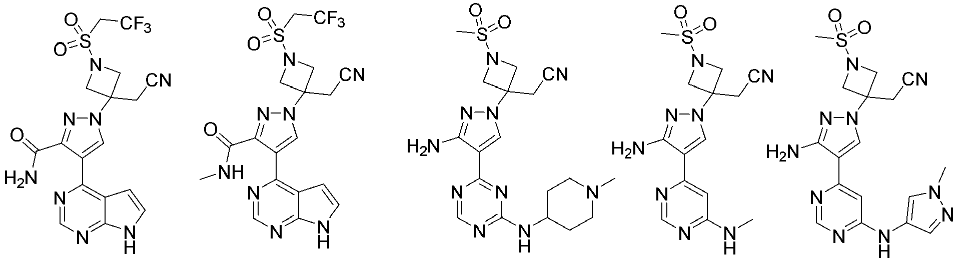

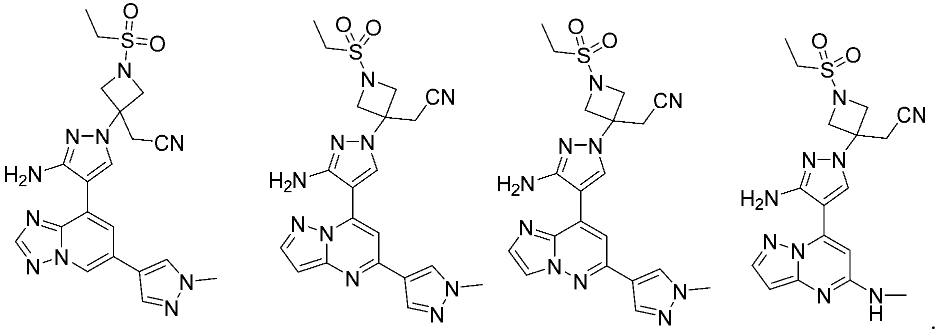

The compounds described in the present disclosure can be prepared by the following methods. The following methods and examples are to illustrate these methods. These procedures and examples should not be construed as limiting the present disclosure in any way. The compounds described herein can also be synthesized using standard synthesis techniques known to those skilled in the art, or methods known in the art and methods described herein can be used in combination.

The chemical reactions in the embodiments of the present disclosure are completed in a suitable solvent, and the solvent must be suitable for the chemical changes of the present disclosure and the reagents and materials required. In order to obtain the compounds of the present disclosure, it is sometimes necessary for those skilled in the art to modify or select the synthesis steps or reaction schemes based on the existing embodiments.

An important consideration in the planning of any synthetic route in the art is to select an appropriate protecting group for the reactive functional group (such as the amino group in the present disclosure). For trained practitioners, Greene and Wuts (Protective Groups In Organic Synthesis, Wiley and Sons, 1991) is the authority in this regard. All references cited in the present disclosure are incorporated into the present disclosure in their entirety.

The reactions described herein can be monitored according to any suitable method known in the art. For example, product formation can be monitored by a broad spectrum method such as nuclear magnetic resonance spectroscopy (such as ¹H or ¹³C), infrared spectroscopy, spectrophotometry (such as UV-visible light), mass spectrometry, etc., or chromatography such as high performance liquid chromatography (HPLC) or thin layer chromatography.

The compound of formula I of the present disclosure can be prepared by those skilled in the field of organic synthesis using standard methods in the art through the following procedures.

### Procedure 1:

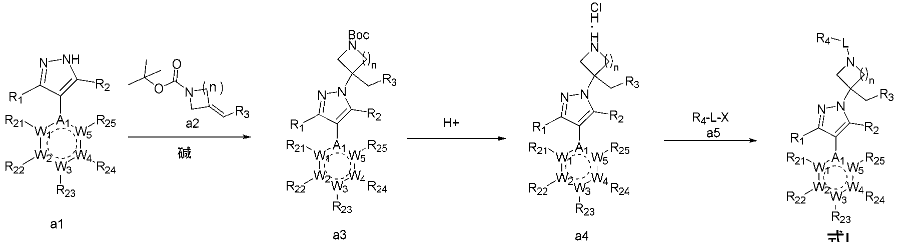

Compound a1 undergoes Michael addition reaction with compound a2 to generate compound a3. Compound a3 is deprotected to obtain compound a4, which undergoes substitution reaction with compound a5 to obtain compound I of the present disclosure.

### Procedure 2:

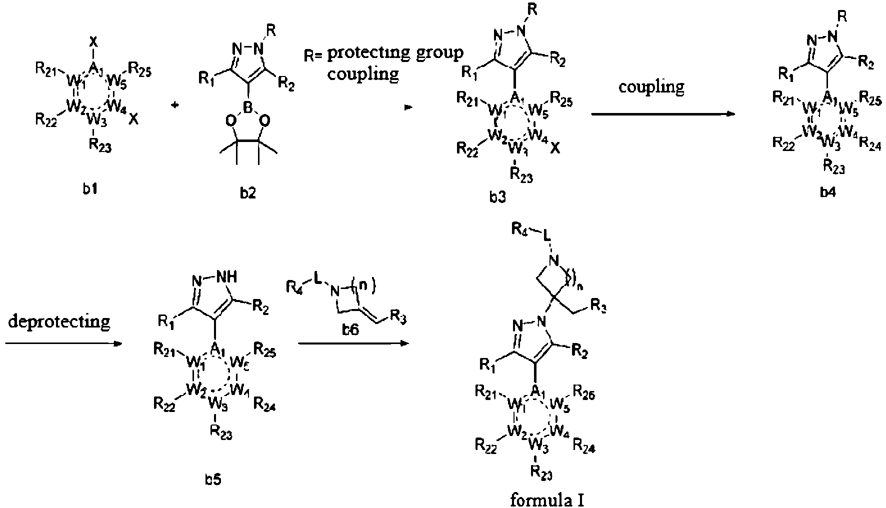

Compound b1 undergoes coupling reaction with compound b2 to generate compound b3, which reacts with substituted boronic acid or substituted amino compound to generate compound b4. Compound b4 is deprotected to generate compound b5, which undergoes Michael addition reaction with compound b6 to generate compound I of the present disclosure.

### Pharmaceutical composition and use thereof

The present invention also provides a pharmaceutical composition, which comprises the compound or pharmaceutically acceptable salts, solvates, active metabolites, polymorphs, isotopic markers, isomers or prodrugs thereof in any one of the above embodiments, and a pharmaceutically acceptable carrier.

The pharmaceutical composition comprises, but not limited to, oral, parenteral, topical and rectal dosage forms, etc. For example, the pharmaceutical composition can be in the form of a tablet, capsule, pill, powder, sustained release preparation, solution or suspension for oral administration; a sterile solution, suspension or emulsion for parenteral injection; an ointment, a cream, or a gel, etc. for external use; eye drops for external use; inhalants for external use; or a suppository for rectal administration.

The pharmaceutical composition can also comprise other active ingredients or pharmaceuticals, in combination with the compound or pharmaceutically acceptable salts, solvates, active metabolites, polymorphs, isotopic markers, isomers or prodrugs thereof.

The present invention also provides use of the above mentioned compound or pharmaceutically acceptable salts, solvates, active metabolites, polymorphs, isotopic markers, isomers or prodrugs thereof, as well as the above mentioned pharmaceutical composition in manufacture of pharmaceuticals for treating Rho kinase mediated diseases, wherein the Rho kinases can comprise types such as ROCK1 and ROCK2.

The present invention also relates to method for treating Rho kinase mediated diseases, which comprises administering a therapeutically effective amount of the above mentioned azacycloalkane compound or pharmaceutically acceptable salts, solvates, active metabolites, polymorphs, isotopic markers, isomers or prodrugs thereof, or the above mentioned pharmaceutical composition to a patient in need of administration, wherein the Rho kinases can comprise types such as ROCK1 and ROCK2.

In one embodiment, the Rho kinase mediated diseases are asthma, cancer, glaucoma, insulin resistance, kidney failure, neuronal degeneration, or osteoporosis.

The azacycloalkane compound and the pharmaceutical composition thereof provided in the present disclosure have significant Rho-associated kinase inhibitory activity, the enzymatic activity and cell activity of the azacycloalkane compound and the pharmaceutical composition thereof are superior to those of existing ROCK inhibitors such as Ripasudil, Netarsudil, and Belumosudil, and thus the azacycloalkane compound and the pharmaceutical composition thereof have great potential for application.

In order to clarify the purpose, technical solution, and advantages of the present disclosure, the technical solution of exemplary embodiments of the present disclosure will be further described below.

### Example 1:

### 2-(3-(3-amino-4-(7-H-pyrrolo[2,3-d]pyrimidin-4-yl)-1H-pyrazol-1-yl)-1-((2,2,2-trifluoroethyl)sulfonyl)azetidine-3-yl)acetonitrile

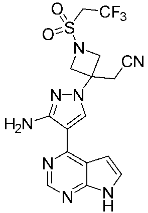

### Synthesis route:

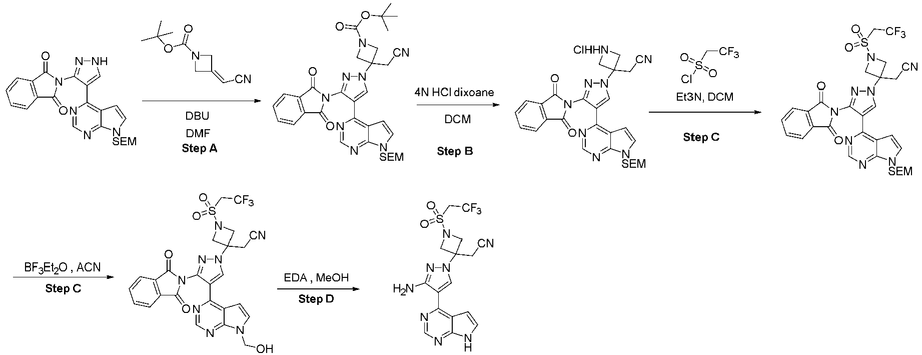

### Step A: tert-butyl 3- (cyanomethyl)-3-(3-(1,3-dioxoisoindol-2-yl)-4-(7-(2-(trimethylsilyl)ethoxy)methyl)-7H-pyrolo[2,3-d]pyramidin-4-yl)-1H-pyrazol-1-yl)azetidine-1-carboxylate

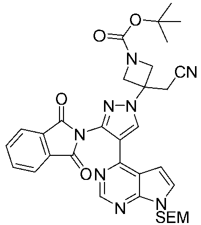

46g (0.1mol, 1.0 eq) 2-(4-(7-((2-(trimethylsilyl)ethoxy)methyl)-7H-pyrrolo[2,3-d]pyrimidin-4-yl)-1H-pyrazol-3-yl)isoindole-1,3-dione and 19.5g (0.1mol, 1.0 eq) tert-butyl 3-(cyanomethyl)azetidine-1- carboxylate were dissolved in 200mL N,N-dimethylformamide. 1.5g (0.01mol, 0.1 eq) 1,8-diazabicyclo[5.4.0]undec-7-ene was slowly added dropwise to the reaction system at room temperature, and the reaction was carried out overnight with stirring at room temperature. The reactrion was quenched by using water. The aqueous phase was extracted by using ethyl acetate, and washed twice with water. The organic layer was dried over anhydrous sodium sulfate, and evaporated under reduced pressure to obtain the product (61g, yield=93%).

### Step B: 2-(3-(1,3-dioxoisoindol-2-yl)-4-(7-(2-(trimethylsilyl)ethoxy)methyl)-7H-pyrrolo[2,3-d]pyrimidin-4-yl)-1H-pyrazol-1-yl)azetidine-3-yl)acetonitrile hydrochloride

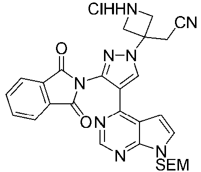

61g (93 mmol, 1.0 eq) tert-butyl 3-(cyanomethyl)-3-(3-(1,3-dioxoisoindole-2-yl)-4-(7-(2-(trimethylsilyl)ethoxy)methyl)-7H-pyrrolo[2,3-d]pyrimidin-4-yl)-1H-pyrazol-1-yl)azetidine-1-carboxylate was dissolved in 300mL dichloromethane. 50mL 4N hydrochloric acid dioxane solution was added at room temperature, and the reaction was carried out overnight with stirring at room temperature. A completed reaction was monitored by TLC. The reaction solution was evaporated to obtain the product (55g, yield=100%).

### Step C: 2-(3-(3-(1,3-dioxoisoindolin-2-yl)-4-(7-(2-(trimethylsilyl)ethoxy)methyl)-7H-pyrrolo[2,3-d]pyrimidin-4-yl)-1H-pyrazol-1-yl)-1-((2,2,2-trifluoroethyl)sulfonyl)azetidine-3-yl)acetonitrile

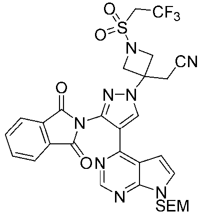

Under an ice bath, 2-(3-(3-(1,3-dioxoisoindolin-2-yl)-4-(7-((2-(trimethylsilyl)ethoxy)methyl)-7H-pyrrolo[2,3-d]pyrimidin-4-yl)-1H-pyrazol-1-yl)azetidine-3-yl)acetonitrile (3 g, 5.41 mmol, 1.0eq) was dissolved in dichloromethane (30 mL). Triethylamine (3.2 g, 32.46 mmol, 6.0 eq) was slowly added dropwise to the reaction solution, and stirring was carried out for 15 minutes. Then, 2,2,2-trifluoroethane-1-sulfonyl chloride (987 mg, 5.41 mmol, 1.0eq) dissolved in 5 ml dichloromethane was added dropwise to the reaction solution. The reaction was carried out for 2h at 25 °C. After the reaction had been completed, the reaction solution was evaporated to dryness under reduced pressure, and the concentrated residue of the reaction solution was passed through column chromatography (PE: EA=1:2) to obtain the product 2-(3-(3-(1,3-dioxoisoindolin-2-yl)-4-(7-(2-trimethylsilylethoxy)methyl)-7H-pyrrolopyrrolidin-4-yl-1H-pyrazolin-1-(2,2,2-trifluoroethylsulfonyl)azetidine-3-yl)acetonitrile (2.1 g, 55.41%) as a yellow oily substance.

### LC-MS (ESI), m/z: [M+H]⁺ =700.9.

### Step D: 2-(3-(3-(1,3-dioxoisoindol-2-yl)-4-(7-(methylol)-7H-pyrrolo[2,3-d]pyrimidin-4-yl)-1H-pyrazol-1-yl)-1-((2,2,2-trifluoroethyl)sulfonyl)azetidine-3-yl)acetonitrile

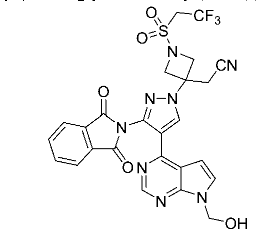

2-(3-(3-(1,3-dioxoisoindolin-2-yl)-4-(7-((2-(trimethylsilyl)ethoxy)methyl)-7H-pyrrolo[2,3-d]pyrimidin-4-yl)-1H-pyrazol-1-yl)-1-((2,2,2-trifluoroethyl)sulfonyl)azetidine-3-yl)acetonitrile (700 mg, 1.0mmol, 1.0eq) was dissolved in acetonitrile (6 mL). Under an ice bath, boron trifluoride-ether solution (2 mL) was added dropwise. The reaction was carried out for 2h at 25 °C. After the reaction had been completed, it was evaporated to dryness under reduced pressure to obtain the crude product 2-(3-amino-4-methylol-7H-pyrrolyl)pyrimidin-4-yl-1-pyrazol-1-(2,2,2-trifluoroethylsulfonyl)azetidine-3-yl acetonitrile (450 mg, crude) as a yellow oily substance.

### LC-MS (ESI), m/z: [M+H]⁺ =601.

### Step E: 2-(3-(3-amino-4-(7H-pyrrolo[2,3-d]pyrimidin-4-yl)-1H-pyrazol-1-yl)-1-((2,2,2-trifluoroethyl)sulfonyl)azetidine-3-yl)acetonitrile

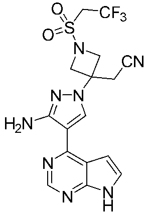

2-(3-(3-(1,3-dioxoisoindole-2-yl)-4-(7-(methylol)-7H-pyrrolo[2,3-d]pyrimidin-4-yl)-1H-pyrazol-1-yl)-1-((2,2,2-trifluoroethyl)sulfonyl)azetidine-3-yl)acetonitrile (450 mg, 0.75mmol) was dissolved in methanol (7 mL). Under an ice bath, ethylenediamine (1 ml) was added. After the reaction had been completed, water (20 mL) was added into the reaction liquid, and then extraction was carried out with ethyl acetate (20 mL*3). The combined organic phase was washed with saturated saline solution (20 mL), dried over anhydrous sodium sulfate, and evaporated under reduced pressure. The residue was passed through Prep-HPLC (chromatographic column type: gemini-C18 150 x 21.2 mm, 5µm, mobile phase: ACN-H2O (0.1% FA), gradient 20% -50%, flow rate: 20 mL/min) to obtain the product (45 mg, yield=11 %).

### LC-MS (ESI), m/z: [M+H]⁺ =440.9.

¹H-NMR(400MHz, D₂O) δ 8.61 (s, 1H), 8.29 (s, 1H), 7.51 (d, J = 3.6 Hz, 1H), 6.76 (d, J = 3.6 Hz, 1H), 4.33 (d, J = 9.3 Hz, 2H), 3.88 (s, 4H), 3.44 (s, 2H).

The following examples were prepared according to the experimental route and method in Example 1:

| Example 2 : 2-(3-(3-amino-4-(7H-pyrrolo[2,3-d]pyrimidin-4-yl)-1H-pyrazol-1-yl)-1-((trifluoromethyl)sulfonyl)azetidine-3-yl)acetonitrile | |
|---|---|
| 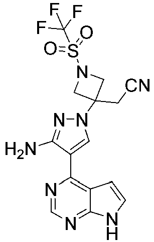 | LC-MS(ESI), m/z: [M+H]⁺ =426.9. |
| | ¹H-NMR(400MHz, DMSO-*d*6) δ 12.09 (s, 1H), 8.68 (s, 1H), 8.65 (s, 1H), 7.58 (s, 1H), 7.09 (d, J = 2.1 Hz,1H), 6.46 (s, 2H), 4.91 (d, J = 9.0 Hz, 2H), 4.59 (d, J = 9.0 Hz, 2H), 3.71 (s, 2H). |

| Example 3: 2-(3-(3-amino-4-(7H-pyrrolo[2,3-d]pyrimidin-4-yl)-1H-pyrazol-1-yl)-1-(methylsulfonyl)pyrrolidin-3-yl)acetonitrile | |
|---|---|
| 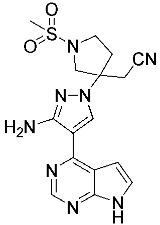 | LC-MS(ESI), m/z: [M+H]⁺ =387.0. |
| | ¹H-NMR(400MHz, CD₃OD) δ 8.66 (s, 1H), 8.49 (s, 1H), 7.42 (d, J = 3.6 Hz, 1H), 6.94 (d, J = 3.6 Hz, 1H), 4.36 (dd, J = 11.4, 1.2 Hz, 1H), 3.69 (d, J = 11.4 Hz, 1H), 3.62 (dd, J = 8.9, 3.2 Hz, 1H), 3.51 (dt, J = 17.3, 8.6 Hz, 1H), 3.27 (d, J = 1.6 Hz, 1H), 3.21 (d, J = 1.4 Hz, 1H), 3.06 - 3.02 (m, 1H), 2.77 (s, 3H), 2.55-2.48 (m, 1H). |

| Example 4 : 2-(3-(3-amino-4-(7H-pyrrolo[2,3-d]pyrimidin-4-yl)-1H-pyrazol-1-yl)-1-((3,3,3-trifluoropropyl)sulfonyl)azetidine-3-yl)acetonitrile | |
|---|---|
| 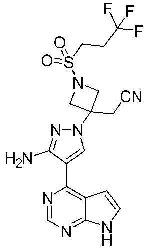 | LC-MS (ESI), m/z: [M+H]⁺ = 454.9. |
| | ¹H-NMR(400MHz, DMSO-*d*6) δ 12.05 (s, 1H), 8.63 (s, 1H), 8.58 (s, 1H), 7.53 (dd, J = 3.4, 2.5 Hz, 1H), 7.06 (dd, J = 3.6, 1.8 Hz, 1H), 6.38 (s, 2H), 4.59 (d, J = 9.0 Hz, 2H), 4.17 (d, J = 9.0 Hz, 2H), 3.58 (s, 2H), 3.56 - 3.46 (m, 2H), 2.89 - 2.67 (m, 2H). |

| Example 5 : N-(1-(3-(cyanomethyl)-1-((trifluoromethyl)sulfonyl)azetidine-3-yl)-4-(7H-pyrrolo[2,3-d]pyrimidin-4-yl)-1H-pyrazol-3-yl amide | |
|---|---|
| 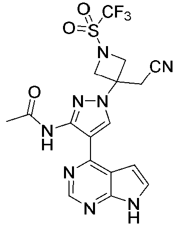 | LC-MS(ESI), m/z: [M+H]⁺ =468.8. |
| | ¹H-NMR(400MHz, CD₃OD) δ 8.88 (s, 1H), 8.81 (s, 1H), 7.70 (s, 1H), 7.06 (d, J = 3.7 Hz, 1H), 5.02 (d, J = 9.3 Hz, 2H), 4.66 (d, J = 9.3 Hz, 2H), 3.68 (s, 2H), 2.20 (s, 2H). |

| Example 6 : 2-(3-(3-(ethylamino)-4-(7H-pyrrolo[2,3-d]pyrimidin-4-yl)-1H-pyrazol-1-yl)-1-((trifluoromethyl)sulfonyl)azetidine-3-yl)acetonitrile | |
|---|---|
| 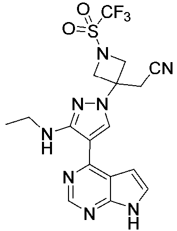 | LC-MS (ESI), m/z: [M+H]⁺ = 454.9. |
| | ¹H-NMR(400MHz, DMSO-*d*6) δ 8.70 (s, 1H), 8.66 (s, 1H), 7.60 (d, J = 3.6 Hz, 1H), 7.09 (d, J = 3.6 Hz, 1H), 4.93 (d, J = 8.9 Hz, 2H), 4.60 (d, J = 9.0 Hz, 2H), 3.76 (s, 2H), 3.35 (q, J = 7.1 Hz, 2H), 1.24 (t, J = 7.1 Hz, 3H). |

| Example 7: 2-(3-(3-((2-(dimethylamino)ethyl)amino)-4-(7H-pyrrolo[2,3-d]pyrimidin-4-yl)-1H-pyrazol-1-yl)-1-((trifluoromethyl)sulfonyl)azetidine-3-yl)acetonitrile | |
|---|---|
| 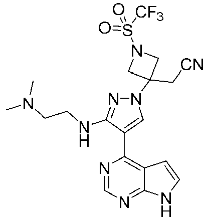 | LC-MS(ESI), m/z: [M+H]⁺ =498.0. |
| | ¹H-NMR(400MHz, CD₃OD) δ 8.72 (s, 1H), 8.63 (s, 1H), 7.50 (d, J = 3.6 Hz, 1H), 7.00 (d, J = 3.6 Hz, 1H), 5.00 (d, J = 9.0 Hz, 2H), 4.60 (d, J = 9.1 Hz, 2H), 3.85 (t, J = 5.8 Hz, 2H), 3.67 (s, 2H), 3.58 (t, J = 5.7 Hz, 2H), 3.00 (s, 6H). |

| Example 7A: 2-(3-(3-((2-(pyrrolidin-1-yl)ethyl)amino)-4-(7H-pyrrolo[2,3-d]pyrimidin-4-yl)-1H-pyrazol-1-yl)-1-((trifluoromethyl)sulfonyl)azetidine-3-yl)acetonitrile | |
|---|---|
| 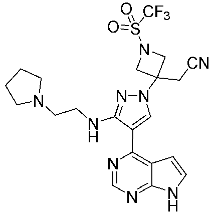 | LC-MS(ESI), m/z: [M+H]⁺ =524.1. |

| Example 7B : 2-(3-(3-((2-morpholinethyl)amino)-4-(7H-pyrrolo[2,3-d]pyrimidin-4-yl)-1H-pyrazol-1-yl)-1-((trifluoromethyl)sulfonyl)azetidine-3-yl)acetonitrile | |
|---|---|
| 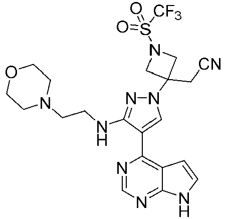 | LC-MS(ESI), m/z: [M+H]⁺ =540.2. |

| Example 7C : 2-(3-(3-((2-(4-methylpiperazin-1-yl)ethyl)amino)-4-(7H-pyrrolo[2,3-d]pyrimidin-4-yl)-1H-pyrazol-1-yl)-1-((trifluoromethyl)sulfonyl)azetidine-3-yl)acetonitrile | |
|---|---|
| 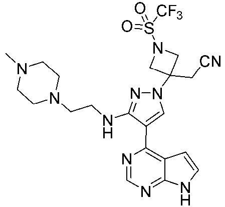 | LC-MS(ESI), m/z: [M+H]⁺ =553.2_{∘} |

| Example 7D: 2-(3-(3-((2-oxo-2-(piperidin-1-yl)ethyl)amino)-4-(7H-pyrrolo[2,3-d]pyrimidin-4-yl)-1H-pyrazol-1-yl)-1-((trifluoromethyl)sulfonyl)azetidine-3-yl)acetonitrile | |
|---|---|
| 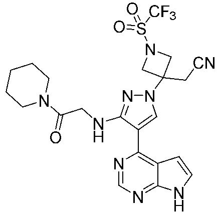 | LC-MS(ESI), m/z: [M+H]⁺ =552.2. |

| Example 8 : 2-(3-(3-amino-4-(7H-pyrrolo[2,3-d]pyrimidin-4-yl)-1H-pyrazol-1-yl)-1-((methylsulfonyl)methyl)sulfonyl)azetidine-3-yl)acetonitrile | |
|---|---|
| 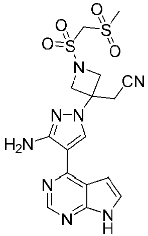 | LC-MS (ESI), m/z: [M+H]⁺ = 450.8_{∘} |
| | ¹H-NMR(400MHz, DMSO-d6) δ 12.05 (d, J = 1.0 Hz, 1H), 8.63 (s, 1H), 8.58 (s, 1H), 7.53 (d, J = 3.6 Hz, 1H), 7.05 (d, J = 3.6 Hz, 1H), 6.38 (s, 2H), 5.54 (s, 2H), 4.69 (d, J = 9.2 Hz, 2H), 4.32 (d, J = 9.2 Hz, 2H), 3.55 (s, 2H), 3.17 (s, 3H)_{∘} |

| Example 9 : 2-((3-(3-amino-4-(7H-pyrrolo[2,3-d]pyrimidin-4-yl)-1H-pyrazol-1-yl)-3-(cyanomethyl)azetidine-1-yl sulfonyl)acethydrazide | |
|---|---|
| 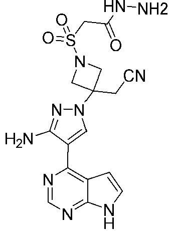 | LC-MS (ESI), m/z: [M+H]⁺ = 430.9. |
| | ¹H-NMR(400MHz, DMSO-*d*6) δ 12.08 (s, 1H), 9.50 (s, 1H), 8.67 (s, 2H), 7.59 - 7.54 (m, 1H), 7.12 (d, J = 2.4 Hz, 1H), 6.42 (s, 2H), 4.69 (d, J = 9.0 Hz, 2H), 4.49 (s, 2H), 4.32 (d, J = 9.0 Hz, 2H), 4.24 (s, 2H), 3.64 (s, 2H). |

### Example 10: 3-(3-amino-4-{7H-pyrrolo[2,3-d]pyrimidin-4-yl}pyrazol-1-yl)-3-(cyanomethyl)-N-methylazetidine-1-formamide

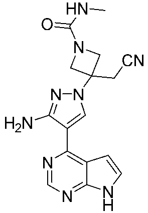

### Synthesis route:

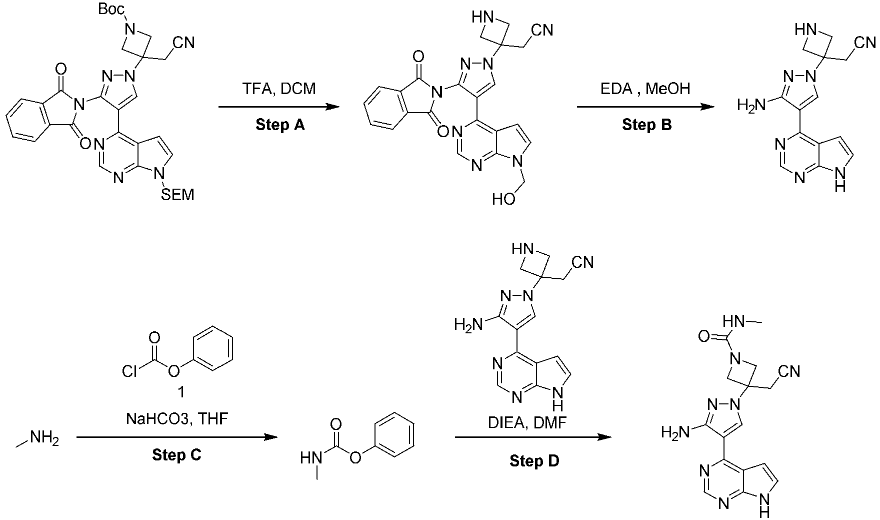

### Step A: 2-{3-[3-(1,3-dioxoindole)-4-[7-(methylol)pyrrolidine[2,3-d]pyrimidine]pyrazole] 3-azetidine}acetonitrile

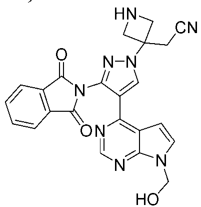

tert-butyl 3-(cyanomethyl)-3-[3-(1,3-dioisoxoindol-2-yl)-4-(7-{[2-(trimethylsilyl)ethoxy]methyl}pyrrolo[2,3-d]pyrimidin-4-yl)pyrazol-1-yl]azetidine-1-carboxylate (10.1 g, 0.015mol) was added to dichloromethane(200 mL) at room temperature. After sufficient dissolution, trifluoroacetic acid (100 mL) was added, and stirring was carried out overnight in an oil bath at 30 °C. After rotary drying, the product 2-{3-[3-(1,3-dioxoisoindol)-4-[7-(methylol)pyrrolidine[2,3-d]pyrimidine]pyrazole]3-azetidine } acetonitrile was obtained (12.0 g, crude).

### LC-MS (ESI), m/z: [M+H]⁺ = 455.0.

### Step B: 2-[3-(3-amino-4-{7H-pyrrolo[2,3-d]pyrimidin-4-yl}pyrazol-1-yl)azetidine-3-yl]acetonitrile

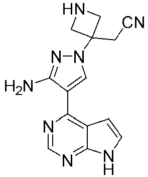

2-{3-[3-(1,3-dioxoisoindol)-4-[7-(methylol)pyrrolidine[2,3-d]pyrimidine]pyrazole]3-azetidine}acetonitrile (12 g, 0.026 mol) was added to methanol (100 mL) at room temperature. After sufficient dissolution, ethylenediamine (4.68 g, 0.078 mol) was added. The reaction was carried out for 4h at room temperature. The reaction solution was diluted with water (100 mL), extracted with ethyl acetate (100 mL * 5). The organic phase was collected. After dring and concentrating under reduced pressure, the residue was purified using a C18 column to obtain the product 2-[3-(3-amino-4-{7H-pyrrolo[2,3-d]pyrimidin-4-yl}pyrazol-1-yl)azetidine-3-yl]acetonitrile (1.9 g, 25.1%).

### LC-MS (ESI), m/z: [M+H]⁺ = 295.0.

### Step C: phenyl N-methyl carbamate

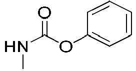

Sodium bicarbonate (2.77 g,0.03 mol) was added to a solution of methylamine (2 g, 0.03 mol) in tetrahydrofuran (15 mL) at 25 °C. The reaction mixture was stirred at 25 °C for 15 minutes. Phenyl chloroformate (4.5 g, 0.03 mol) was added, and the reaction mixture was stirred at 25 °C for 2 hours. The reaction solution was quenched with water (30 mL) and extracted with ethyl acetate (20 mL * 3). The organic phase was dried and concentrated. The concentrated residue of the reaction solution was passed through column chromatography (DCM: MeOH=20:1) to obtain the product (1g, 22%).

### LC-MS (ESI), m/z: [M+H]⁺ = 152.1.

### Step D: 3-(3-amino-4-{7H-pyrrolo[2,3-d]pyrimidin-4-yl}pyrazol-1-yl)-3-(cyanomethyl)-N-methylazetidine-1-formamide

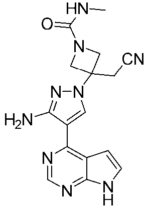

Phenyl N-methyl carbamate (51 mg, 0.339 mmol) and 2-[3-(3-amino-4-{7H-pyrrolo[2,3-d]pyrimidin-4-yl}pyrazol-1-yl)azetidine-3-yl]acetonitrile (99.77 mg, 0, 339 mmol) were added into N,N-dimethylformamide (2.5 mL). Stirring was carried out for 5 minutes. N,N-diisopropylethylamine (131 mg, 1.017 mmol) was added. The reaction mixture was stirred at room temperature for 16 hours. The reaction solution was concentrated. The residue was passed through Prep-HPLC (chromatographic column type: gemini-C18 150 x 21.2 mm, 5µm, mobile phase: ACN-H2O (0.1% TFA), gradient 25%-30%, flow rate: 20 mL/min) to obtain the product (20 mg, 16.72 %).

### LC-MS (ESI), m/z: [M+H]⁺ = 352.0.

¹H-NMR(400MHz, DMSO-*d*6) δ 12.07 (s, 1H), 8.66 (s, 1H), 8.57 (s, 1H), 7.59 - 7.52 (m, 1H), 7.11 - 7.05 (m, 1H), 6.50 (d, J = 4.5 Hz, 1H), 6.38 (s, 2H), 4.38 (d, J = 9.0 Hz, 2H), 4.03 (d, J = 8.9 Hz, 2H), 3.58 (s, 2H), 2.57 (d, J = 4.5 Hz, 3H).

The following examples were prepared according to the experimental route and method in Example 10:

| Example 11: 3-(3-amino-4-(7H-pyrrolo[2,3-d]pyrimidin-4-yl)-1H-pyrazol-1-yl)-3-(cyanomethyl)-N-(2,2,2-trifluoroethyl)azetidine-1-formamide | |
|---|---|
| 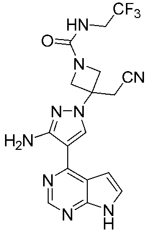 | LC-MS (ESI), m/z: [M+H]⁺ =419.9. |
| | ¹H-NMR(400MHz, DMSO-*d*6) δ 14.27 (s, 1H), 10.84 (s,1H), 10.77 (s, 1H), 9.74 (dd, J = 3.4, 2.6 Hz, 1H), 9.48 (t, J = 6.3 Hz, 1H), 9.27 (dd, J = 3.6, 1.8 Hz, 1H), 6.64 (d, J = 9.2 Hz, 2H), 6.50-6.40(brs, 2H), 6.30 (d, J = 9.2 Hz, 2H), 6.05 - 5.96 (m, 2H), 5.80 (s, 2H). |

| Example 12: 3-(3-amino-4-(7H-pyrrolo[2,3-d]pyrimidin-4-yl)-1H-pyrazol-1-yl)-3- | |
|---|---|
| (cyanomethyl)-N-cyclopropylazetidine-1-formamide | |
| 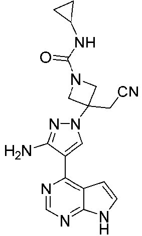 | LC-MS(ESI), m/z: [M+H]⁺ =378.0. |
| | ¹H-NMR(400MHz, DMSO-*d*6) δ 12.08 (s, 1H), 8.61 (d, J = 39.2 Hz, 1H), 8.41 (s, 1H), 7.55 (d, J = 3.0 Hz, 1H), 7.08 (s, 1H), 6.71 (s, 1H), 6.38 (s, 2H), 4.37 (d, J = 9.0 Hz, 2H), 4.02 (d, J = 9.1 Hz, 2H), 3.57 (s, 2H), 0.85 (s, 1H), 0.56 (m, 2H), 0.39 (m, 2H). |

| Example 13: 2-(3-(3-amino-4-(7H-pyrrolo[2,3-d]pyrimidin-4-yl)-1H-pyrazol-1-yl)-1-(3,3-difluoroazetidine-1-carbonyl)azetidin-3-yl)acetonitrile | |
|---|---|
| 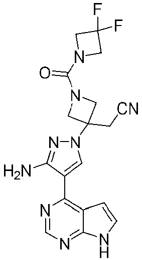 | LC-MS(ESI), m/z: [M+H]⁺ =414.0. |
| | ¹H-NMR(400MHz, DMSO-*d*6) δ 12.09 (s, 1H), 8.62 (d, J = 30.2 Hz, 1H), 8.45 (s, 1H), 7.56 (m, 1H), 7.09 (d, J = 1.9 Hz, 1H), 6.40 (s, 2H), 4.52 (d, J = 9.3 Hz, 2H), 4.35 (t, J = 12.8 Hz, 4H), 4.16 (d, J = 9.2 Hz, 2H), 3.59 (s, 2H). |

| Example 14: 3-(3-amino-4-(7H-pyrrolo[2,3-d]pyrimidin-4-yl)-1H-pyrazol-1-yl)-3-(cyanomethyl)-N-ethylazetidine-1-formamide | |
|---|---|
| 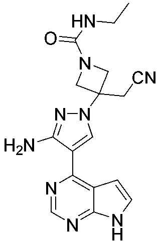 | LC-MS (ESI), m/z: [M+H]⁺ = 366.0. |
| | ¹H-NMR(400MHz, DMSO-*d*6) δ 12.03 (s, 1H), 8.62 (s, 1H), 8.53 (s, 1H), 7.51 (dd, J = 3.5, 2.5 Hz, 1H), 7.04 (dd, J = 3.6, 1.8 Hz, 1H), 6.52 (t, J = 5.6 Hz, 1H), 6.34 (s, 2H), 4.34 (d, J = 9.0 Hz, 2H), 3.99 (d, J = 9.0 Hz, 2H), 3.54 (s, 2H), 3.17 - 2.80 (m, 2H), 0.97 (t, J = 7.1 Hz, 3H). |

| Example 15: 3-(3-amino-4-(7H-pyrrolo[2,3-d]pyrimidin-4-yl)-1H-pyrazol-1-yl)-3-(cyanomethyl)-N-isopropylaza-1-formamide | |
|---|---|
| 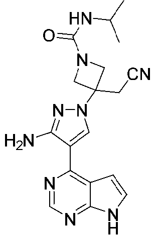 | LC-MS (ESI), m/z: [M+H]⁺ = 380.3. |
| | ¹H-NMR(400MHz, DMSO-*d*6) δ 12.03 (s, 1H), 8.62 (s, 1H), 8.52 (s, 1H), 7.51 (dd, J = 3.4, 2.5 Hz, 1H), 7.04 (dd, J = 3.6, 1.7 Hz, 1H), 6.34 (s, 2H), 6.29 (d, J = 7.9 Hz, 1H), 4.33 (d, J = 9.0 Hz, 2H), 3.99 (d, J = 9.0 Hz, 2H), 3.82 - 3.61 (m, 1H), 3.54 (s, 2H), 1.01 (d, J = 6.6 Hz, 6H). |

| Example 16: (S)-3-(3-amino-4-(7H-pyrrolo[2,3-d]pyrimidin-4-yl)-1H-pyrazol-1-yl)-3- | |
|---|---|
| (cyanomethyl)-N-(1,1,1-trifluoropropan-2-yl)azetidine-1-formamide | |
| 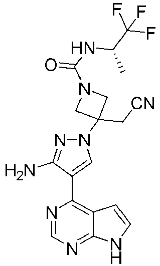 | LC-MS(ESI), m/z: [M+H]⁺ =434.0. |
| | ¹H-NMR(400MHz, CD₃OD) δ 8.75 (s, 1H), 8.53 (s, 1H), 7.58 (d, J = 3.7 Hz, 1H), 7.06 (d, J = 3.6 Hz, 1H), 4.55 (dd, J = 9.3, 5.9 Hz, 2H), 4.48 (dt, J = 14.4, 7.1 Hz, 1H), 4.29 (dd, J = 12.1, 9.3 Hz, 2H), 3.54 (d, J = 28.8 Hz, 2H), 1.32 (d, J = 7.1 Hz, 3H). |

| Example 17: 3-(3-amino-4-(7H-pyrrolo[2,3-d]pyrimidin-4-yl)-1H-pyrazol-1-yl)-3-(cyanomethyl)-N-(2-hydroxyethyl)azetidine-1-formamide | |
|---|---|
| 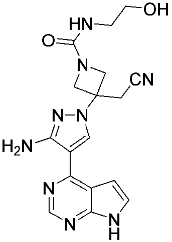 | LC-MS (ESI), m/z: [M+H]⁺ = 382.1. |
| | ¹H-NMR(400MHz, CD₃OD) δ 8.70 (s, 1H), 8.49 (s, 1H), 7.47 (d, J = 3.6 Hz, 1H), 6.98 (d, J = 3.6 Hz, 1H), 4.55 (d, J = 9.1 Hz, 2H), 4.26 (d, J = 9.2 Hz, 2H), 3.61 (t, J = 5.8 Hz, 2H), 3.49 (s, 2H), 3.30 (t, J = 5.8 Hz, 2H). |

| Example 18: 3-(3-amino-4-(7H-pyrrolo[2,3-d]pyrimidin-4-yl)-1H-pyrazol-1-yl)-N,3-bis(cyanomethyl)azetidine-1-formamide | |
|---|---|
| 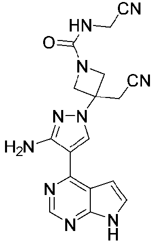 | LC-MS(ESI), m/z: [M+H]⁺ =377.0. |
| | ¹H-NMR(400MHz, DMSO-*d*6) δ 12.01 (s, 1H), 8.61 (s, 1H), 8.54 (s, 1H), δ 7.51 (dd, J = 3.5, 2.5 Hz, 1H), 7.32 (t, J = 5.8 Hz, 1H), 7.04 (dd, J = 3.6, 1.8 Hz, 1H), 6.36 (s, 2H), 4.41 (d, J = 9.1 Hz, 2H), 4.07 (d, J = 9.1 Hz, 2H), 4.04 (d, J = 5.7 Hz, 2H), 3.59 (s, 2H). |

| Example 19: 3-(3-amino-4-(7H-pyrrolo[2,3-d]pyrimidin-4-yl)-1H-pyrazol-1-yl)-3-(cyanomethyl)-N-(3-methoxy-1,2,4-thiadiazol-5-yl)azetidine-1-formamide | |
|---|---|
| 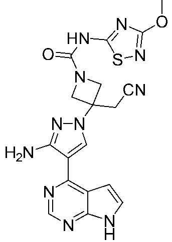 | LC-MS(ESI), m/z: [M+H]⁺ =452.0. |
| | ¹H-NMR(400MHz, CD₃OD) δ 8.72 (d, J = 2.1 Hz, 1H), 8.54 (s, 1H), 7.55 (t, J = 4.3 Hz, 1H), 7.04 (t, J = 2.9 Hz, 1H), 4.72 (d, J = 9.7 Hz, 2H), 4.43 (d, J = 9.7 Hz, 2H), 3.94 (s, 3H), 3.55 (s, 2H). |
| Example 20: (S)-3-(3-amino-4-(7H-pyrrolo[2,3-d]pyrimidin-4-yl)-1H-pyrazol-1-yl)-3- | |
| (cyanomethyl)-N-(2,2-difluorocyclopropyl)azetidine-1-formamide | |
| 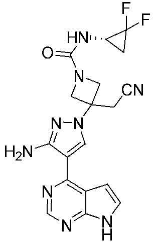 | LC-MS (ESI), m/z: [M+H]⁺ = 414.0. |
| | ¹H-NMR(400MHz, DMSO-*d*6) δ 12.02 (s, 1H), 8.62 (s, 1H), 8.53 (s, 1H),7.50 (d, J = 2.5 Hz, 1H), 7.03 (s, 2H), 6.34 (s, 2H), 4.40 (m, 2H), 4.05 (d, J = 9.1 Hz, 2H), 3.56 (s, 2H), 3.09 (m, 1H), 1.46-1.43 (m, 1H), 1.40-1.35 (m, 1H). |

### Example 21: 3-(3-(3-amino-4-(7H-pyrrolo[2,3-d]pyrimidin-4-yl)-1H-pyrazol-1-yl)-3-(cyanomethyl)azetidine-1-yl]-3-oxopropionitrile

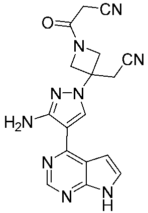

### Synthesis route:

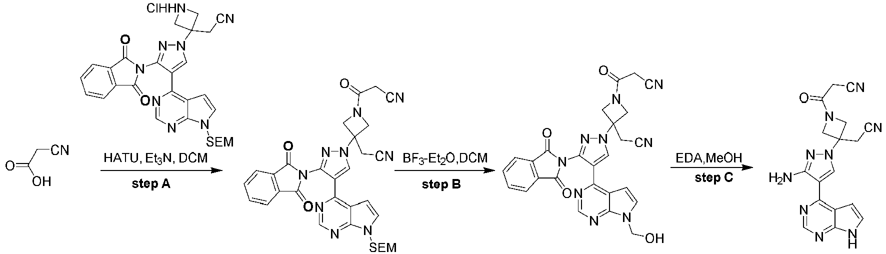

### Step A : 3-(3-(cyanomethyl)-3-(3-(1,3-dioxoisoindolin-2-yl)-4-(7-((2-(trimethylsilyl)ethoxy)methyl)-7H-pyrrolo[2,3-d]pyrimidin-4-yl)-1H-pyrazol-1-yl)azetidine-1-yl)-3-oxopropionitrile

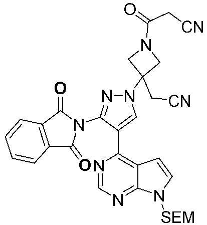

2-(7-azabenzotriazole)-N,N,N',N'-tetramethylurea hexafluorophosphate was added to a solution of 2-cyanoacetic acid (92 mg, 1.08 mmol, 2.0 eq) and triethylamine (220 mg, 2.2 mmol, 4.0 eq) in dichloromethane (5 mL). The reaction mixture was stirred at 25 °C for 0.5 hours. Then, 2-(3-(3-(1,3-dioxoisoindolin-2-yl)-4-(7-((2-(trimethylsilyl)ethoxy)methyl)-7H-pyrrolo[2,3-d]pyrimidin-4) -yl)-1H-pyrazol-1-yl)azetidine-3-yl)acetonitrile (300 mg, 0.54 mmol, 1.0eq) was added into the reaction mixture. The reaction solution was stirred at 25°C for 2h, diluted with water (20 mL), and then extracted with dichloromethane (20 mL*3). The combined organic phase was washed with saturated saline solution (20 mL), dried over anhydrous sodium sulfate, and evaporated under reduced pressure. The Crude product was passed through Combiflash column (dichloromethane: methanol=35:1) to obtain the product 3-(3-(cyanomethyl)-3-(3-(1,3-dioxoisoindolin-2-yl)-4-(7-(methylol)-7H-pyrrolo[2,3-d]pyrimidin-4-yl))-1H-pyrazol-1-yl)azetidine-1-yl)-3-oxopropionitrile (220 mg, yield: 65%) in white solid form.

### LC-MS(ESI), m/z: [M+H]⁺ =622.0.

### Step B: 3-(3-(cyanomethyl)-3-(3-(1,3-dioxoisoindolin-2-yl)-4-(7-(methylol)-7H-pyrrolo[2,3-d]pyrimidin-4-yl) )-1H-pyrazol-1-yl)azetidine-1-yl)-3-oxopropionitrile

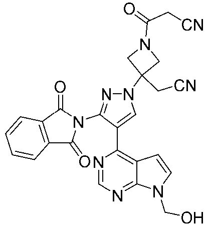

3-(3-(cyanomethyl)-3-(3-(1,3-dioxoisoindolin-2-yl)-4-(7-((2-(trimethylsilyl)ethoxy)methyl)-7H-pyrrolo[2,3-d]pyrimidin-4-yl)-1H-pyrazol-1-yl)azetidine-1-yl)-3-oxopropionitrile (220 mg, 0.35 mmol, 1.0eq) was added to dichloromethane (5 mL) at room temperature, and boron trifluoride ether solution (0.5 ml) was added at 0 °C. The reaction solution was stirred at 25°C for 2h, diluted with water (20 mL), and then extracted with ethyl acetate (10 mL*3). The organic layer was dried over anhydrous sodium sulfate, and evaporated under reduced pressure to obtain the crude product (140 mg, yield: 76%).

### LC-MS(ESI), m/z: [M+H]⁺ =522.0.

### Step C: 3-(3-(3-amino-4-(7H-pyrrolo[2,3-d]pyrimidin-4-yl)-1H-pyrazol-1-yl)-3-(cyanomethyl)azetidine-1-yl) -3-oxopropionitrile

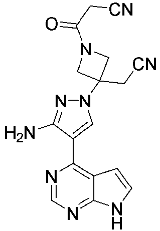

3-(3-(cyanomethyl)-3-(3-(1,3-dioxoisoindolin-2-yl)-4-(7-(methylol)-7H-pyrrolo[2,3-d]pyrimidin-4-yl))-1H-pyrazol-1-yl)azetidine-1-yl)-3-oxopropionitrile (140 mg, 0.27 mmol, 1.0eq) was dissolved in methanol (5 mL). Under an ice bath, ethylenediamine (0.5 mL) was added in order to adjust the pH to 8-9. The reaction system was stirred at 25 °C for 1 h. The reaction solution was concentrated, and then passed through Prep-HPLC (chromatographic column type: gemini-C18 150 x 21.2 mm, 5µm, mobile phase: ACN-H2O (0.1% TFA), gradient 25% -40%, flow rate: 20 mL/min) to obtain the product (31.9 mg, 33%).

### LC-MS(ESI), m/z: [M+H]⁺ =362.0.

¹H-NMR(400MHz, DMSO-*d*6) δ 8.63 (d, J = 9.9 Hz, 1H), 8.57 (d, J = 7.4 Hz, 1H), 7.54 (d, J = 5.3 Hz, 1H), 7.03 (d, J = 9.7 Hz, 1H), 4.67 (d, J = 12.9 Hz, 1H), 4.52 (d, J = 10.6 Hz, 1H), 4.38 (d, J = 10.6 Hz, 1H), 4.14 (d, J = 12.1 Hz, 1H), 3.78 (s, 2H), 3.56 (d, J = 9.3 Hz, 2H).

The following examples were prepared according to the experimental route and method in Example 21:

| | |
|---|---|
| Example 22 : 2-(3-(3-amino-4-(7H-pyrrolo[2,3-d]pyrimidin-4-yl)-1H-pyrazol-1-yl)-1-(cyclopropanecarbonyl)azetidine-3-yl)acetonitrile | |
| 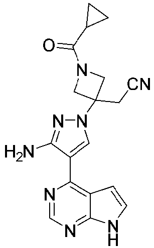 | LC-MS(ESI), m/z: [M+H]⁺ =363.1. ¹H-NMR(400 MHz, CD₃OD) δ 8.88 (s, 1H), 8.70 (s, 1H), 7.76 (d, J = 7.0 Hz, 1H), 7.22 (d, J = 3.6 Hz, 1H), 4.99 (d, J = 11.2 Hz, 2H), 4.61 (d, J = 10.6 Hz, 2H), 3.66 (s, 2H), 3.07 (m, 1H), 2.91 (d, J = 13.0 Hz, 1H), 2.71 (d, J = 14.4 Hz, 2H), 1.33 (m, J = 9.1, 5.5 Hz, 1H). |
| Example 23: 2-(1-alaninyl-3-(3-amino-4-(7H-pyrrolo[2,3-d]pyrimidin-4-yl)-1H-pyrazol-1-yl)azetidine-3-yl)acetonitrile | |
| 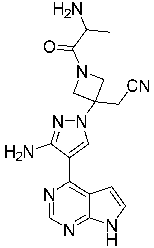 | LC-MS(ESI), m/z: [M+H]⁺ =366.0. ¹H-NMR(400 MHz, CD₃OD) δ 8.62 (d, J = 2.3 Hz, 1H), 8.44 (d, J = 6.9 Hz, 1H), 7.41 (t, J = 3.5 Hz, 1H), 6.91 (dd, J = 10.0, 3.6 Hz, 1H), 4.85 (dd, J = 9.8, 5.3 Hz, 1H), 4.58 (m, 2H), 4.31 (dd, J = 24.1, 11.0 Hz, 1H), 4.05 (m, 1H), 3.45 (d, J = 11.8 Hz, 2H), 1.41 (dd, J = 14.7, 7.0 Hz, 3H). |
| Example 24 : 2-(3-(3-amino-4-(7H-pyrrolo[2,3-d]pyrimidin-4-yl)-1H-pyrazol-1-yl)-1-(1-fluorocyclopropane-1-carbonyl)azetidine-3-yl)acetonitrile | |
| 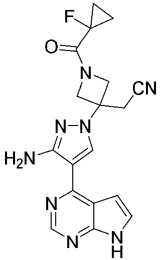 | LC-MS(ESI), m/z: [M+H]⁺ =381.0. |
| | ¹H-NMR(400 MHz, DMSO-*d*6) δ 8.71 (s, 1H), 8.67 (s, 1H), 7.61 (d, J = 3.6 Hz, 1H), 7.14 (d, J = 3.6 Hz, 1H), 4.98 (d, J = 8.2 Hz, 1H), 4.68 (d, J = 6.1 Hz, 1H), 4.58 (d, J = 10.5 Hz, 1H), 4.27 (d, J = 10.6 Hz, 1H), 3.65 (d, J = 21.7 Hz, 2H), 1.34 (m, 2H), 1.24 (d, J = 8.6 Hz, 2H). |
| Example 25 : 2-(3-(3-amino-4-(7H-pyrrolo[2,3-d]pyrimidin-4-yl)-1H-pyrazol-1-yl)-1-(1-(trifluoromethyl)cyclopropane-1-carbonyl)azetidine-3-yl)acetonitrile | |
| 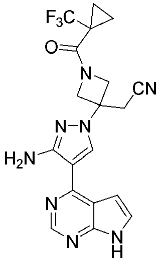 | LC-MS (ESI), m/z: [M+H]⁺ = 430.9. |
| | ¹H-NMR(400 MHz, DMSO-*d*6) δ 12.08 (s, 1H), 8.67 (s, 1H), 8.62 (s, 1H), 7.57 (dd, J = 3.3, 2.3 Hz, 1H), 7.09 (dd, J = 3.5, 1.3 Hz, 1H), 6.42 (s, 2H), 4.87 - 4.23 (m, 4H), 3.63 (s, 2H), 1.31 (s, 2H), 1.23 (s, 2H). |
| Example 26 : 1-(3-(3-amino-4-(7H-pyrrolo[2,3-d]pyrimidin-4-yl)-1H-pyrazol-1-yl)-3-(cyanomethyl)azetidine-1-carbonyl)cyclopropane-1-nitrile | |
| 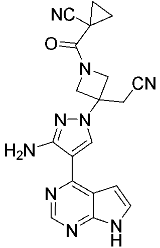 | LC-MS(ESI), m/z: [M+H]⁺ =388.0. |
| | ¹H-NMR(400 MHz, DMSO-*d*6) δ 12.13 (s, 1H), 8.68 (s, 1H), 8.66 (s, 1H), 7.58 (s, 1H), 7.11 (dt, J = 11.7, 5.8 Hz, 1H), 5.07 (d, J = 9.2 Hz, 1H), 4.81 (d, J = 9.2 Hz, 1H), 4.60 (d, J = 10.3 Hz, 1H), 4.24 (d, J = 10.3 Hz, 1H), 3.68 (s, 2H), 1.62 (d, J = 3.6 Hz,2H), 1.54 (d, J = 3.6 Hz, 2H). |
| Example 27 : 2-(3-(3-amino-4-(7H-pyrrolo[2,3-d]pyrimidin-4-yl)-1H-pyrazol-1-yl)-1-(2,2,3,3-pentafluoropropyl)azetidine-3-yl)acetonitrile | |
| 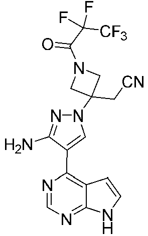 | LC-MS (ESI), m/z: [M+H]⁺ = 440.9. |
| | ¹H-NMR(400 MHz, CD₃OD) δ 8.59 (s, 1H), 8.47 (d, J = 13.7 Hz, 1H), 7.36 (d, J = 3.6 Hz, 1H), 6.90 (d, J = 3.6 Hz, 1H), 5.04 (d, J = 10.6 Hz, 1H), 4.72 (d, J = 11.4 Hz, 2H), 4.41 (d, J = 11.3 Hz, 1H), 3.47 (s, 2H). |
| Example 28 : 2-(3-(3-amino-4-(7H-pyrrolo[2,3-d]pyrimidin-4-yl)-1H-pyrazol-1-yl)-1-(dimethylglycine)azetidine-3-yl)acetonitrile | |
| 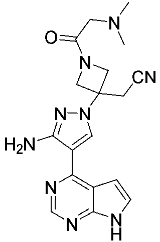 | LC-MS(ESI), m/z: [M+H]⁺ =380.0. |
| | ¹H-NMR(400 MHz, DMSO-*d*6) δ 8.66 (m, 1H), 8.54 (m, 1H), 7.50 (m, 1H), 6.98 (m, 1H), 4.67 (t, J = 16.6 Hz, 1H), 4.53 (m, 1H), 4.40 (d, J = 9.6 Hz, 1H), 4.22 (m, 1H), 3.99 (m, 2H), 3.52 (s, 2H), 2.75 (s, 6H). |
| Example 29 : 2,2'-(3-(3-amino-4-(7H-pyrrolo[2,3-d]pyrimidin-4-yl)-1H-pyrazol-1-yl)azetidine-1,3-diyl)diacetonitrile | |
| 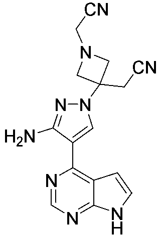 | LC-MS (ESI), m/z: [M+H]⁺ = 334.0. |
| | ¹H-NMR(400 MHz, CD₃OD) δ 8.74 (s, 1H), 8.51 (s, 1H), 7.59 (d, J = 3.7 Hz, 1H), 7.07 (d, J = 3.7 Hz, 1H), 3.90 (d, J = 8.9 Hz, 2H), 3.76 - 3.72 (m, 4H), 3.45 (s, 2H). |
| Example 30: 2-(3-(3-amino-4-(7H-pyrrolo[2,3-d]pyrimidin-4-yl)-1H-pyrazol-1-yl)-1-(3,3,3-trifluoropropyl)azetidine-3-yl)acetonitrile | |
| 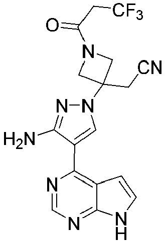 | LC-MS (ESI), m/z: [M+H]⁺ = 405. |
| | ¹H-NMR(400 MHz, DMSO-*d*6) δ 12.13 (s, 1H), 8.68 (s, 1H), 8.62 (s, 1H), 7.58 (dd, J = 3.4, 2.5 Hz, 1H), 7.10 (dd, J = 3.5, 1.7 Hz, 1H), 4.76 (d, J = 9.6 Hz, 1H), 4.58 (d, J = 10.7 Hz, 1H), 4.47 (d, J = 9.6 Hz, 1H), 4.21 (d, J = 10.7 Hz, 1H), 3.62 (d, J = 1.5 Hz, 2H), 3.47 (dd, J = 11.2, 2.0 Hz, 2H). |
| Example 31 : 2-(3-(3-amino-4-(7H-pyrrolo[2,3-d]pyrimidin-4-yl)-1H-pyrazol-1-yl)-1-((1R, 2S)-2-fluorocyclopropane-1-carbonyl)azetidine-3-yl)acetonitrile | |
| 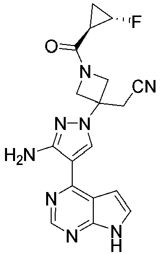 | LC-MS(ESI), m/z: [M+H]⁺ =381.0. |
| | ¹H-NMR(400 MHz, DMSO-*d*6) δ 8.73 (s, 1H), 8.64 (d, J = 4.6 Hz, 1H), 7.62 (d, J = 3.5 Hz, 1H), 7.11 (t, J = 3.4 Hz, 1H), 4.86 (dd, J = 43.3, 34.6 Hz, 2H), 4.61 (d, J = 9.6 Hz, 1H), 4.49 (dd, J = 10.6, 3.8 Hz, 1H), 4.19 (t, J = 10.3 Hz, 1H), 3.64 (d, J = 8.5 Hz, 2H), 2.23-2.19 (m, 1H), 1.49-1.44 (m, 1H), 1.18-1.14 (m, 1H). |
| Example 32 : 2-(3-(3-amino-4-(7H-pyrrolo[2,3-d]pyrimidin-4-yl)-1H-pyrazol-1-yl)-1-(3,3-difluorocyclobutane-1-carbonyl)azetidine-3-yl)acetonitrile | |
| 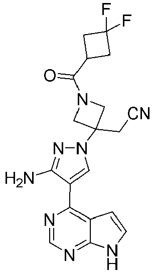 | LC-MS(ESI), m/z: [M+H]⁺=413.1. |
| | ¹H-NMR(400 MHz, DMSO-*d*6) δ 12.09 (s, 1H), 8.67 (s, 1H), 8.60 (s, 1H), 7.57 (m, 1H), 7.09 (dd, J = 3.5, 1.7 Hz, 1H), 4.71 (d, J = 9.5 Hz, 1H), 4.53 (d, J = 10.4 Hz, 1H), 4.38 (d, J = 9.5 Hz, 1H), 4.17 (d, J = 10.5 Hz, 1H), 3.62 (d, J = 1.4 Hz, 2H), 3.04 (m, 1H), 2.75 (m, 4H). |
| Example 33: (S) -2-(3-(3-amino-4-(7H-pyrrolo[2,3-d]pyrimidin-4-yl)-1H-pyrazol-1-yl)-1-(2-hydroxypropionyl)azetidine-3-yl)acetonitrile | |
| 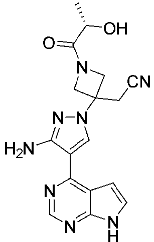 | LC-MS (ESI), m/z: [M+H]⁺ = 367.2. |
| | ¹H-NMR(400 MHz, DMSO-*d*6) δ 12.04 (s, 1H), 8.62 (s, 1H), 8.57 (s, 1H), 7.52 (d, J = 3.5 Hz, 1H), 7.06 (d, J = 3.5 Hz, 1H), 6.37 (s, 2H), 5.25(s, 1H), 4.81 (t, J = 9.9 Hz, 1H), 4.50 (dd, J = 20.4, 10.5 Hz, 2H), 4.12 (dd, J = 17.7, 7.5 Hz, 2H), 3.56 (s, 2H), 1.18 (dd, J = 6.6, 3.9 Hz, 3H). |
| Example 34 : (S) -2-(3-(3-amino-4-(7H-pyrrolo[2,3-d]pyrimidin-4-yl)-1H-pyrazol-1-yl)-1-(2,2-difluorocyclopropane-1-carbonyl)azetidine-3-yl)acetonitrile | |
| 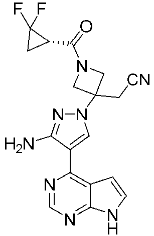 | LC-MS (ESI), m/z: [M+H]⁺ = 399.0. |
| | ¹H-NMR(400 MHz, DMSO-*d*6) δ 12.08 (s, 1H), 8.67 (s, 1H), 8.62 (s, 1H), 7.56 (d, J = 3.2 Hz, 1H), 7.10 (t,J = 3.6 Hz, 1H), 6.42 (s, 2H), 4.86 (dd, J = 32.9, 9.5 Hz, 1H), 4.66 - 4.44 (m, 2H), 4.21 (t, J = 10.2 Hz,1H), 3.66 (s, 2H), 2.89 - 2.72 (m, 1H), 2.03 - 1.82 (m, 2H). |
| Example 35: (1R, 2R)-2-(3-(3-amino-4-(7H-pyrrolo[2,3-d]pyrimidin-4-yl)-1H-pyrazol-1-yl)-3-(cyanomethyl)azetidine-1-carbonyl)cyclopropane-1-nitrile | |
| 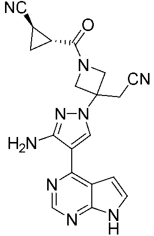 | LC-MS (ESI), m/z: [M+H]+ = 388.0. |
| | ¹H-NMR(400 MHz,DMSO-*d*6) δ 12.06 (s, 1H), 8.63 (d, J = 1.0 Hz, 1H), 8.57 (t, J = 6.7 Hz, 1H), 7.52 (dd, J = 11.5, 8.7 Hz, 1H), 7.05 (dd, J = 9.8, 8.0 Hz, 1H), 6.39 (s, 2H), 4.97 - 4.81 (m, 1H), 4.53 (m, 2H), 4.11 (d, 1H), 3.64 (m, 2H), 2.43 (m, 1H), 1.96 (m, 1H), 1.44 (m 1H), 1.25 (m, 1H). |

### Example 36: 2-(3-(3-amino-4-(7H-pyrrolo[2,3-d]pyrimidin-4-yl)-1H-pyrazol-1-yl)-1-(S-methylsulfonylimino)azetidine-3-yl)acetonitrile

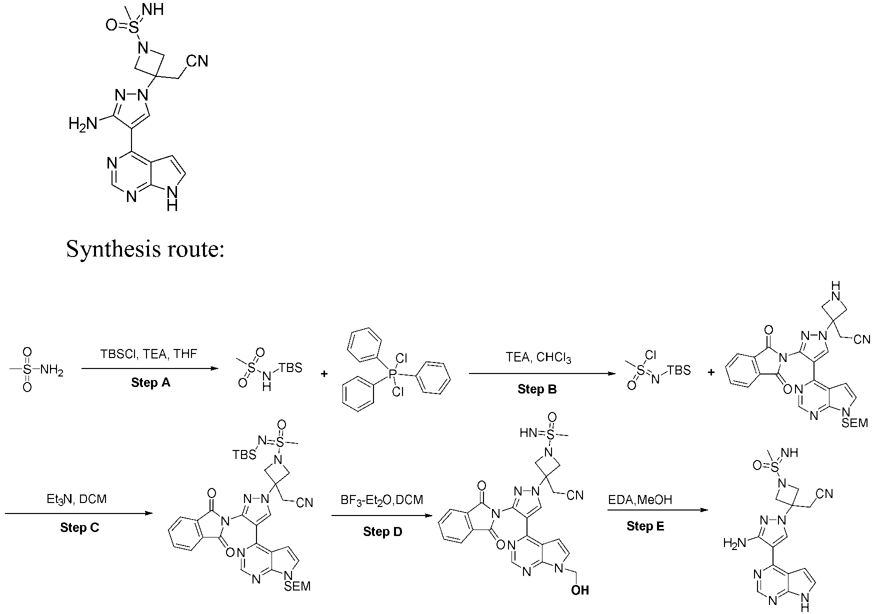

### Step A: N-(tert-butyldimethylsilyl)methanesulfonamide

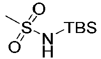

Methyl sulfonamide (1 g, 1.53 mmol, 1.0 eq) was dissolved in tetrahydrofuran (15 mL) under nitrogen protection, triethylamine (464 mg, 4.59 mmol, 3.0 eq) was added at room temperature, and a solution of tert-butyldimethylchlorosilane (462 mg, 3.06 mmol, 2.0 eq) in toluene (50 mL) was added dropwise. After the dropwise addition, the reaction was caried out overnight at room temperature. Filteration was carried out, and the solid was washed with ether (200 ml). Ether (20 mL) was added to the filtrate. After being left for 30 minutes, filteration was carried out again, and the filtrate was concentrated to obtain the crude product. The crude product was purified using Combiflash column (petroleum ether: ethyl acetate=1:1) to obtain the product N-(tert-butyldimethylsilyl)methanesulfonamide in the form of a white solid (1.2 g, yield: 39%).

### LC-MS(ESI), m/z: [M+H]⁺ =210.0.

### Step B&C: 2-(1-(N-(tert-butyldimethylsilyl)-S-methylsulfonylimino)-3-(3-(1,3-dioxoisoindolin-2-yl)-4-(7-((2-(trimethylsilyl)ethoxy))methyl)-7H-pyrrolo[2,3-d]pyrimidin-4-yl)-1H-pyrazol-1-yl)azetidine-3-yl)acetonitrile

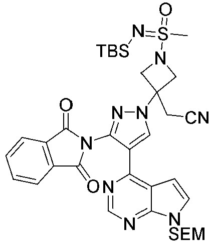

0.36 M suspension of dichlorotriphenylphosphine/chloroform (15 mL, 5.4 mmol, 6.0 eq) was cooled to 0 °C under nitrogen protection, and triethylamine (810 mg, 8.1 mmol, 9.0 eq) was added. After stirring for 15 min, N-(tert-butyldimethylsilyl)methanesulfonamide (1.13 g, 5.4 mmol, 6.0eq) was added at 0 °C. After stirring for 20 min, the reaction solution was added dropwise to a solution of 2-(3-(3-(1,3-dioxoisoindolin-2-yl))-4-(7-((2-(trimethylsilyl)ethoxy)methyl)-7H-pyrrolo[2,3-d]pyrimidin-4))-yl)-1-hydro-pyrazol-1-yl)azetidine-3-yl)acetonitrile (500 mg, 0.90 mmol, 1.0eq) in dichloromethane(2 mL). After the dropwise addition, the temperature was raised to room temperature, stirring was carried out for 2 hours, water was added to quench the reaction, and then extraction was carried out with ethyl acetate (10 mL * 3). The combined organic phase was washed with saturated saline solution (20 mL), dried over anhydrous sodium sulfate, and evaporated under reduced pressure. The crude product was passed through Combiflash column (dichloromethane: methanol =97:3) to purify it. A crude product in the form of a yellow solid was obtained (1.1 g, purity: 50%, yield: 82%).

### LC-MS(ESI), m/z: [M+H]⁺ =746.0.

### Step D: 2-(3-(3-(1,3-dioxoisoindolin-2-yl))-4-(7-(methylol)-7-hydro-pyrrolo[2,3-d]pyrimidin-4-yl)-1-hydro-pyrazol-1-yl)-1-(S-methylsulfonylimino)azetidine-3-yl)acetonitrile

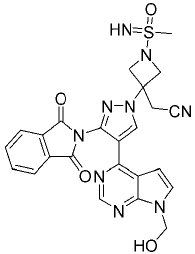

2-(1-(N-(tert-butyldimethylsilyl)-S-methylsulfonylimino)-3-(3-(1,3-dioxoisoindolin-2-yl)-4-(7-((2-(trimethylsilyl)ethoxy))methyl)-7H-pyrrolo[2,3-d]pyrimidin-4-yl)-1H-pyrazol-1-yl)azetidine-3-yl)acetonitrile (1.1 g, 1.34 mmol, 1.0eq) was added to dichloromethane (10 mL) at room temperatre, and boron trifluoride-ether solution (1 mL) was added at 0°C. The reaction solution was stirred at 25 °C for 2 h, diluted with water (10 mL) and extracted with ethyl acetate (10 mL * 3). The combined organic phase was washed with saturated saline solution (20 mL), dried over anhydrous sodium sulfate, and evaporated under reduced pressure to obtain the crude product (500 mg, crude).

### LC-MS(ESI), m/z: [M+H]⁺ =531.9.

### Step E: 2-(3-(3-amino-4-(7H-pyrrolo[2,3-d]pyrimidin-4-yl)-1-hydro-pyrazol-1-yl)-1-(S-methylsulfonylimino)azetidine-3-yl)acetonitrile

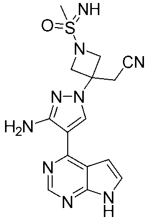

2-(3-(3-(1,3-dioxoisoindolin-2-yl))-4-(7-(methylol)-7-hydro-pyrrolo[2,3-d]pyrimidin-4-yl)-1-hydro-pyrazol-1-yl)-1-(S-methylsulfonylimino)azetidine-3-yl)acetonitrile (500 mg, crude) was dissolved in methanol (10 mL) at room temperature. Under an ice bath, ethylenediamine (0.5 mL) was added in order to adjust the pH to 8-9. The reaction system was stirred at 25 °C for 1 h. The reaction solution was concentrated, and then passed through Prep-HPLC (chromatographic column type: gemini-C18 150 x 21.2 mm, 5µm, mobile phase: ACN-H2O (0.1% TFA), gradient 30% -35%, flow rate: 20 mL/min) to obtain the product (76 mg, 26%).

### LC-MS(ESI), m/z: [M+H]⁺ =372.0.

¹H-NMR(400 MHz, DMSO-*d*6) δ 11.96 (d, 1H), 8.66 (s, 1H), 8.62 (s, 1H), 7.56 (d, J = 3.6 Hz, 1H), 7.12 (d, J = 3.6 Hz, 1H), 6.40 (s, 2H), 4.43 (dd, J = 11.7, 9.3 Hz, 2H), 4.03 (d, J = 9.6 Hz, 2H), 3.92 (s, 1H), 3.53 (s, 2H), 2.96 (s, 3H).

The following examples were prepared according to the experimental route and method in Example 36:

| | |
|---|---|
| Example 37 : 2-(3-(3-amino-4-(7H-pyrrolo[2,3-d]pyrimidin-4-yl)-1H-pyrazol-1-yl)-1-(N,S-dimethylsulfonylimino)azetidine-3-yl)acetonitrile | |
| 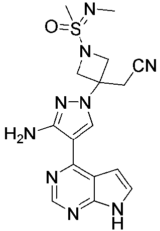 | LC-MS(ESI), m/z: [M+H]⁺ =386.0. |
| | ¹H-NMR(400MHz, CD₃OD) δ 8.74 (s, 1H), 8.56 (s, 1H), 7.56 (d, J = 3.6 Hz, 1H), 7.05 (d, J = 3.7 Hz, 1H), 4.76 (dd, J = 17.6, 9.3 Hz, 2H), 4.36 (t, J = 8.6 Hz, 2H), 3.62 (s, 2H), 3.41 (s, 3H), 3.26 (s, 3H), 2.82 (s, 3H). |
| Example 38: 2-(3-(3-amino-4-(7H-pyrrolo[2,3-d]pyrimidin-4-yl)-1H-pyrazol-1-yl)-1-(ethylsulfonylimino)azetidine-3-yl)acetonitrile | |
| 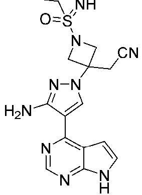 | LC-MS(ESI), m/z: [M+H]⁺ =386.0. |
| | ¹H-NMR(400MHz, CD₃OD) δ 8.77 (s, 1H), 8.58 (dd, J = 3.4, 1.3 Hz, 1H), 7.62 (dd, J = 5.3, 3.2 Hz, 1H), 7.09 (m, 1H), 4.68 (m, 2H), 4.29 (dd, J = 14.1, 7.3 Hz, 2H), 3.56 (s, 2H), 3.40-3.34 (m, 2H), 1.42 (m, 3H). |
| Example 39 : 2-(3-(3-amino-4-(7H-pyrrolo[2,3-d]pyrimidin-4-yl)-1H-pyrazol-1-yl)-1-(prop-2-yl-sulfonylimino)azetidine-3-yl)acetonitrile | |
| 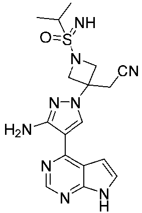 | LC-MS(ESI), m/z: [M+H]⁺ =400.0. |
| | ¹H-NMR(400MHz, CD₃OD) δ 8.79 (s, 1H), 8.60 (s, 1H), 7.64 (d, J = 2.5 Hz, 1H), 7.11 (d, J = 3.6 Hz, 1H), 4.72 (ddd, J = 17.9, 8.9, 4.7 Hz, 2H), 4.28 (m, 2H), 3.61 (m, 1H), 3.57 (s, 2H), 1.44 (dd, J = 6.7, 3.1 Hz, 6H). |

### Example 40 : 3-(3-amino-4-(7H-pyrrolo[2,3-d]pyrimidin-4-yl)-1H-pyrazol-1-yl)-3-(cyanomethyl)-N-(2,2,2-trifluoroethyl)azetidine-1-sulfonamide

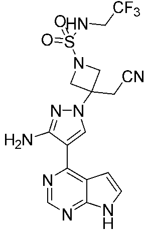

### Synthesis route:

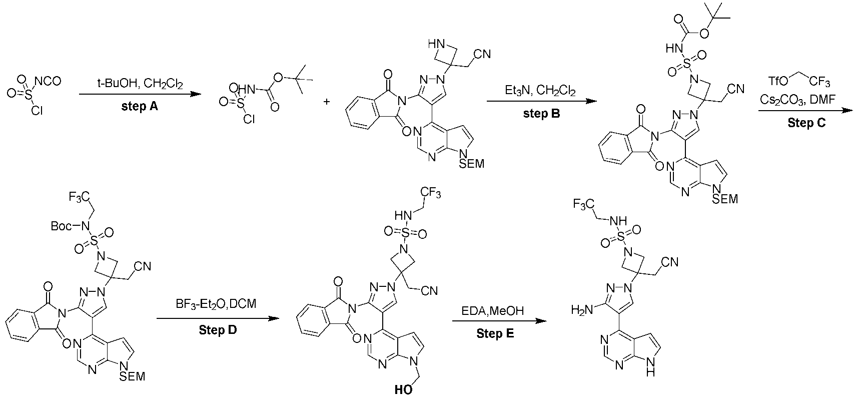

### Step A: tert-butyl (chlorosulfonyl)carbamate

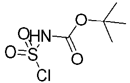

Under nitrogen protection, [(chlorosulfonyl)imino]ketone (4 g, 0.028 mol, 1.0 eq) was added to a solution of tert-butanol (2.73 g, 0.037 mol, 1.3eq) in dichloromethane (20 mL) at 0°C. The obtained reaction mixture as stirred at 25 °C for 20 minutes. After the reaction had been completed, it was concentrated to half of the original volume, freezed in the refrigerator under nitrogen protection for 50 minutes, and n-hexane (10 mL) was added. The reactants were filtered. The precipitate was washed with n-hexane, the filter cake was collected. After drying, the product tert-butyl (chlorosulfonyl)carbamate was obtained (2 g, crude), which was directly used in the next step.

### Step B: tert-butyl [3-(cyanomethyl)-3-[3-(1,3-dioxoisoindol-2-yl)-4-(7-{[2-(trimethylsilyl)ethoxy]methyl}pyrrolo[2,3-d]pyrimidin-4-yl)pyrazol-1-yl]azetidine-1-sulfonyl] carbamate

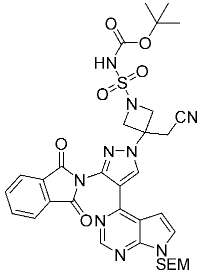

2-{3-[3-(1,3-dioxoindol-2-yl)-4-(7-{[2-(trimethylsilyl)ethoxy]methyl}pyrrolo[2,3-d]pyrimidin-4-yl) pyrazol-1-yl]azetidine-3-yl}acetonitrile (3 g, 5.4mmol, 1.0eq) was added to dichloromethane (20 mL) at 0°C. After dissolution, triethylamine (1.64, 16.2 mmol, 2.0 eq) was added. Stirring was carried out for 15 minutes. Then, tert-butyl (chlorosulfonyl)carbamate (1.75 g, 8.1 mmol, 1.5 eq) was added, and stirring was carried out at room temperature for 2 hours. After the reaction had been completed, dilution was carried out with water (50mL) and extraction with ethyl acetate (50 * 3 mL). The organic phase was collected, dried over anhydrous sodium sulfate, concentrated under reduced pressure, and the obtained residue was purified using a silica gel column (dichloromethane: methanol=10: 1) to obtain tert-butyl [3-(cyanomethyl)-3-[3-(1,3-dioxoisoindol-2-yl)-4-(7-{[2-(trimethylsilyl)ethoxy]methyl}pyrrolo[2,3-d]pyrimidin-4-yl)pyrazol-1-yl]azetidine-1-sulfonyl]carbamate (2.2 g, yield=71%).

### LC-MS (ESI), m/z: [M+H]⁺ = 733.9.

### Step C: tert-butyl ((3-(cyanomethyl)-3-(3-(1,3-dioxoisoindolin-2-yl)-4-(7-((2-(trimethylsilyl)ethoxy)methyl)-7H-pyrrolo[2,3-d]pyrimidin-4-yl)-1H-pyrazol-1-yl)azetidine-1-yl)sulfonyl)(2,2,2-trifluoroethyl)carbamate

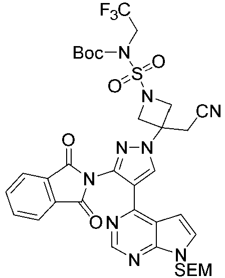

Cesium carbonate (264 mg, 0.81 mmol, 3.0eq) and 2,2,2-trifluoroethyltrifluoromethanesulfonate (175 mg, 0.54 mmol, 2.0eq) were added to a solution of tert-butyl ((3-(cyanomethyl)- 3-(3-(1,3-dioxoisoindolin-2-yl)-4-(7-((2-(trimethylsilyl)ethoxy)methyl)-7H-pyrrolo[2 ,3-d]pyrimidin-4-yl)-1H-pyrazol-1-yl)azetidine-1-yl)sulfonyl)carbamate (200 mg, 0.27 mmol, 1.0eq) in N,N-dimethylformamide (5 mL). The reaction solution was stirred at 25 °C for 2 hours, diluted with water (20mL) and then extracted with ethyl acetate (20 mL * 3). The combined organic phase was washed with saturated saline solution (20 mL), dried over anhydrous sodium sulfate, and evaporated under reduced pressure to obtain the crude product. The crude product was purified through Combiflash column (petroleum ether: ethyl acetate=3:1) to obtain the product tert-butyl ((3-(cyanomethyl)-3-(3-(1,3-dioxoisoindolin-2-yl)-4-(7-((2-(trimethylsilyl)ethoxy)methyl)-7H-pyrrolo[2,3-d]pyrimidin-4-yl)-1H-pyrazol-1-yl)azetidine-1-yl)sulfonyl) (2,2,2-trifluoroethyl)carbamate in a white solid form (95 mg, yield=43%).

### LC-MS(ESI), m/z: [M+H]⁺ =815.9.

### Step D: 3-(cyanomethyl)-3-(3-(1,3-dioxoisoindolin-2-yl)-4-(7-(methylol)-7H-pyrrolo[2,3-d]pyrimidin-4-yl)-1H pyrazol-1-yl)-N-(2,2,2-trifluoroethyl)azetidine-1-sulfonamide

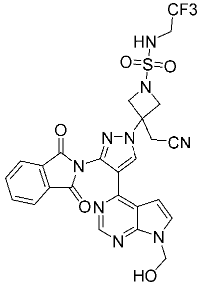

Tert-butyl ((3-(cyanomethyl)-3-(3-(1,3-dioxoisoindolin-2-yl)-4-(7-((2-(trimethylsilyl)ethoxy)methyl)-7H-pyrrolo[2,3-d]pyrimidin-4-yl)-1H-pyrazol-1-yl)azetidine-1-yl)sulfonyl) (2,2,2-trifluoroethyl)carbamate (95 mg, 0.12 mmol, 1.0eq) was added to dichloromethane (5 ml) at room temperature, and boron trifluoride ether solution (0.5 ml) was added at 0 °C. The reaction solution was stirred at 25°C for 2h, and then extracted with ethyl acetate and water. The organic phase was dried and concentrated to obtain the crude product (50 mg , yield=58%).

### LC-MS(ESI), m/z: [M+H]⁺ =615.9.

### Step E: 3-(3-amino-4-(7H-pyrrolo[2,3-d] pyrimidin-4-yl)-1H-pyrazol-1-yl)-3-(cyanomethyl)-N-(2,2,2 -trifluoroethyl)azetidine-1-sulfonamide

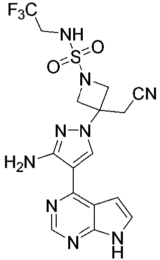

3-(cyanomethyl)-3-(3-(1,3-dioxoisoindolin-2-yl)-4-(7-(methylol)-7H-pyrrolo[2,3-d]pyrimidin-4-yl)-1H pyrazol-1-yl)-N-(2,2,2-trifluoroethyl)azetidine-1-sulfonamide (50 mg, 0.07 mmol, 1.0eq) was dissolved in methanol (2 mL). Under an ice bath, ethylenediamine (0.2 mL) was added in order to adjust the pH to 8-9. The reaction system was stirred at 25 °C for 1 h. The reaction solution was concentrated, and then passed through Prep-HPLC (chromatographic column type: gemini-C18 150 x 21.2 mm, 5µm, mobile phase: ACN-H2O (0.1% TFA), gradient 35%-40%, flow rate: 20 mL/min) to obtain the product (17.5 mg, yield=55%).

### LC-MS(ESI), m/z: [M+H]⁺ =455.7.

¹H-NMR(400 MHz, CD₃OD) δ 8.58 (s, 1H), 8.40 (s, 1H), 7.34 (d, J = 3.6 Hz, 1H), 6.86 (d, J = 3.6 Hz, 1H), 4.39 (d, J = 9.3 Hz, 2H), 4.02 (d, J = 9.4 Hz, 2H), 3.68 (q, J = 9.1 Hz, 2H), 3.38 (s, 2H).

The following examples were prepared according to the experimental route and method in Example 40:

| | |
|---|---|
| Example 41 : 3-(3-amino-4-(7H-pyrrolo[2,3-d]pyrimidin-4-yl)-1H-pyrazol-1-yl)-3-(cyanomethyl)-N-ethylazetidine-1-sulfonamide | |
| 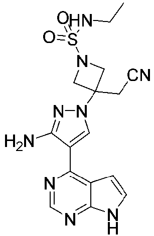 | LC-MS (ESI), m/z: [M+H]⁺ = 402.0. |
| | ¹H-NMR(400MHz, DMSO-*d*6) δ 12.08 (s, 1H), 8.67 (s, 1H), 8.63 (s, 1H), 7.56 (dd, J = 3.4, 2.5 Hz, 1H), 7.45 (t, J = 5.6 Hz, 1H), 7.10 (dd, J = 3.6, 1.7 Hz, 1H), 6.40 (s, 2H), 4.38 (d, J = 9.1 Hz, 2H), 3.97 (d, J = 9.2 Hz, 2H), 3.54 (s, 2H), 3.09 - 2.94 (m, 2H), 1.07 (t, J = 7.2 Hz, 3H). |
| Example 42: 3-(3-amino-4-(7H-pyrrolo[2,3-d]pyrimidin-4-yl)-1H-pyrazol-1-yl)-3-(cyanomethyl)-N-sulfamoylazetidine-1-sulfonamide | |
| 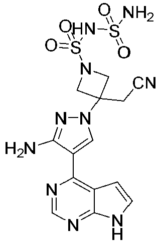 | LC-MS (ESI), m/z: [M+H]⁺ = 452.9. |
| | ¹H-NMR(400MHz, DMSO-*d*6) δ 11.98 (d, J = 23.7 Hz, 1H), 8.61 (s, 1H), 8.55 (s, 1H), 7.49 (d, J = 3.5 Hz, 1H), 7.07 (d, J = 3.5 Hz, 2H), 6.33 (s, 2H), 5.41 (s, 2H), 4.24 (s, 2H), 3.87 (s, 2H), 3.44 (s, 2H). |
| Example 43 : 3-(3-amino-4-(7H-pyrrolo[2,3-d]pyrimidin-4-yl)-1H-pyrazol-1-yl)-3-(cyanomethyl)-N, N-bis(2,2,2-trifluoroethyl)azetidine-1-sulfonamide | |
| 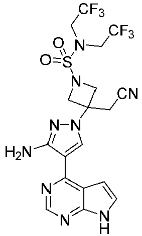 | LC-MS (ESI), m/z: [M+H]⁺ = 537.9. |
| | ¹H-NMR(400MHz, CD₃OD) δ 8.65 (d, J = 8.6 Hz, 1H), 8.51 (d, J = 14.2 Hz, 1H), 7.42 (t, J = 3.4 Hz, 1H), 6.94 (d, J = 3.6 Hz, 1H), 4.58 (d, J = 9.3 Hz, 2H), 4.18 (d, J = 9.3 Hz, 2H), 4.12 (q, J = 8.5 Hz, 4H), 3.46 (d, J = 3.1 Hz, 2H). |
| Example 44 : 3-(3-amino-4-(7H-pyrrolo[2,3-d]pyrimidin-4-yl)-1H-pyrazol-1-yl)-3-(cyanomethyl)-N-(2-hydroxyethyl)azetidine-1-sulfonamide | |
| 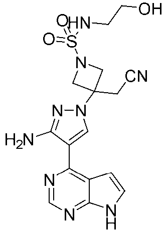 | LC-MS (ESI), m/z: [M+H]⁺ =417.9. |
| | ¹H-NMR(400MHz, DMSO-*d*6) δ 12.04 (s, 1H), 8.63 (s, 1H), 8.58 (s, 1H), 7.52 (dd, J = 3.4, 2.5 Hz, 1H), 7.45 (t, J = 5.8 Hz, 1H), 7.06 (dd, J = 3.6, 1.7 Hz, 1H), 6.36 (s, 2H), 4.71 (t, J = 5.5 Hz, 1H), 4.35 (d, J = 9.1 Hz, 2H), 3.95 (d, J = 9.1 Hz, 2H), 3.50 (s, 2H), 3.43 - 3.37 (m, 2H), 3.01 (q, J = 6.1 Hz, 2H). |
| Example 45 : 3-(3-amino-4-(7H-pyrrolo[2,3-d]pyrimidin-4-yl)-1H-pyrazol-1-yl)-3-(cyanomethyl)azetidine-1-sulfonamide | |
| 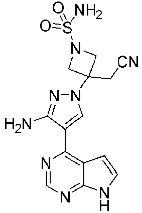 | LC-MS (ESI), m/z: [M+H]⁺ = 373.9. |
| | ¹H-NMR(400MHz, CD₃OD) δ 8.67 (s, 1H), 8.49 (s, 1H), 7.46 (d, J = 3.6 Hz, 1H), 6.96 (d, J = 3.7 Hz, 1H), 4.40 (d, J = 9.5 Hz, 2H), 4.07 (d, J = 9.6 Hz, 2H), 3.47 (s, 2H). |
| Example 46 : 3-(3-amino-4-(7H-pyrrolo[2,3-d]pyrimidin-4-yl)-1H-pyrazol-1-yl)-N-(2-chloroethyl)-3-(cyanomethyl)azetidine-1-sulfonamide | |
| 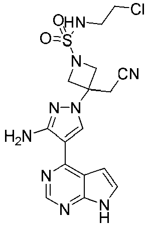 | LC-MS (ESI), m/z: [M+H]⁺ =435.9. |
| | ¹H-NMR(400MHz, DMSO-*d*6) δ 12.05 (s, 1H), 8.63 (s, 1H), 8.58 (s, 1H), 7.86 (s, 1H), 7.52 (dd, J = 3.5, 2.4 Hz, 1H), 7.08 - 7.04 (m, 1H), 6.36 (s, 2H), 4.37 (d, J = 9.1 Hz, 2H), 3.96 (d, J = 9.1 Hz, 2H), 3.62 (t, 2H), 3.52 (s, 2H), 3.27 (m, 2H). |
| Example 47 : 2-(3-(3-amino-4-(7H-pyrrolo[2,3-d]pyrimidin-4-yl)-1H-pyrazol-1-yl)-1-(aziridin-1-ylsulfonyl)azetidine-3-yl)acetonitrile | |
| 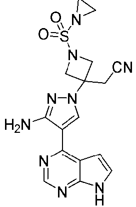 | LC-MS (ESI), m/z: [M+H]⁺ =400.0. |
| | ¹H-NMR(400MHz, DMSO-*d*6) δ 12.10 (s, 1H), 8.67 (s, 1H), 8.66 (s, 1H), 7.57 (s, 1H), 7.11 (d, J = 3.3 Hz, 1H), 6.43 (s, 2H), 4.69 (d, J = 9.2 Hz, 2H), 4.25 (d, J = 9.2 Hz, 2H), 3.61 (s, 2H), 2.37 (s, 4H). |
| Example 48 : 3-(3-amino-4-(7H-pyrrolo[2,3-d]pyrimidin-4-yl)-1H-pyrazol-1-yl)-3-(cyanomethyl)-N-(2-methoxyethyl)azetidine-1-sulfonamide | |
| 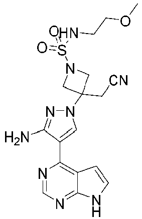 | LC-MS (ESI), m/z: [M+H]⁺ =432.0. |
| | ¹H-NMR(400MHz, DMSO-*d*6) δ 12.04 (s, 1H), 8.62 (s, 1H), 8.58 (s, 1H), 7.56 (s, 1H), 7.52 (dd, J = 3.4, 2.4 Hz, 1H), 7.06 (dd, J = 3.6, 1.7 Hz, 1H), 6.36 (s, 2H), 4.35 (d, J = 9.1 Hz, 2H), 3.94 (d, J = 9.2 Hz, 2H), 3.50 (s, 2H), 3.33 (t, J = 5.7 Hz, 2H), 3.18 (s, 3H), 3.11 (d, J = 5.5 Hz, 2H). |
| Example 49 : 3-(3-amino-4-(7H-pyrrolo[2,3-d]pyrimidin-4-yl)-1H-pyrazol-1-yl)-3-(cyanomethyl)-N-methylazetidine-1-sulfonamide | |
| 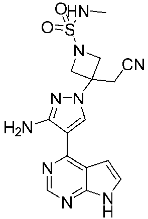 | LC-MS (ESI), m/z: [M+H]⁺ =387.9. |
| | ¹H-NMR(400MHz, DMSO-*d*6) δ 12.04 (s, 1H), 8.63 (s, 1H), 8.59 (s, 1H), 7.52 (dd, J = 3.5, 2.4 Hz, 1H), 7.31 (d, J = 4.9 Hz, 1H), 7.06 (dd, J = 3.6, 1.8 Hz, 1H), 6.36 (s, 2H), 4.37 (d, J = 9.2 Hz, 2H), 3.94 (d, J = 9.3 Hz, 2H), 3.52 (s, 2H), 2.58 (d, J = 4.8 Hz, 3H). |
| Example 50 : 3-(3-amino-4-(7H-pyrrolo[2,3-d]pyrimidin-4-yl)-1H-pyrazol-1-yl)-N,3-bis(cyanomethyl)azetidine-1-sulfonamide | |
| 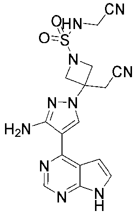 | LC-MS (ESI), m/z: [M+H]⁺ =412.9. |
| | ¹H-NMR(400MHz, DMSO-*d*6) δ 8.63 (s, 1H), 8.58 (s, 1H), 7.53 (d, J = 3.6 Hz, 1H), 7.05 (d, J = 3.6 Hz, 1H), 6.38 (s, 2H), 4.39 (s, 2H), 4.16 (s, 2H), 4.00 (s, 2H), 3.54 (s, 2H). |
| Example 51 : 3-(3-amino-4-(7H-pyrrolo[2,3-d]pyrimidin-4-yl)-1H-pyrazol-1-yl)-3-(cyanomethyl)-N,N-dimethylazetidine-1-sulfonamide | |
| 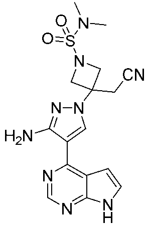 | LC-MS (ESI), m/z: [M+H]⁺ =401.9. |
| | ¹H-NMR(400MHz, DMSO-*d*6) δ 12.05 (s, 1H), 8.63 (s, 1H), 8.58 (s, 1H), 7.53 (s, 1H), 7.06 (dd, J = 3.6, 1.7 Hz, 1H), 6.35 (brs, 2H), 4.46 (d, J = 9.0 Hz, 2H), 4.03 (d, J = 9.0 Hz, 2H), 3.55 (s, 2H), 2.78 - 2.73 (m, 6H). |
| Example 52 : 3-(3-amino-4-(7H-pyrrolo[2,3-d]pyrimidin-4-yl)-1H-pyrazol-1-yl)-3-(cyanomethyl)-N-(2-(dimethylamino)ethyl)azetidine-1-sulfonamide | |
| 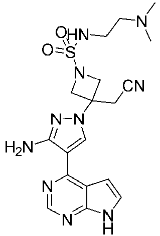 | LC-MS (ESI), m/z: [M+H]⁺ =445.0. |
| | ¹H-NMR(400MHz, DMSO-*d*6) δ 12.09 (s, 1H), 8.67 (s, 1H), 8.63 (s, 1H), 8.17 (s, 1H), 7.56 (d, J = 3.4 Hz, 1H), 7.10 (d, J = 3.5 Hz, 1H), 6.40 (s, 2H), 4.40 (d, J = 9.0 Hz, 2H), 3.99 (d, J = 9.1 Hz, 2H), 3.54 (s, 2H), 3.08 (d, J = 6.3 Hz, 2H), 2.37 (d, J = 6.4 Hz, 2H), 2.15 (d, J = 6.3 Hz, 6H). |
| Example 53: methyl (3-(3-amino-4-(7H-pyrrolo[2,3-d]pyrimidin-4-yl)-1H-pyrazol-1-yl)-3-(cyanomethyl)azetidine-1-ylsulfonyl)glycinate | |
| 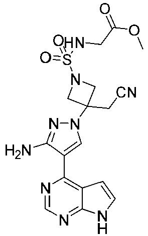 | LC-MS (ESI), m/z: [M+H]⁺ =445.9. |
| | ¹H-NMR(400MHz, DMSO-*d*6) δ 12.18 (s, 1H), 8.69 (s, 1H), 8.63 (s, 1H), 8.12 (s, 1H), 7.62 - 7.58 (m, 1H), 7.11 (dd, J = 3.4, 1.6 Hz, 1H), 5.80 (brs, 2H), 4.40 (d, J = 9.1 Hz, 2H), 4.01 (d, J = 9.1 Hz, 2H), 3.86 (d, J = 6.1 Hz, 2H), 3.63 (s, 3H), 3.53 (s, 2H). |
| Example 54 : (3-(3-amino-4-(7H-pyrrolo[2,3-d]pyrimidin-4-yl)-1H-pyrazol-1-yl)-3-(cyanomethyl)azetidine-1-yl(sulfonyl)glycine | |
| 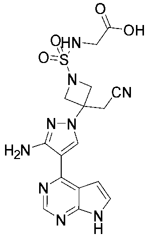 | LC-MS (ESI), m/z: [M+H]⁺ =431.9. |
| | ¹H-NMR(400MHz, DMSO-*d*6) δ 12.08 (s, 1H), 8.67 (s, 1H), 8.62 (s, 1H), 7.96 (s, 1H), 7.56 (s, 1H), 7.10 (s, 1H), 6.40 (s, 2H), 4.40 (d, J = 9.0 Hz, 2H), 4.00 (d, J = 8.9 Hz, 2H), 3.76 (d, J = 5.9 Hz, 2H), 3.53 (s, 2H). |

### Example 55: 3-(cyanomethyl)-3-(3-hydroxyl -4-(7H-pyrrolo[2,3-d]pyrimidin-4-yl)-1H-pyrazol-1-yl)-N-(2,2,2-trifluoroethyl)azetidine-1-formamide

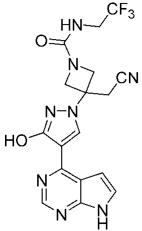

### Synthesis route:

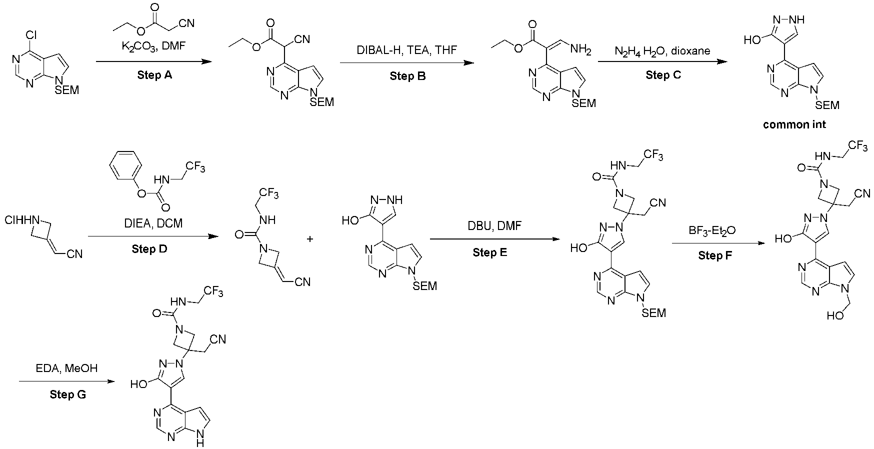

### Step A: ethyl 2-cyano-2-(7-{[2-(trimethylsilyl)ethoxy]methyl}pyrrolo[2,3-d]pyrimidin-4-yl)acetate

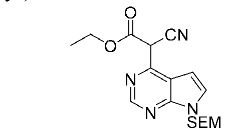

4-chloro-7-{[2-(trimethylsilyl)ethoxy]methyl}pyrrolo[2,3-d]pyrimidine (1 g, 3.5 mmol, 1.0eq), ethyl 2-cyanoacetate (1196 mg, 10.6 mmol, 3.0 eq) and potassium carbonate (974 mg, 7 mmol, 2.0 eq) were added in N,N-dimethylformamide (10 mL). Stirring was carried out at 60°C for 0.5 hours. The solution was heated to 130 °C and stirring was carried out again for 1 hour. After the reaction had been completed, dilution was carried out with water (50 mL), extraction with ethyl acetate (20 mL * 3). The organic phase was dried, concentrated and evaporated, and purify with a silica gel column (petroleum ether: ethyl acetate=5:1) to obtain the product ethyl 2-cyano-2-(7-{[2-(trimethylsilyl)ethoxy]methyl}pyrrolo[2,3-d]pyrimidin-4-yl)acetate (1.1g, yield=88%).

### LC-MS (ESI), m/z: [M+H]⁺ = 361.0.

### Step B: ethyl (2E)-3-amino-2-(7-{[2-(trimethylsilyl)ethoxy]methyl}pyrrolo[2,3-d] pyrimidin-4-yl)prop-2-enoate

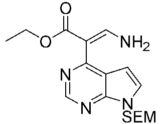

ethyl 2-cyano-2-(7-{[2-(trimethylsilyl)ethoxy]methyl}pyrrolo[2,3-d]pyrimidin-4-yl)acetate (1000 mg, 2.8 mmol, 1.0eq) and triethylamine (310mg, 3.0 mmol, 1.1eq) were added to dry tetrahydrofuran (20 mL). Diisobutylaluminum hydride (1 M in THF, 8.0 mL) was added dropwise at 0 °C, and the solution was stirred at 25 °C for 3 hours. After the reaction had been completed, dilution was carried out with water (50 mL), extraction with ethyl acetate (30 mL * 3). The organic phase was dried, concentrated and evaporated and purify with a silica gel column (petroleum ether: ethyl acetate=1:1) to obtain the product ethyl (2E)-3-amino-2-(7-{[2-(trimethylsilyl)ethoxy]methyl}pyrrolo[2,3-d]pyrimidin-4-yl)prop-2-enoate (700 mg, yield=70.0%).

### LC-MS (ESI), m/z: [M+H]⁺ = 363.1.

### Step C: 4-{7-[(2-methoxyethyl)trimethyl]-{5}-silanyl]pyrrolo[2,3-d]pyrimidin-4-yl}-1H-pyrazol-3-ol

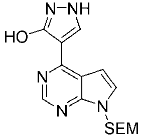

Ethyl (2E)-3-amino-2-(7-{[2-(trimethylsilyl)ethoxy]methyl}pyrrolo[2,3-d] pyrimidin-4-yl)prop-2-enoate (700 mg, 1.9 mmol, 1.0eq), water and hydrazine (193 mg, 3.86 mmol, 2.0eq) were added to dioxane (10 mL). Stirring was carried out for 2 hours at 80°C. The reaction solution was concentrated, evaporated and purified using silica gel column (dichloromethane : methanol=20 : 1) to obntian the product 4-{7-[(2-methoxyethyl)trimethyl]-{5}-silanyl]pyrrolo[2,3-d]pyrimidin-4-yl}-1H-pyrazol-3-ol (600 mg, yield=76%).

### LC-MS (ESI), m/z: [M+H]⁺ = 332.1.

### Step D: 3-cyanomethylene-N-(2,2,2-trifluoroethyl)azetidine-1-formamide

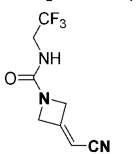

2- (azetidine-3-ylidene)acetonitrile hydrochloride (200 mg, 1.53 mmol, 1.0eq) was dissolved in dichloromethane (8 ml), and then N,N-diisopropylethylamine (593 mg, 4.59 mmol, 3.0eq) was slowly dropped into the reaction solution. After stirring the reaction system at 25 °C for 15 minutes, phenyl (2,2,2-trifluoroethyl)carbamate (337 mg, 1.53 mmol, 1.0 eq) was added to the reaction solution, and the reaction system was allowed to react at 25 °C for 18 hours. After the reaction had been completed, the reaction solution was evaporated to dryness under reduced pressure. The concentrated residue of the reaction solution was passed through column chromatography (petroleum ether: ethyl acetate=1:1) to obtain the product 3-cyanomethylene-N-(2,2,2-trifluoroethyl)azetidine-1-formamide (200 mg, yield=95%) as a white solid.

### LC-MS (ESI), m/z: [M+H]⁺ =262.0.

### Step E: 3-cyanomethyl-3-hydroxyl -4-(7-(2-trimethylsilylethoxymethyl)-7H-pyrrolidin-4-ylpyrimidin-1-pyrazol-1-yl-N-(2,2,2-trifluoroethyl)azetidine-1-formamide

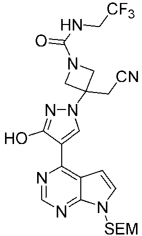

4-(2-trimethylsilylethoxymethyl)pyrrolopyrrolidin-4-ylpyrazole (302 mg, 0.91 mmol, 1.0eq) and 3-cyanomethylene-N-(2,2,2-trifluoroethyl)azetidine-1-formamide (200 mg, 0.91 mmol, 1.0eq) were dissolved in N,N-dimethylformamide (8 mL). Then, 1,8-diazabicyclo[5.4.0] undecene 7-ene (92 mg, 0.91 mmol, 1.0eq) was added in the reaction solution. The reaction system was reacted at 25 °C for 18 hours. After the reaction had been completed, water (20 mL) was added to the reaction liquid and extraction was carried out with dichloromethane (20 mL * 3). The combined organic phase was washed with saturated saline solution (20 mL), dried over anhydrous sodium sulfate. The concentrated residue of the reaction solution was passed through column chromatography (petroleum ether: ethyl acetate=1:1) to obtain the product 3-cyanomethyl-3-hydroxyl-4-(7-(2-trimethylsilylethoxymethyl)-7H-pyrrolidin-4-ylpyrimidin-1-pyrazol-1-yl-N-(2,2,2-trifluoroethyl)azetidine-1-formamide in the form of yellow oily substance (100 mg, yield=30%).

### LC-MS (ESI), m/z: [M+H]⁺ =551.0.

### Step F: 3-cyanomethyl-3-(3-hydroxy-4-methylol-7H-pyrrolylpyrrolyl)-2,3-d-pyrrolidin-4-yl-1-pyrazolyl-N-(2,2,2-trifluoroethyl)azetidine-1 -formamide

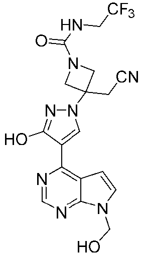

3-cyanomethyl-3-hydroxy-4-(7-(2-trimethylsilylethoxymethyl)-7H-pyrrolidin-4-yl-pyrimidin-1-pyrazol-1-yl-N-(2,2,2-trifluoroethyl)azetidine-1-formamide(150 mg, 0.27 mmol, 1.0eq) was dissolved in dichloromethane (6 ml). Under an ice bath, boron trifluoride-ether solution (2 mL) was added dropwise. The reaction solution was stirred for 2h at 25 °C. After the reaction had been completed, it was evaporated to dryness under reduced pressure to obian the crude product 3-cyanomethyl-3-(3-hydroxy-4-methylol-7H-pyrrolylpyrrolyl)-2,3-d-pyrrolidin-4-yl-1-pyrazolyl-N-(2,2,2-trifluoroethyl)azetidine-1-formamide as a yellow oily substance (82 mg, curd).

### LC-MS (ESI), m/z: [M+H]⁺ =451.0.

### Step D: 3-cyanomethyl-3-hydroxy-4-pyrrolidin-4-ylpyrimidin-2, 3-dimethylpyrazol-1-yl-N-(2,2,2-trifluoroethyl)azetidine-1-formamide

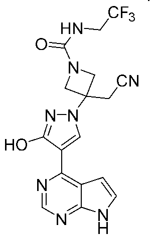

3-cyanomethyl-3-(3-hydroxy-4-methylol-7H-pyrrolylpyrrolyl)-2,3-d-pyrrolidin-4-yl-1-pyrazolyl-N-(2,2,2-trifluoroethyl)azetidine-1-formamide (82 mg, 0.18mmol, 10.0eq) was dissoved in methanol (5 mL). Under an ice bath, ethylenediamine (0.5 ml) was added. After the reaction had been completed, water (20 mL) was added into the reaction liquid, and then extraction was carried out with dichloromethane (10 mL*3). The combined organic phase was washed with saturated saline solution (20 mL), dried over anhydrous sodium sulfate, and evaporated under reduced pressure. The residue was passed through Prep-HPLC (chromatographic column type: gemini-C18 150 x 21.2 mm, 5µm, mobile phase: ACN-H2O (0.1% FA), gradient 10%-40%, flow rate: 20 mL/min) to obtain the product (5.3 mg, yield=7%).

### LC-MS (ESI), m/z: [M+H]⁺ =421.0.

¹H-NMR(400MHz, CD₃OD) δ 8.67 (s, 1H), 8.60 (s, 1H), 7.49 (d, J = 3.6 Hz, 1H), 7.05 (d, J = 3.6 Hz, 1H), 4.56 (d, J = 9.3 Hz, 2H), 4.28 (d, J = 9.3 Hz, 2H), 3.82 (d, J = 9.3 Hz, 2H), 3.48 (s, 2H).

The following examples were prepared according to the experimental route and method in Example 55:

| Example 56: 2-(1-(ethylsulfonyl)-3-(3-hydroxy-4-(7H-pyrrolo[2,3-d]pyrimidin-4-yl)-1H-pyrazol-1-yl)azetidine-3-yl)acetonitrile | |
|---|---|
| 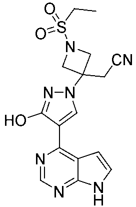 | LC-MS (ESI), m/z: [M+H]⁺ = 388.0. |
| | ¹H-NMR(400MHz, CD₃OD) δ 8.75 (d, J = 5.6 Hz, 2H), 7.64 (d, J = 3.6 Hz, 1H), 7.21 (t, J = 6.4 Hz, 1H), 4.61 (d, J = 9.3 Hz, 2H), 4.20 (d, J = 9.6 Hz, 2H), 3.53 (s, 2H), 3.16 - 3.09 (m, 2H), 1.34 (t, J = 7.4 Hz, 3H). |
| Example 57: 2-(3-(3-hydroxy-4-(7H-pyrrolo[2,3-d]pyrimidin-4-yl)-1H-pyrazol-1-yl)-1-(isopropylsulfonyl)azetidine-3-yl)acetonitrile | |
| 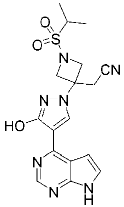 | LC-MS (ESI), m/z: [M+H]⁺ = 402.0. |
| | ¹H-NMR(400MHz, CD₃OD) δ 8.67 (s, 1H), 8.63 (s, 1H), 7.49 (d, J = 3.6 Hz, 1H), 7.06 (d, J = 3.6 Hz, 1H), 4.61 (d, J = 9.3 Hz, 2H), 4.18 (d, J = 9.4 Hz, 2H), 3.48 (s, 2H), 3.25 (d, J = 1.8 Hz, 1H), 1.35 (s, 3H), 1.33 (s, 3H). |
| Example 58: 2-(1-(cyclopropylsulfonyl)-3-(3-hydroxy-4-(7H-pyrrolo[2,3-d]pyrimidin-4-yl)-1H-pyrazol-1-yl)azetidine-3-yl)acetonitrile | |
| 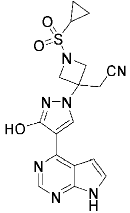 | LC-MS (ESI), m/z: [M+H]⁺ = 400.1. |
| | ¹H-NMR(400MHz, CD₃OD) δ 8.67 (s, 1H), 8.65 (s, 1H), 7.49 (d, J = 3.5 Hz, 1H), 7.06 (d, J = 3.6 Hz, 1H), 4.62 (d, J = 9.3 Hz, 2H), 4.24 (d, J = 9.5 Hz, 2H), 3.50 (s, 2H), 2.72 - 2.59 (m, 1H), 1.08 (dd, J = 6.4, 4.9 Hz, 4H). |
| Example 59: 2-(1-(isopropyl sulfonyl)-3-(3-methoxy-4-(7H-pyrrolo[2,3-d]pyrimidin-4-yl)-1H-pyrazol-1-yl)azetidine-3-yl)acetonitrile | |
| 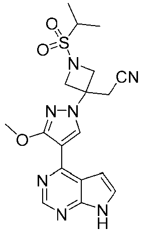 | LC-MS (ESI), m/z: [M+H]⁺ =416.1_{∘} |
| | ¹H-NMR(400MHz, DMSO-*d*6 ) (400 MHz, DMSO) δ 12.09 (s, 1H), 8.68 (s, 1H), 8.65 (s, 1H), 7.55 - 7.51 (m, 1H), 6.94 (dd, J = 3.5, 1.7 Hz, 1H), 4.54 (d, J = 9.0 Hz, 2H), 4.15 (d, J = 9.0 Hz, 2H), 4.00 (s, 3H), 3.63 (s, 2H), 3.36 (d, J = 6.8 Hz, 1H), 1.28 (s, 3H), 1.26 (s, 3H)_{∘} |

### Example 60: 1-(3-(cyanomethyl)-1-((2,2,2-trifluoroethyl)sulfonyl)azetidine-3-yl)-4-(7H-pyrrolo[2,3-d]pyrimidin-4-yl)-1H-pyrazole-3-formamide

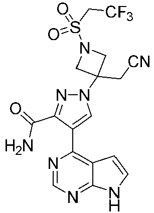

### Synthesis route:

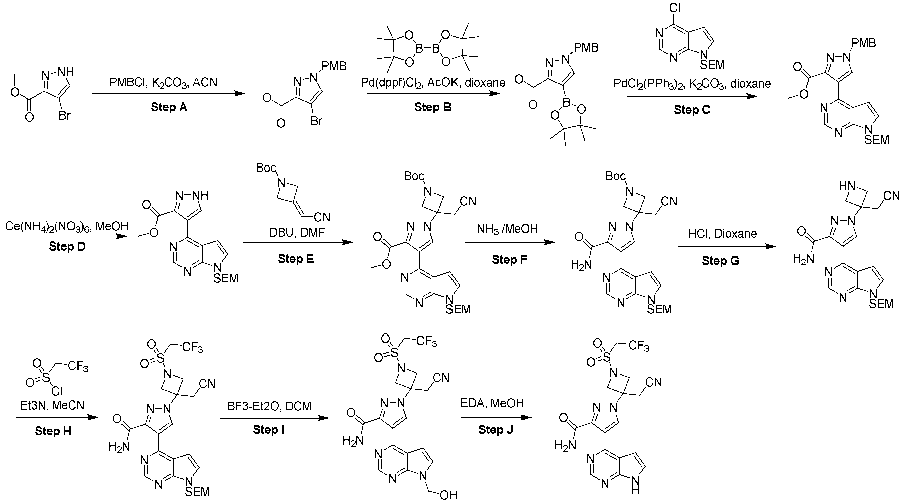

### Step A: methyl 4-bromo-1- (4-methoxybenzyl)-1H-pyrazole-3-carboxylate

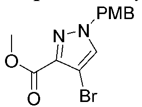

Potassium carbonate (6.78 g, 50mmol, 2.0eq) and p-methoxybenzyl chloride (7.69 g, 25mmol, 1.0eq) was added to the solution of methyl 4-bromo-1-1H-pyrazole-3-carboxylate (5 g, 25mmol, 1.0eq) in acetonitrile (50 mL). The reaction solution was stirred for 2 h at 25 °C. After the reaction had been completed, the reaction solution was concentrated under reduced pressure to obtain the product methyl 4-bromo-1- (4-methoxybenzyl)-1H-pyrazole-3-carboxylate in the yellow solid form (5.88 g, crude).

### LC-MS(ESI), m/z: [M+H]⁺ =325.0.

### Step B: methyl 1-(4-methoxybenzyl)-4-(4,4,5,5-tetramethyl-1,3,2-dioxaborocyclopentan-2-yl)-1H-pyrazole-3-carboxylate

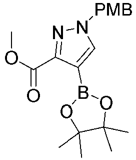

[1,1'-bis(diphenylphosphine)ferrocene] dichloride palladium (II) (1.316 g, 1.8mmol, 0.1eq) was added to the solution of methyl 4-bromo-1- (4-methoxybenzyl)-1H-pyrazole-3-carboxylate (5.88 g, 18mmol, 1.0eq), bisoprolol borate ester (4.58 g, 18 mmol, 1.0 eq) and potassium acetate (5.292 g, 54 mmol l, 3.0 eq) in 1,4-dioxane (200 mL). Under nitrogen protection, the reaction solution was stirred for 16 h at 90 °C, was diluted with water (20 mL) and then extracted with ethyl acetate (20 mL * 3). The combined organic phase was washed with saturated saline solution (20 mL), dried over anhydrous sodium sulfate, and evaporated under reduced pressure to obtain the crude product. The crude product was purified using Combiflash column (petroleum ether: ethyl acetate=2:1) to obtain the crude product in the form of a yellow solid (3.74 g, crude).

### LC-MS(ESI), m/z: [M+H]⁺ =373.1.

### Step C: methyl 1-(4-methoxybenzyl)-4-(7-((2-(trimethylsilyl)ethoxy)methyl)-7H-pyrrolo[2,3-d]pyrimidin-4-yl)-1H-pyrazole-3-carboxylate

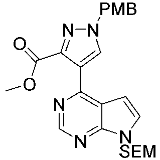

Bistriphenylphosphine palladium dichloride (0.7 g, 1mmol, 0.1eq) was added to the solution of methyl 1-(4-methoxybenzyl)-4-(4,4,5,5-tetramethyl-1,3,2-dioxaborocyclopentan-2-yl)-1H-pyrazole-3-carboxylate (3.74 g, 10mmol, 1.0eq), 4-chloro-7-((2-(trimethylsilyl) ethoxy)methyl)-7H-pyrrolo[2,3-d]pyrimidine (2.83 g, 10mmol, 1.0eq) and potassium acetate (4.14 g, 30mmol, 3.0eq) in 1,4-dioxane. Under nitrogen protection, the reaction solution was stirred for 16 h at 90 °C, was diluted with water (20 mL) and then extracted with ethyl acetate (20 mL * 3). The combined organic phase was washed with saturated saline solution (20 mL), dried over anhydrous sodium sulfate, and evaporated under reduced pressure to obtain the crude product. The crude product was purified using Combiflash column (dichloromethane: methanol=20:1) to obtain the crude product in the form of a yellow solid (1.85 g, crude).

### LC-MS(ESI), m/z: [M+H]+ =494.1.

### Step D: methyl 4-(7-((2-(trimethylsilyl)ethoxy)methyl)-7H-pyrrolo[2,3-d]pyrimidin-4-yl)-1H-pyrazole-3-carboxylate

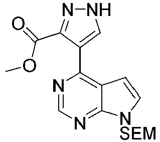

Ceric ammonium nitrate (9.8 g, 18.5mmol, 5.0eq) was added to the solution of methyl 1-(4-methoxybenzyl)-4-(7-((2-(trimethylsilyl)ethoxy)methyl)-7H-pyrrolo[2,3-d]pyrimidin-4-yl)-1H-pyrazol-3- carboxylate (1.85 g, 3.7mmol, 1.0eq) in methanol (20 mL). The reaction solution was stirred overnight at 25 °C, diluted with water (20 mL) and then extracted with ethyl acetate (20 mL * 3). The combined organic phase was washed with saturated saline solution (20 mL), dried over anhydrous sodium sulfate, and evaporated under reduced pressure to obtain the crude product. The crude product was purified using Combiflash column (dichloromethane: methanol=15:1) to obtain the crude product in the form of a yellow solid (900 mg, yield=65%).

### LC-MS(ESI), m/z: [M+H]⁺ =374.1.

### Step E: methyl 1-(1-(tert-butoxycarbonyl)-3-(cyanomethyl)azetidine-3-yl)-4-(7-((2-(trimethylsilyl)ethoxy)methyl)-7H-pyrrolo[2,3-d]pyrimidin-4-yl)-1H-pyrazol-3-carboxylate

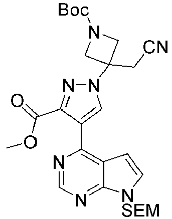

Tert-butyl 3-(cyanomethylene)azetidine-1-carboxylate (472 mg, 2.41 mmol, 1.0eq) was added to the solution of methyl 4-(7-((2-(trimethylsilyl)ethoxy)methyl)-7H-pyrrolo[2,3-d]pyrimidin-4-yl)-1H-pyrazole-3-carboxylate (900 mg, 2.41 mmol, 1.0eq) and 1,8-diazoheterobisspiro[5.4.0]undec-7-ene (367 mg, 2.41 mmol, 1.0eq) in N,N-dimethylformamide (5 mL). The reaction solution was stirred overnight at 25 °C, diluted with water (20 mL) and then extracted with ethyl acetate (20 mL * 3). The combined organic phase was washed with saturated saline solution (20 mL), dried over anhydrous sodium sulfate, and evaporated under reduced pressure to obtain the crude product. The crude product was purified using Combiflash column (dichloromethane: methanol=20:1) to obtain the crude product in the form of a white solid (800 mg, yield=58%).

### LC-MS(ESI), m/z: [M+H]⁺ =568.2.

### Step F: tert-butyl 3-(3-aminoform-4-(7-(2-(trimethylsilyl)ethoxy)-7H-pyrrolo[2,3-3-]pyrimidin-4-yl)-1H-pyrazol-1-yl)-3-(cyanomethyl)aziridine-1-carboxylate

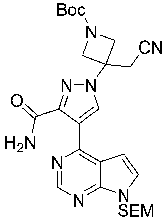

The solution of methyl 1-(1-(tert-butoxycarbonyl)-3-(cyanomethyl)azetidine-3-yl)-4-(7-((2-(trimethylsilyl)ethoxy)methyl)-7H-pyrrolo[2,3-d]pyrimidin-4-yl)-1H-pyrazol-3-carboxylate (250 mg, 0.44 mmol, 1.0eq) and ammonia gas in methanol (5 mL) was stirred overnight at 25 °C, and evaporated under reduced pressure to obtain the crude product tert-butyl 3-(3-aminoform-4-(7-(2-(trimethylsilyl)ethoxy)-7h-pyrrolo[2,3-3-]pyrimidin-4-yl)-1H-pyrazol-1-yl)-3-(cyanomethyl)aziridine-1-carboxylate (195 mg, yield=80%).

### LC-MS(ESI), m/z: [M+H]⁺ =553.2.

### Step G: 1-(3-(cyanomethyl)azapyridin-3-yl)-4-(7-(2-(trimethylsilyl)ethoxy)methyl)- 7H - pyrrolo[2,3-d]pyrimidin-4-yl)-1H-pyrazole-3-carboxamide

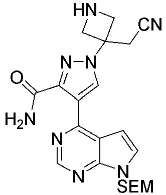

The solution of tert-butyl 3-(3-aminoform-4-(7-(2-(trimethylsilyl)ethoxy)-7H-pyrrolo[2,3-3-]pyrimidin-4-yl) -1H-pyrazol-1-yl)-3-(cyanomethyl)aziridine-1-carboxylate (195 mg, 0.35 mmol, 1.0eq) in (4M) hydrochloric acid-1,4-dioxane (5 mL) was stirred for 2 h at 25 °C, and evaporated under reduced pressure to obtain the crude product 1-(3-(cyanomethyl)azapyridin-3-yl)-4-(7-(2-(trimethylsilyl)ethoxy)methyl)-7H-pyrrolo[2,3-d]pyrimidin-4-yl)-1H-pyrazole-3-carboxamide (135 mg, crude).

### LC-MS(ESI), m/z: [M+H]⁺ =453.1.

### Step H: 1-(3-(cyanomethyl)-1-((2,2,2-trifluoroethyl)sulfonyl)aziridin-3-yl)-4-(7-((2-(trimethylsilyl)ethoxy)methyl)- 7H -pyrrolo[2,3-d]pyrimidin-4-yl)-1H-pyrazole-3-carboxamide

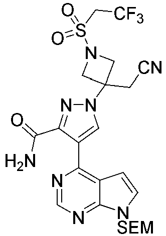

2,2,2-trifluoroethane-1-sulfonyl chloride (54.3 mg, 0.3 mmol, 1.0eq) was added to the solution of 1-(3-(cyanomethyl)azapyridin-3-yl)-4-(7-(2-(trimethylsilyl)ethoxy)methyl)-7H-pyrrolo[2,3-d]pyrimidin-4-yl)-1H-pyrazole-3- carboxamide (135 mg, 0.3 mmol, 1.0eq) and triethylamine (60 mg, 0.6 mmol, 2.0eq) in acetonitrile (5 mL). The reaction solution was stirred for 2 h at 25 °C. The reaction solution was stirred overnight at 25 °C. The reaction solution was diluted with water (20mL) and then extracted with ethyl acetate (20mL * 3). The combined organic phase was washed with saturated saline solution (20 mL), dried over anhydrous sodium sulfate, and evaporated under reduced pressure to obtain the crude product. The crude product was purified using Combiflash column (dichloromethane: methanol=20:1) to obtain the crude product 1-(3-(cyanomethyl)-1-((2,2,2-trifluoroethyl)sulfonyl)aziridin-3-yl)-4-(7-((2-(trimethylsilyl)ethoxy)methyl)- 7H -pyrrolo[2,3-d]pyrimidin-4-yl)-1H-pyrazole-3-carboxamide in the form of a yellow solid (100 mg, yield=56%).

### LC-MS(ESI), m/z: [M+H]⁺ =599.0.

### Step I: 1-(3-(cyanomethyl)-1-((2,2,2-trifluoroethyl)sulfonyl)azapyridin-3-yl)-4-(7-(methylol)-7H-pyrrolo[2,3-d]pyrimidin-4-yl)-1H-pyrazol-3-carboxamide

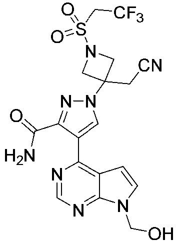

Boron trifluoride-ether solution (0.5 mL) was added to the solution of 1-(3-(cyanomethyl)-1-((2,2,2-trifluoroethyl)sulfonyl)aziridin-3-yl)-4-(7-((2-(trimethylsilyl)ethoxy)methyl)-7H-pyrrolo[2,3-d]pyrimidin-4-yl)-1H-pyrazole-3-carboxamide (100 mg, 0.17 mmol, 1.0eq) in dichloromethane (5 mL). The reaction solution was stirred for 2 h at 25 °C. After the reaction had been completed, the reaction solution was concentrated under reduced pressure to obtain the product 1-(3-(cyanomethyl)-1-((2,2,2-trifluoroethyl)sulfonyl)azapyridin-3-yl)-4-(7-(methylol)-7H-pyrrolo[2,3-d]pyrimidin-4-yl)-1H-pyrazol-3-carboxamide in a yellow solid form (63 mg, curde).

### LC-MS(ESI), m/z: [M+H]⁺ =499.0.

### Step J: 1-(3-(cyanomethyl)-1-((2,2,2-trifluoroethyl)sulfonyl)azapyridin-3-yl)-4-(7H-pyrrolo[2,3-d]pyrimidin-4-yl)-1H-pyrazole-3-carboxamide

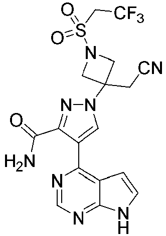

Ethylenediamine (0.2 mL) was added to the solution of 1-(3-(cyanomethyl)-1-((2,2,2-trifluoroethyl)sulfonyl)azapyridin-3-yl)-4-(7-(methylol)-7H-pyrrolo[2,3-d]pyrimidin-4-yl)-1H-pyrazol-3-carboxamide (63 mg, 0.13 mmol, 1.0eq) in methanol (2 mL). The reaction solution was stirred for 2 h at 25 °C. The solution was concentrated under vacuum. The reaction solution was stirred for 2 h at 25 °C. The reaction solution was diluted with water (20mL) and then extracted with ethyl acetate (10mL * 3). The combined organic phase was washed with saturated saline solution (20 mL), dried over anhydrous sodium sulfate, and evaporated under reduced pressure to obtain the crude product. The residue was passed through Prep-HPLC (chromatographic column type: gemini-C18 150 x 21.2 mm, 5µm, mobile phase: ACN-H2O (0.1% TFA), gradient 25%-30%, flow rate: 20 mL/min) to obtain the product (3.2 mg, yield=5.2%).

### LC-MS(ESI), m/z: [M+H]⁺ =469.0.

¹H-NMR(400MHz, D₂O) δ 8.72 (s, 1H), 8.69 (s, 1H), 8.30 (s, 1H), 7.57 (d, J = 3.6 Hz, 1H), 6.74 (d, J = 3.5 Hz, 1H), 4.77 (d, J = 9.4 Hz, 2H), 4.46 (d, J = 9.3 Hz, 2H), 4.39 (q, J = 9.2 Hz, 2H), 3.60 (s, 2H).

The following examples were prepared according to the experimental route and method in Example 60:

| | |
|---|---|
| Example 61: 1-(3-(cyanomethyl}-1-((2,2,2-trifluoroethyl)carbamoyl)azetidine-3-yl)-4-(7H-pyrrolo[2,3-d]pyrimidin-4-yl)-1H-pyrazol-3-formamide | |

| | |
|---|---|
| 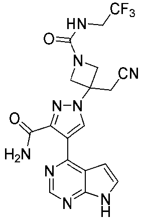 | LC-MS (ESI), m/z: [M+H]⁺ = 448.1. |
| | ¹H-NMR(400MHz, CD₃OD) δ 8.77 (s, 1H), 8.74 (s, 1H), 7.56 (d, J = 3.7 Hz, 1H), 6.88 (d, J = 3.7 Hz, 1H), 4.60 (d, J = 9.5 Hz, 2H), 4.31 (d, J = 9.5 Hz, 2H), 3.75 (q, J = 9.3 Hz, 2H), 3.55 (s, 2H). |
| Example 62 : 1-(3-(cyanomethyl)-1-((2,2,2-trifluoroethyl)sulfonyl)azetidine-3-yl)-N-methyl-4-(7H-pyrrolo[2,3-d]pyrimidin-4-yl)-1H-pyrazol-3-formamide | |
| 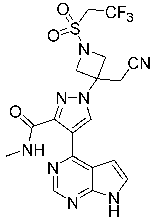 | LC-MS (ESI), m/z: [M+H]⁺ =483.0. |
| | ¹H-NMR(400MHz, D₂O) δ 8.60 (s, 1H), 8.45 (s, 1H), 8.34 (s, 1H), 7.42 (d, J = 3.5 Hz, 1H), 6.47 (d, J = 3.6 Hz, 1H), 4.74 (m, 2H), 4.47 (d, J = 9.4 Hz, 2H), 3.87 (s, 2H), 3.57 (s, 2H), 2.77 (s, 3H). |
| Example 63 : 1-(3-(cyanomethyl)-1-(ethylsulfonyl)azetidine-3-yl)-N-methyl-4-(7H-pyrrolo[2,3-d]pyrimidin-4-yl)-1H-pyrazol-3-formamide | |
| 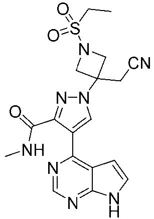 | LC-MS (ESI), m/z: [M+H]⁺ = 429.0. |
| | ¹H-NMR(400MHz, CD₃OD) δ 8.81 (s, 1H), 8.69 (s, 1H), 7.54 (d, J = 3.6 Hz, 1H), 6.78 (d, J = 3.6 Hz, 1H), 4.72 (d, J = 9.6 Hz, 2H), 4.35 (d, J = 9.7 Hz, 2H), 3.66 (s, 2H), 3.19 (p, J = 7.3 Hz, 2H), 2.98 (s, 3H), 1.38 (t, J = 7.4 Hz, 3H). |
| Example 64: 1-(3-(cyanomethyl)-1-(isopropylsulfonyl)azetidine-3-yl)-N-methyl-4-(7H-pyrrolo[2,3-d]pyrimidin-4-yl)-1H-pyrazol-3-formamide | |
| 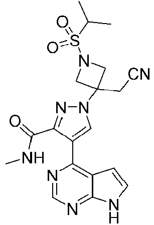 | LC-MS (ESI), m/z: [M+H]⁺ =443.0. |
| | ¹H-NMR(400MHz, CD₃OD) δ 9.33 (s, 1H), 9.11 (s, 1H), 7.96 (d, J = 3.8 Hz, 1H), 7.48 (d, 1H), 4.79 (d, J = 9.8 Hz, 2H), 4.33 (m, 2H), 3.68 (d, J = 6.3 Hz, 2H), 3.28 (m, 1H), 3.04 (s, 3H), 1.35 (m, 6H). |
| Example 65 : 1-(3-(cyanomethyl)-1-(cyclopropylsulfonyl)azetidine-3-yl)-N-methyl-4-(7H-pyrrolo[2,3-d]pyrimidin-4-yl)-1H-pyrazol-3-formamide | |

| | |
|---|---|
| 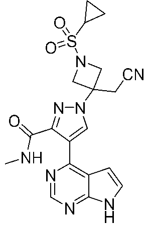 | LC-MS (ESI), m/z: [M+H]⁺ =441.1. |
| | ¹H-NMR(400MHz, CD₃OD) δ 8.78 (s, 1H), 8.69 (s, 1H), 8.40 (s, 1H), 7.51 (t, J = 5.1 Hz, 1H), 6.76 (d, J = 3.6 Hz, 1H), 4.71 (d, J = 9.5 Hz, 2H), 4.34 (d, J = 9.5 Hz, 2H), 3.62 (s, 2H), 2.94 (s, 3H), 2.68 (m, 1H), 1.07 (dd, J = 11.8, 6.8 Hz, 4H). |
| Example 66 : 1-(3-(cyanomethyl)-1-(ethylsulfonyl)azetidine-3-yl)-4-(7H-pyrrolo[2,3-d]pyrimidin-4-yl)-1H-pyrazol-3-formamide | |
| 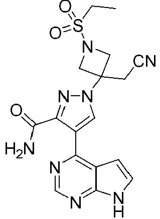 | LC-MS (ESI), m/z: [M+H]⁺ =415.0. |
| | ¹H-NMR(400MHz, CD₃OD) δ 8.81 (m, 1H), 8.14 (s, 1H), 7.60 (dd, J = 3.7, 0.8 Hz, 1H), 6.91 (m, 1H), 4.70 (d, J = 9.3 Hz, 2H), 4.32 (d, J = 9.1 Hz, 2H), 3.63 (s, 2H), 3.16 (m, 2H), 1.35 (m, 3H). |
| Exmple 67 : 1-(3-(cyanomethyl)-1-(cyclopropylsulfonyl)azetidine-3-yl)-4-(7H-pyrrolo[2,3-d]pyrimidin-4-yl)-1H-pyrazol-3-formamide | |
| 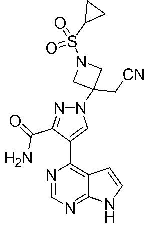 | LC-MS (ESI), m/z: [M+H]⁺ =427.0. |
| | ¹H-NMR(400MHz, CD₃OD) δ 9.34 (s, 1H), 9.09 (s, 1H), 7.95 (d, J = 3.8 Hz, 1H), 7.47 (d, J = 3.8 Hz, 1H), 4.78 (d, J = 9.4 Hz, 2H), 4.44 - 4.38 (m, 2H), 3.68 (s, 2H), 2.67 - 2.61 (m, 1H), 1.09 (dd, J = 9.1, 2.9 Hz, 4H). |
| Example 68 : 1-(3-(cyanomethyl)-1-(isopropylsulfonyl)azetidine-3-yl)-4-(7H-pyrrolo[2,3-d]pyrimidin-4-yl)-1H-pyrazol-3-formamide | |
| 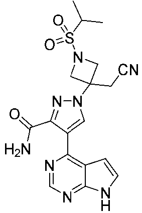 | LC-MS (ESI), m/z: [M+H]⁺ =429.0. |
| | ¹H-NMR(400MHz, CD₃OD) δ 9.35 (s, 1H), 9.12 (s, 1H), 7.99 (s, 1H), 7.49 (s, 1H), 4.83 (d, J = 9.4 Hz, 2H), 4.38 (d, J = 9.3 Hz, 2H), 3.73 (s, 2H), 3.33 (m, 1H), 1.40 (d, J = 6.8 Hz, 6H). |

### Example 69: 2-[3-(3-amino-4-{6-[(1-methylpyrazol-4-yl)amino]pyrimidin-4-yl}pyrazol-1-yl)-1-methylsulfonylaziridin-3-yl]acetonitrile

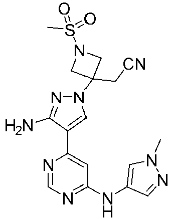

### Synthesis route:

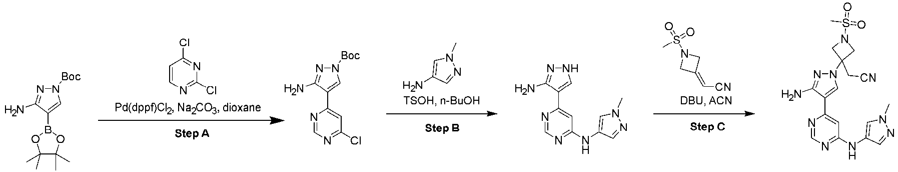

### Step A: tert-butyl [3-amino-4-(2-chloropyrimidin-4-yl)pyrazol-1-yl]formate

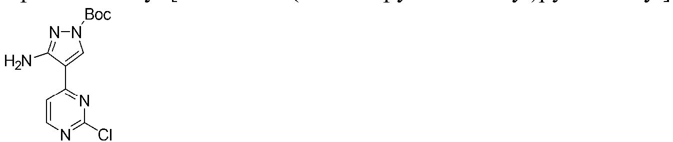

2,4-dichloropyrimidine (644.44 mg, 4.28 mmol, 1.5 eq), sodium carbonate (611 mg, 5.82 mmol, 2.0 eq), and bis(triphenylphosphine) palladium dichloride (141.81mg, 0.194 mmol, 0.07eq) was added to the solution of tert-butyl [3-amino-4-(4,4,5,5-tetramethyl-1,3,2-dioxaboran-2-yl) pyrazol-1-yl]formate in 1,4-dioxane (10 mL). Under nitrogen protection, the reaction solution was stirred for 3 hours on a 110 °C oil bath. After the reaction had been completed, the reaction solution was evaporated and purified using silica gel column (petroleum ether: ethyl acetate=1:1) to obtain tert-butyl [3-amino-4-(2-chloropyrimidin-4-yl)pyrazol-1-yl]formate (150 mg, yield=35 %).

### LC-MS (ESI), m/z: [M+H]⁺ = 296.1.

### Step B: 6-(3-amino-1H-pyrazol-4-yl)-N-(1-methylpyrazol-4-yl)pyrimidin-4-amine

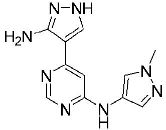

tert-butyl [3-amino-4-(2-chloropyrimidin-4-yl)pyrazol-1-yl]formate (100 mg, 0.33 mmol, 1.0eq) and 1-methylpyrazole-4-amine (46.7 mg, 0.495 mmol, 1.5eq) were added to n-butanol (5 mL). After dissolution, p-toluenesulfonic acid (170 mg, 0.99 mmol, 3.0 eq) was added, stirring was carried out at 110 °C for 4 hours, and then concentration and evaporation was carried out. Purification was carried out using silica gel column (dichloromethane: methanol=10:1) to obtain 6-(3-amino-1H-pyrazol-4-yl)-N-(1-methylpyrazol-4-yl)pyrimidin-4-amine (70 mg ,yield= 80 %).

### LC-MS (ESI), m/z: [M+H]⁺ = 257.0.

### Step C: 2-[3-(3-amino-4-{6-[(1-methylpyrazol-4-yl)amino]pyrimidin-4-yl}pyrazol-1-yl)-1-methylsulfonylaziridin-3-yl]acetonitrile

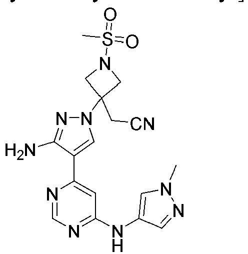

6-(3-amino-1H-pyrazol-4-yl)-N-(1-methylpyrazol-4-yl)pyrimidin-4-amine (70 mg, 0.27 mmol, 1.0eq) and 2-(1-methylsulfonyl aziridine-3-ylidene)acetonitrile (70.5 mg, 0.41 mmol, 1.5eq) were added to acetonitrile (5 mL). After dissolution, 1,8-diazabicyclo[5.4.0]undec-7-ene(41.04 mg, 0.27 mmol, 1.0eq) was added. Stirring was carried out at 60 °C for 3 hours, and The reaction solution was concentrated. The residue was passed through Prep-HPLC (chromatographic column type: gemini-C18 150 x 21.2 mm, 5µm, mobile phase: ACN-H2O (0.1% TFA), gradient 25%-30%, flow rate: 20 mL/min) to obtain the product 2-[3-(3-amino-4-{6-[(1-methylpyrazol-4-yl)amino]pyrimidin-4-yl}pyrazol-1-yl)-1-methylsulfonylaziridin-3-yl]acetonitrile (2 mg, yield=2%).

### LC-MS (ESI), m/z: [M+H]⁺ = 429.0.

¹H-NMR(400MHz, CD₃OD) δ 8.66 (s, 1H), 8.39 (s, 1H), 8.05 (s, 1H), 7.66 (s, 1H), 7.00 (s, 1H), 4.47 (d, J = 9.2 Hz, 2H), 4.20 (d, J = 9.2 Hz, 2H), 3.91 (s, 3H), 3.51 (s, 2H), 3.04 (s, 3H).

The following examples were prepared according to the experimental route and method in Example 69:

| | |
|---|---|
| Example 70: 2-(3-(3-amino-4-(4-((2-hydroxyethyl)amino)-1,3,5-triazin-2-yl)-1H-pyrazol-1-yl)-1-(methylsulfonyl)azetidine-3-yl)acetonitrile | |
| 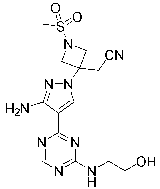 | LC-MS (ESI), m/z: [M+H]⁺ =394.1. |
| | ¹H-NMR(400MHz, D₂O) δ 8.29 (d, J = 16.8 Hz, 1H), 8.24 (d, J = 5.0 Hz, 1H), 4.46 (d, J = 9.1 Hz, 2H), 4.23 (d, J = 9.1 Hz, 2H), 3.74 - 3.63 (m, 2H), 3.51 (s, 2H), 3.42 (s, 2H), 3.04 (s, 3H). |
| Example 71: 2-(3-(3-amino-4-(4-((1-methylpiperidin-4-yl)amino)-1,3,5-triazin-2-yl)-1H- | |
| pyrazol-1-yl)-1-(methylsulfonyl)azetidine-3-yl)acetonitrile | |
| 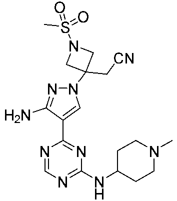 | LC-MS (ESI), m/z: [M+H]⁺ =447.0. |
| | ¹H-NMR(400MHz, CD₃OD) δ 8.33 (s, 1H), 7.90 (s, 1H), 4.60-4.57 (m, 2H), 4.25-4.23 (m, 2H), 3.65 - 3.60 (m, 3H), 3.34 (s, 2H), 3.25 - 2.68 (m, 8H), 2.40-2.21 (m, 2H), 1.94-1.85(m, 2H). |
| Example 72: 2-(3-(3-amino-4-(4-((tetrahydro-2H-pyran-4-yl)amino)-1,3,5-triazin-2-yl)-1H-pyrazol-1-yl)-1-(methylsulfonyl)azetidine-3-yl)acetonitrile | |
| 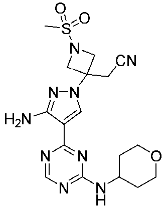 | LC-MS (ESI), m/z: [M+H]⁺ = 434.0. |
| | ¹H-NMR(400MHz, DMSO-*d*6) δ 10.24 - 9.79 (m, 1H), 8.10 (d, J = 30.7 Hz, 1H), 7.98 (d, J = 2.5 Hz, 1H), 4.37 (d, J = 9.1 Hz, 2H), 4.17 - 4.10 (m, 2H), 3.99- 3.76 (m, 3H), 3.51 (d, J = 7.4 Hz, 2H), 3.37 -3.25 (m, 2H), 3.07 (dd, J = 8.9, 4.3 Hz, 3H), 1.87 -1.67 (m, 2H), 1.54 -1.37 (m, 2H). |
| Example 73 : 2-(3-(3-amino-4-(4-((tetrahydro-2H-pyran-4-yl)methyl)amino)-1,3,5-triazin-2-yl)-1H-pyrazol-1-yl)-1-(methylsulfonyl)azetidine-3-yl)acetonitrile | |
| 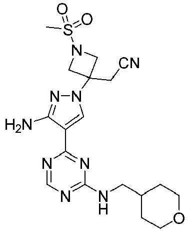 | LC-MS (ESI), m/z: [M+H]⁺ =448.0. |
| | ¹H-NMR(400MHz, CD₃OD) δ 8.39 (s, 1H), 8.06 - 7.92 (m, 1H), 4.56 (d, J = 12.1 Hz, 2H), 4.20 (dd, J = 12.4, 6.4 Hz, 2H), 4.02 - 3.88 (m, 2H), 3.59 (dd, J = 13.5, 9.0 Hz, 2H), 3.59 (dd, J = 13.5, 9.0 Hz, 2H), 3.43 - 3.33 (m, 2H), 3.02 (s, 3H), 1.95 (dtd, J = 11.2, 7.3, 3.4 Hz, 1H), 1.66 (dd, J = 13.0, 1.7 Hz, 2H), 1.40 - 1.24 (m, 2H). |
| Example 74: 2-(3-(3-amino-4-(6-((2-hydroxyethyl)amino)pyrimidin-4-yl)-1H-pyrazol-1-yl)-1-(methylsulfonyl)azetidine-3-yl)acetonitrile | |
| 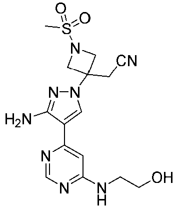 | LC-MS (ESI), m/z: [M+H]⁺ =393.0. |
| | ¹H-NMR(400MHz, CD₃OD) δ 8.42 (s, 1H), 8.25 (s, 1H), 6.79 (s, 1H), 4.36 (d, J = 9.3 Hz, 2H), 4.10 (d, J = 9.4 Hz, 2H), 3.66 (t, J = 5.4 Hz, 2H), 3.54 (s, 2H), 2.94 (s, 3H), 2.80 (s, 2H). |
| Example 75: 2-(3-(3-amino-4-(6-((tetrahydro-2H-pyran-4-yl)amino)pyrimidin-4-yl)-1H-pyrazol-1-yl)-1-(methylsulfonyl)azetidine-3-yl)acetonitrile | |
| 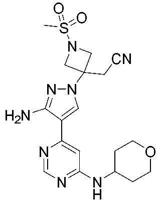 | LC-MS (ESI), m/z: [M+H]⁺ =433.1. |
| | ¹H-NMR(400MHz, CD₃OD) δ 8.55 (s, 1H), 8.31 (s, 1H), 6.86 (s, 1H), 4.43 (d, J = 9.3 Hz, 2H), 4.18 (d, J = 9.5 Hz, 2H), 3.97 (m, 2H), 3.53 (m, 2H), 3.49 (s, 3H), 3.02 (s, 2H), 2.96 (m, 1H), 1.95 (m, 2H), 1.63 (m, 2H). |
| Example 76: 2-(3-(3-amino-4-(6-((tetrahydro-2H-pyran-4-yl)methyl)amino)pyrimidin-4-yl-1H-pyrazol-1-yl)-1-(methylsulfonyl)azetidine-3-yl)acetonitrile | |
| 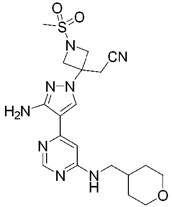 | LC-MS (ESI), m/z: [M+H]⁺ =447.1. |
| | ¹H-NMR(400MHz, CD₃OD) δ 8.55 (s, 1H), 8.33 (s, 1H), 6.89 (s, 1H), 4.43 (d, J = 9.1 Hz, 2H), 4.18 (d, J = 9.2 Hz, 2H), 3.93 (dd, J = 11.2, 3.2 Hz, 2H), 3.48 (d, J = 7.7 Hz, 3H), 3.39 (dd, J = 21.0, 9.8 Hz, 2H), 3.28 (dt, J = 3.1, 1.6 Hz, 2H), 3.03 (m, 2H), 1.89 (m, 1H), 1.66 (d, J = 13.4 Hz, 2H), 1.32 (m, 2H). |
| Example 77 : 2-(3-(3-amino-4-(6-(methylamino)pyrimidin-4-yl)-1H-pyrazol-1-yl)-1-(methylsulfonyl)azetidine-3-yl)acetonitrile | |
| 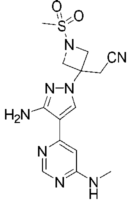 | LC-MS (ESI), m/z: [M+H]⁺ =363.2. |
| | ¹H-NMR(400MHz, CD₃OD) δ 8.34 (s, 1H), 8.03 (s, 1H), 7.81 (d, J = 2.6 Hz, 1H), 6.64 (s, 1H), 6.20 (d, J = 2.5 Hz, 1H), 4.52 (d, J = 9.2 Hz, 2H), 4.17 (d, J = 9.0 Hz, 2H), 3.50 (s, 2H), 3.01 (s, 3H), 2.86 (s, 3H). |
| Example 78: 2-(3-(3-amino-4-(2-(2-hydroxyethyl)amino)pyrimidin-4-yl)-1H-pyrazol-1-yl)-1-(methylsulfonyl)azetidine-3-yl)acetonitrile | |
| 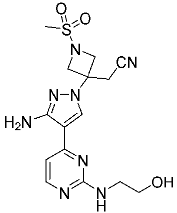 | LC-MS (ESI), m/z: [M+H]⁺ =393.1. |
| | ¹H-NMR(400MHz, CD₃OD) δ 8.38 (s, 1H), 7.84 (d, J = 2.6 Hz, 1H), 7.78 (d, J = 6.5 Hz, 1H), 4.49 (d, J = 9.3 Hz, 2H), 4.17 (d, J = 9.5 Hz, 2H), 3.71 (t, J = 5.6 Hz, 2H), 3.51 (d, J = 5.1 Hz, 2H), 3.49 - 3.42 (m, 2H), 3.01 (s, 3H). |
| Example 79 : 2-(3-(3-amino-4-(2-(benzylamino)pyrimidin-4-yl)-1H-pyrazol-1-yl)-1-(methylsulfonyl)azetidine-3-yl)acetonitrile | |
| 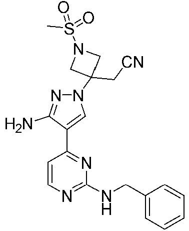 | LC-MS (ESI), m/z: [M+H]⁺ =439.0. |
| | ¹H-NMR(400MHz, CD₃OD) δ 7.86 (s, 1H), 7.76 (d, 1H), 7.37 - 7.30 (m, 6H), 6.68 (s, 1H), 6.34 (d, 1H), 4.76 - 4.70 (m, 2H), 4.50-4.46 (m, 2H), 4.20-4.18 (m , 2H), 3.52-3.48 (m, 2H), 3.03 (s, 3H). |

### Example 80:

### 2-(3-(3-amino-4-(6-(1-methyl-1H-pyrazol-4-yl)imidazolo[1,2-b]pyridazin-8-yl)-1H-pyridin-1-yl)-1-(ethylsulfonyl)azetidine-3-yl)acetonitrile

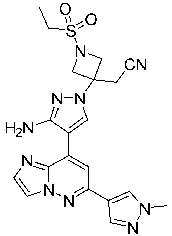

### Synthesis route:

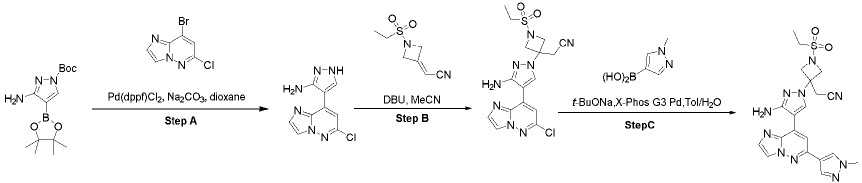

### Step A: 4-(6-chloroimidazolo[1,2-b]pyridazin-8-yl)-1H-pyrazol-3-amine

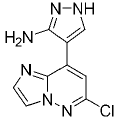

Tert-butyl 3-amino-4-(4,4,5,5-tetramethyl-1,3,2-dioxaborolan-2-yl)-1H-pyrazole-1-carboxylate (300 mg, 0.97 mmol, 1.0eq), 8-bromo-6-chloroimidazo[1,2-b]pyridazine (338 mg, 1.46 mmol, 1.5eq), sodium carbonate (305.8 mg, 2.9 mmol, 3.0eq) and [1,1'-bis(diphenylphosphine)ferrocene] dichloride palladium (II) (71 mg, 0.09mmol, 0.1eq) were dissolved in 1,4-dioxane (10 ml). The reaction solution was stirred at 80 ° C for 2 h. After the reaction had been completed, it was evaporated to dryness under reduced pressure. The concentrated residue of the reaction solution was passed through column chromatography (DCM: MeOH=20:1) to obtain the product 4-(6-chloroimidazolo[1,2-b]pyridazin-8-yl)-1H-pyrazol-3-amine as a yellow oily substance (131 mg, yield=58%).

### LC-MS (ESI), m/z: [M+H]⁺ =235.0.

### Step B: 2- (3-(3-amino-4-(6-chloroimidazo[1,2-b]pyridazin-8-yl)-1H-pyrazol-1-yl)-1-(ethylsulfonyl)azetidin-3-yl)acetylnitrile

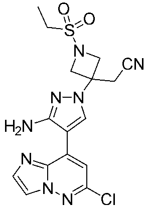

4-(6-chloroimidazolo[1,2-b]pyridazin-8-yl)-1H-pyrazol-3-amine (131 mg, 0.59mmol, 1.0eq) and 2-(1-(ethylsulfonyl)azetidin-3-ylidene)acetyl nitrile (104 mg, 0.59 mmol, 1.0eq) were dissolved in acetonitrile (5 ml). Then, 1,8-diazabicyclo[5.4.0]undec-7-ene (85 mg, 0.59 mmol, 1.0 eq) was added dropwise to the reaction solution and stirring was carried out at room temperature for 4 h. After the reaction had been completed, ice water (20 ml) was added to the reaction liquid and extraction was carried out with ethyl acetate (10 mL * 3). The combined organic phase was washed with saturated saline solution (20 mL), dried over anhydrous sodium sulfate, and evaporated under reduced pressure. The residue was passed through Prep-HPLC (chromatographic column type: gemini-C18 150 x 21.2 mm, 5µm, mobile phase: ACN-H2O (0.1% TFA), gradient 10%-55%, flow rate: 20 mL/min) to obtain the product (200 mg, yield=80.1%).

### LC-MS (ESI), m/z: [M+H]⁺ =420.8.

¹H-NMR(400MHz, CD₃OD) δ 8.68 (s, 1H), 8.05 (s, 1H), 7.74 (s, 1H), 7.43 (s, 1H), 4.54 (d, J = 8.8 Hz, 2H), 4.16 (d, J = 9.0 Hz, 2H), 3.48 (s, 2H), 3.17 - 3.11 (m, 2H), 1.33 (t, J = 7.3 Hz, 3H).

### Step C: 2-(3-(3-amino-4-(6-(1-methyl-1H-pyrazol-4-yl)imidazolo[1,2-b]pyridazin-8-yl)-1H-pyridin-1-yl)-1-(ethylsulfonyl)azaheterocycl-3-yl) acetonitrile

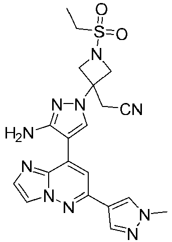

Methanesulfonic acid (2-dicyclohexylphosphon-2',4',6 '-triisopropyl-1,1'- biphenyl)(2'-amino-1,1'- biphenyl-2-yl) palladium (II) (5 mg, 0.005 mmol, 0.03eq) was added to the solution of 2-(3-(3-amino-4-(6-chloroimidazolo[1,2-b]pyridazin-8-yl)-1H-pyrazol-1-yl)-1-(ethylsulfonyl)azaheterocycl-3-yl)acetonitrile (88 mg, 0.21 mmol, 1.0eq), (1-methyl-1H-pyrazol-4-yl) boronic acid (27 mg, 0.21 mmol, 1.0eq) and sodium tert-butoxide (61 mg, 0.63 mmol, 3.0eq) in toluene/methanol (1:1, 5 mL). Under nitrogen protection, the reaction solution was stirred for 16 h at 110 °C, diluted with water (20 mL), and then extracted with ethyl acetate (10 mL * 3). The combined organic phase was washed with saturated saline solution (20 mL), dried over anhydrous sodium sulfate, and evaporated under reduced pressure to obtain the crude product. The crude product was purified using Combiflash column (petroleum ether: ethyl acetate=5:1) to obtain the product (12.8 mg, yield=13%).

### LC-MS(ESI), m/z: [M+H]⁺ =467.0.

1H-NMR (400MHz, CD₃OD) δ 8.67 (s, 1H), 8.31 (s, 1H), 8.14 (s, 1H), 8.07 (d, J = 1.2 Hz, 1H), 7.72 (d, J = 1.2 Hz, 1H), 7.67 (s, 1H), 4.61 (d, J = 9.2 Hz, 2H), 4.22 (d, J = 9.3 Hz, 2H), 4.01 (s, 3H), 3.54 (s, 2H), 3.18 (dt, J = 12.3, 6.2 Hz, 2H), 1.38 (m, 3H).

The following examples were prepared according to the experimental route and method in Example 80:

| | |
|---|---|
| Example 81 : 2-(3-(3-amino-4-(6-(1-methyl-1H-pyrazol-4-yl)-[1,2,4]triazolo[1,5-a] pyridin-8-yl)-1H-pyridin-1-yl)-1-(ethylsulfonyl)azetidine-3-yl)acetonitrile | |
| 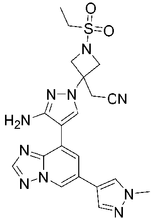 | LC-MS (ESI), m/z: [M+H]⁺ =467.0. |
| | ¹H-NMR(400MHz, CD₃OD) δ 8.93 (d, J = 1.4 Hz, 1H), 8.60 (s, 1H), 8.42 (s, 1H), 8.13 (s, 1H), 8.09 (d, J = 1.4 Hz, 1H), 7.98 (t, J = 1.8 Hz, 1H), 4.58 (d, J = 9.3 Hz, 2H), 4.20 (m, 2H), 3.95 (s, 3H), 3.53 (s, 2H), 3.14 (m, 2H), 1.34 (m, 3H). |
| Example 82: 2-(3-(3-amino-4-(5-(1-methyl-1H-pyrazol-4-yl)pyrazolo[1,5-a]pyrimidin-7-yl)-1H-pyridin-1-yl)-1-(ethylsulfonyl)azetidine-3-yl)acetonitrile | |
| 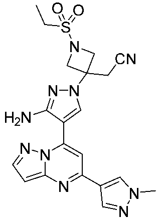 | LC-MS (ESI), m/z: [M+H]⁺ =467.0. |
| | ¹H-NMR(400MHz, CD₃OD) δ 8.75 (s, 1H), 8.24 (s, 1H), 8.08 (s, 2H), 7.40 (s, 1H), 6.51 (d, J = 2.4 Hz, 1H), 4.49 (d, J = 9.2 Hz, 2H), 4.10 (d, J = 9.3 Hz, 2H), 3.89 (s, 3H), 3.44 (s, 2H), 3.07-3.03 (m, 2H), 1.26 (t, J = 8.0 Hz, 3H). |
| Example 83: 2-(3-(3-amino-4-(6-chloro-[1,2,4]triazolo[1,5-a]pyridin-8-yl)-1H-pyrazol-1-yl)-1-(ethylsulfonyl)azetidine-3-yl)acetonitrile | |
| 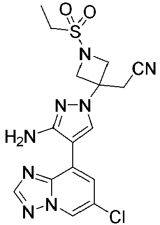 | LC-MS (ESI), m/z: [M+H]⁺ = 421. |
| | ¹H-NMR(400MHz, CD₃OD) δ 8.90 (d, J = 1.8 Hz, 1H), 8.58 (s, 1H), 8.49 (s, 1H), 7.95 (t, J = 5.0 Hz, 1H), 4.59 (d, J = 9.2 Hz, 2H), 4.21 (d, J = 9.3 Hz, 2H), 3.51 (m, 2H), 3.16 (m, 2H), 1.38 (t, J = 7.4 Hz, 3H). |
| Example 84: 2-(3-(3-amino-4-(5-chloropyrazolo[1,5-a]pyrimidin-7-yl)-1H-pyrazol-1-yl)-1-(ethylsulfonyl)azetidine-3-yl)acetonitrile | |
| 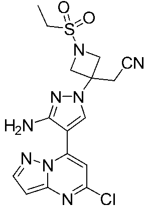 | LC-MS (ESI), m/z: [M+H]⁺ =421.0. |
| | ¹H-NMR(400MHz, CD₃OD) δ 8.93 (s, 1H), 8.23 (d, J = 2.4 Hz, 1H), 7.30 (s, 1H), 6.62 (d, J = 2.4 Hz, 1H), 4.54 (d, J = 9.3 Hz, 2H), 4.18 (d, J = 9.4 Hz, 2H), 3.51 (s, 2H), 3.12 (m, 2H), 1.33 (t, J = 7.4 Hz, 3H). |
| Example 85 : 2-(3-(3-amino-4-(5-(methylamino)pyrazolo[1,5-a]pyrimidin-7-yl)-1H-pyrazol-1-yl)-1-(ethylsulfonyl)azetidine-3-yl)acetonitrile | |
| 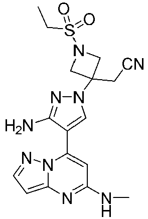 | LC-MS (ESI), m/z: [M+H]⁺ =416.0. |
| | ¹H-NMR(400MHz, CD₃OD) δ 8.70 (s, 1H), 8.55 (s, 1H), 7.46 (d, J = 3.3 Hz, 1H), 7.00 (d, J = 3.4 Hz, 1H), 4.76 (d, J = 10.2 Hz, 1H), 4.63 (m, 1H), 4.34 (m, 1H), 4.17 (dd, J = 19.2, 12.4 Hz, 1H), 3.55 (d, J = 21.3 Hz, 2H), 1.47 (s, 3H), 1.41 (s, 2H), 1.31 (m, 3H). |

### Example 86: 2-(3-(3-amino-4-(7H-pyrrolo[2,3-d]pyrimidin-4-yl)-1H-pyrazol-1-yl)-1-((2-aminopropyl)sulfonyl)azetidine-3-yl)acetonitrile

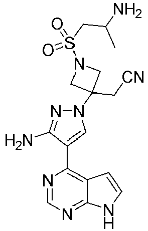

### Synthesis route:

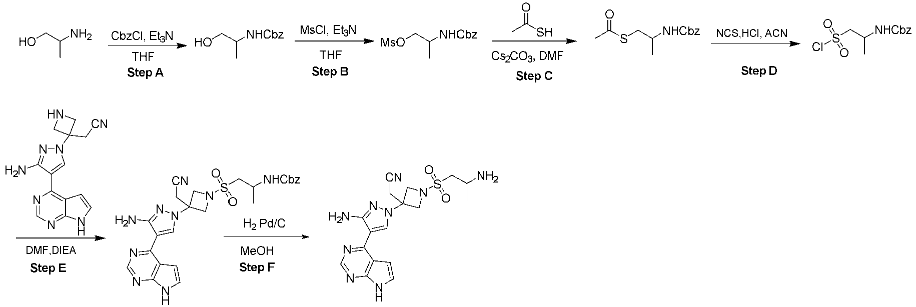

### Step A: benzyl(1-hydroxypropan-2-yl)carbamate

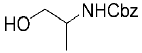

10.0 g (133 mmol, 1.0 eq) 2-aminopropane-1-ol was dissolved in 250 mL tetrahydrofuran, and then, under an ice bath, 18.7 mL (133 mmol, 1.0 eq) benzyl chloroformate and 22.2 mL (160 mmol, 1.2 eq) triethylamine was slowly dripped. Stirring was carried out overnight at room temperature. A completed reaction was confirmed by TLC, and 200 mL water was added. Then, extraction was carried out with ethyl acetate (100 mL*3). The organic layer was washed with 100 mL saturated saline solution, dried over anhydrous sodium sulfate, and evaporated under reduced pressure. The residue was passed through column chromatography (PE/EA=10/1~1:1) to obtain the product (13.6 g, yield=43%).

### LC-MS (ESI), m/z: [M+H]⁺ =210.0.

¹H-NMR(400MHz, DMSO-*d*₆) δ 7.40-7.03 (m, 5H), 7.02 (d, *J* = 8.0 Hz, 1H), 5.01 (s, 2H), 4.64 (t, *J =* 5.8 Hz, 1H), 3.48-3.55 (m, 1H), 3.32-3.37 (m, 1H), 3.17-3.23 (m, 1H), 1.02 (d, *J* = 6.8 Hz, 3H).

### Step B: 2-((benzyloxy)carbonyl)amino)propyl methane sulfonate

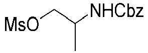

13.6 g (64.12 mmol, 1.0 eq) benzyl(1-hydroxypropan-2-yl) carbamate was dissoved in 50 mL tetrahydrofuran, and then under an ice bath, 8.8 g methanesulfonyl chloride (76.94 mmol, 1.2 eq) and 20.7 mL triethylamine (160.3 mmol, 2.5 eq) were slowly dripped. Stirring was carried out overnight at room temperature. A completed reaction was confirmed by TLC, and 100 mL water was added. Then, extraction was carried out with ethyl acetate (100 mL*3). The organic layer was washed with 100 mL saturated saline solution, dried over anhydrous sodium sulfate, and evaporated under reduced pressure. The residue was passed through column chromatography (PE/EA = 0~2:1) to obtain the product (13.4 g, yield=60.1%).

### LC-MS (ESI), m/z: [M+H]⁺ =310.0.

¹H-NMR(400MHz, CDCl₃) δ 7.41-7.32 (m, 5H), 5.13 (s, 2H), 4.89 (s, 1H), 4.29-4.27 (m, 1H), 4.21-4.15 (m, 1H), 4.09-4.06 (m, 1H), 3.00 (s, 3H), 1.29 (d, *J =* 7.0 Hz, 3H).

### Step C: S-(2-((benzyloxy)carbonyl)amino)propyl)ethyl sulfate

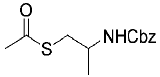

10.0 g (34.8 mmol, 1.0 eq) propyl 2- (benzyloxycarbonyl)amino)methylsulfonate was dissolved in 60 mL *N,N*-dimethylformamide, and then 22.7 g (69.6 mmol, 2.0 eq) cesium carbonate and 5.30 g (69.6 mmol, 2.0 eq) thioacetic acid were added. Stirring was carried out overnight at room temperature. After confirming that the product had been generated through LC-MS, 150 mL water was added, and then, extraction was carried out with ethyl acetate 3 times. The organic layer was washed with 100 mL saturated saline solution, dried over anhydrous sodium sulfate, and evaporated under reduced pressure. The residue was passed through column chromatography (PE/EA = 0~5:1) to obtain the product (3.34 g, yield=28%).

### LC-MS (ESI), m/z: [M+H]⁺ =268.0.

¹H-NMR(400MHz, CDCl₃) δ 8.04 (s, 1H), 7.40-7.32 (m, 5H), 5.11 (s, 2H), 3.99-3.92 (m, 1H), 3.08-3.06 (m, 2H), 2.36 (s, 3H), 1.22 (d, *J =* 6.8 Hz, 3H).

### Step D: benzyl (1-(chlorosulfonyl)prop-2-yl)carbamate

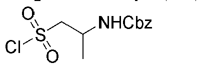

Hydrochloric acid solution (2 M, 3.34 mL, 6.7 mmol,0.5eq) was added to the solution of N-chlorosuccinimide (6.68 g, 50mmol, 3.0eq) in acetonitrile (20mL) at 0 °C. The mixture was stirred for 15 minutes. Then, *S*-(2-(benzyloxy)carbonyl)amino)propyl)ethyl sulfate (3.34g, 13mmol, 1.0eq) was dissolved in acetonitrile (20 mL) and the solution was added dropwise to the mixture. Stirring was carried out for 1 h at room temperature. A completed reaction was confirmed by TLC. The reaction solution was diluted with sodium bicarbonate (20mL) and then extracted with ethyl acetate (20mL * 3). The combined organic phase was washed with saturated saline solution (20 mL), dried over anhydrous sodium sulfate, and evaporated under reduced pressure to obtain the crude product. The crude product was purified using Combiflash column (petroleum ether: ethyl acetate=5:1) to obtain the product benzyl (1-(chlorosulfonyl)prop-2-yl)carbamate in the form of a shite solid (1.2 g, yield=29%).

### LC-MS (ESI), m/z: [M+H]⁺ =291.9.

### Step E: benzyl(1-(3-(3-amino-4-(7H-pyrrolo[2,3-d]pyrimidin-4-yl)-1H-pyrazol-1-yl)-3-(cyanomethyl)azetidin-1-yl)sulfonyl)propan-2-yl)carbamate

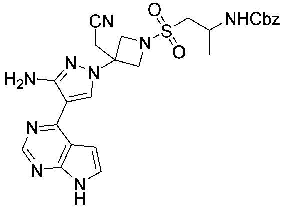

N,N-diisopropylethylamine (787 mg, 6.1 mmol, 3.0eq) and benzyl (1-(chlorosulfonyl)prop-2-yl)carbamate (1.2 g, 2.04 mmol, 1.0eq) were added to the solution of 2-(3-(3-amino-4-(7H-pyrrolo[2,3-d]pyrimidin-4-yl)-1H-pyrazol-1-yl)azetidin-3-yl)acetonitrile (600 mg, 2.04 mmol, 1.0eq) dissolved in N,N-dimethylformamide (10 mL). The reaction solution was stirred for 2 h at room temperature, and monitored by LC-MC. The reaction solution was dried with an oil pump. The crude product was purified through a reverse phase Combiflash column (acetonite: water=3:7) to obtain the product benzyl(1-(3-(3-amino-4-(7H-pyrrolo[2,3-d]pyrimidin-4-yl)-1H-pyrazol-1-yl)-3-(cyanomethyl)azetidin-1-yl)sulfonyl)propan-2-yl)carbamate in the form of a light yellow solid (500 mg, yield=45%).

### LC-MS (ESI), m/z: [M+H]⁺ =549.9.

### Step F: 2-(3-(3-amino-4-(7H-pyrrolo[2,3-d]pyrimidin-4-yl)-1H-pyrazol-1-yl)-1-(2-aminopropyl)sulfonyl)azetidin-3-yl)acetonitrile

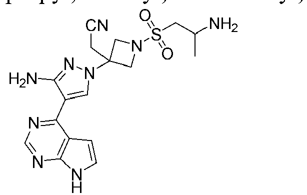

benzyl(1-(3-(3-amino-4-(7H-pyrrolo[2,3-d]pyrimidin-4-yl)-1H-pyrazol-1-yl)-3-(cyanomethyl)azetidin-1-yl)sulfonyl)propan-2-yl)carbamate was dissolved in 5 mL methanol solution, and then 19 mg (0.18 mmol, 5.0 eq) 10% palladium on carbon was added. Stirring was carried out at room temperature for 3 h in a hydrogen atmosphere. After confirming that the product had been generated through LC-MS, the reaction solution was rotary dried. Preparation and purification (mobile phase A: 0.1% formic acid +0.05% triethylamine; mobile phase B: acetonitrile; gradient: 30%-70% B, 55 min; flow rate: 70mL/min), and desalination and purification (95% acetonitrile:5% water, 5min, then 90% acetonitrile: 70% water: methanol flow rate: 70 mL/min) were carried out to obtain a white solid (3.7 mg, yield=24%).

### LC-MS: (M+H)⁺; m/z=416.0.

1H-NMR (400MHz, CD₃OD) δ 8.70 (s, 1H), 8.52 (s, 1H), 7.46 (d, *J =* 3.6 Hz, 1H), 6.98 (d, *J =* 4.0 Hz, 1H), 4.68-4.64 (m, 2H), 4.28 (d, *J =* 9.2 Hz, 2H), 3.65-3.58 (m, 1H), 3.55 (s, 2H), 3.40-3.34 (m, 1H), 3.29-3.23 (m, 1H), 1.31 (d, *J* =6.8 Hz, 3H).

The following examples were prepared according to the experimental route and method in Example 86:

| | |
|---|---|
| Example 87: (S) -2-(3-(3-amino-4-(7H-pyrrolo[2,3-d]pyrimidin-4-yl)-1H-pyrazol-1-yl)-1-((2-aminopropyl)sulfonyl)azetidine-3-yl)acetonitrile trifluoroacetate salt | |
| 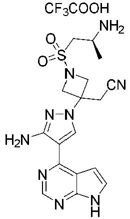 | LC-MS (ESI), m/z: [M+H]⁺ =416.0. |
| | ¹H-NMR(400MHz, CD₃OD) δ 8.78 (s, 1H), 8.58 (s, 1H), 7.60 (d, J = 3.6 Hz, 1H), 7.08 (d, J = 3.6 Hz, 1H), 4.70 (d, 2H), 4.32 (d, 2H), 3.90-3.80 (m, 1H), 3.61-3.30 (m, 4H), 1.50 (d, J =4.0Hz, 3H). |
| Example 88: (R) -2-(3-(3-amino-4-(7H-pyrrolo[2,3-d]pyrimidin-4-yl)-1H-pyrazol-1-yl)-1-((2-aminopropyl)sulfonyl)azetidine-3-yl)acetonitrile trifluoroacetate salt | |
| 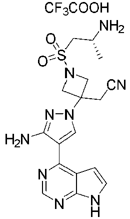 | LC-MS (ESI), m/z: [M+H]⁺ =416.0. |
| | ¹H-NMR(400MHz, DMSO-*d*6) δ 8.70 (s, 1H), 8.63 (s, 1H), 7.59 (d, J = 3.6 Hz, 1H), 7.08 (d, J = 3.6 Hz, 1H), 4.60 (t, J = 9.0 Hz, 2H), 4.22 (d, J = 8.6 Hz, 2H), 3.71 (dd, J = 12.8, 6.4 Hz, 1H), 3.64 (s, 2H), 3.54 (dd, J = 14.4, 6.4 Hz, 2H), 1.38 (t, J = 11.9 Hz, 3H). |

### Biological activity experiment

### 1. Detection of enzymatic activity (IC₅₀) of compounds

(1) ROCK1/2 detection experimental method
   (a) 50 nL of diluted compound working solution was transferred to each well of the reaction plate (784075, Greiner) using Echo 655.
   (b) the reaction plate was sealed with a sealing film and centrifugation was carried out at 1000g for 1 minute.
   (c) kinase solution was prepared.
   (d) 5 µL of kinase solution was added to each well of the reaction plate. The plate was sealed with a sealing film and centrifugation was carried out at 1000g for 30 seconds, then it was left at room temperature for 10 minutes.
   (e) a mixture of STK2 substrate biotin kinase and ATP was prepared.
   (f) 5 µL of the mixture of STK2 substrate biotin kinase and ATP was added to the reaction plate, centrifugation was carried out at 1000g for 30 seconds, and the reaction started.
   (g) ROCK1 kinase reacted at room temperature for 20 minutes, and ROCK2 kinase reacted at room temperature for 30 minutes.
   (h) a mixture of Sa-XL 665 (125 nM) and STK antibody Cryptate was prepared using HTRF detection buffer.
   (i) 10 µL of the mixture of Sa-XL 665 and STK antibody Cryptate was added to each well, centrifugation was carried out at 1000 g for 30 seconds, and the reaction was carried out at room temperature for 1 hour.

Envision 2104 was used to read 615 nm (Cryptate) and 665 nm (XL665) signals, and the signal intensity was used to characterize the activity level of the kinase.

The kinase activity data was expressed by comparing the kinase activity of the test compound and that of the blank group (containing only DMSO), and the IC₅₀ value was obtained by curve fitting using Prism software (GraphPad7.0).

The specific test results are shown in Table 1 below:

**Table 1: enzymatic activity of compounds (IC₅₀)**

| Examples | IC₅₀ (nM) | |
|---|---|---|
| | ROCK1 | ROCK2 |
| 1 | <10 | <10 |
| 2 | <5 | <5 |
| 3 | <5 | <5 |
| 4 | <5 | <5 |
| 5 | <5 | <5 |
| 6 | <5 | <5 |
| 7 | <5 | <5 |
| 8 | <5 | <5 |
| 9 | <10 | <10 |
| 10 | <5 | <5 |
| 11 | <10 | <10 |
| 12 | <50 | <50 |
| 13 | <50 | <50 |
| 14 | <5 | <5 |
| 15 | <50 | <50 |
| 16 | <50 | <50 |
| 17 | <50 | <50 |
| 18 | <5 | <5 |
| 19 | <10 | <10 |
| 20 | <10 | <10 |
| 21 | <5 | <10 |
| 22 | <50 | <50 |
| 23 | <50 | <50 |
| 24 | <50 | <50 |
| 25 | <10 | <10 |
| 26 | <10 | <10 |
| 27 | <10 | <10 |
| 28 | <50 | <50 |
| 29 | <10 | <10 |
| 30 | <5 | <10 |
| 31 | <5 | <5 |
| 32 | <5 | <10 |
| 33 | <10 | <10 |
| 34 | <10 | <10 |
| 35 | <10 | <10 |
| 36 | <5 | <5 |
| 37 | <50 | <50 |
| 38 | <5 | <5 |
| 39 | <50 | <50 |
| 40 | <10 | <10 |
| 41 | <10 | <10 |
| 42 | <100 | <50 |
| 43 | <10 | <10 |
| 44 | <50 | <50 |
| 45 | <5 | <5 |
| 46 | <10 | <5 |
| 47 | <5 | <5 |
| 48 | <50 | <10 |
| 49 | <10 | <10 |
| 50 | <10 | <10 |
| 51 | <10 | <10 |
| 52 | <10 | <10 |
| 53 | <10 | <10 |
| 54 | <50 | <50 |
| 55 | <50 | >100 |
| 56 | <50 | >100 |
| 57 | <50 | >100 |
| 58 | <50 | >100 |
| 59 | <50 | >100 |
| 60 | >100 | >100 |
| 61 | >100 | >100 |
| 62 | >100 | >100 |
| 63 | >100 | >100 |
| 64 | >100 | >100 |
| 65 | >100 | >100 |
| 66 | >100 | >100 |
| 67 | >100 | >100 |
| 68 | >100 | >100 |
| 69 | >100 | >100 |
| 70 | >100 | >100 |
| 71 | >100 | >100 |
| 72 | >100 | >100 |
| 73 | >100 | >100 |
| 74 | >100 | >100 |
| 75 | >100 | >100 |
| 76 | >100 | >100 |
| 77 | >100 | >100 |
| 78 | >100 | >100 |
| 79 | >100 | >100 |
| 80 | >100 | >100 |
| 81 | >100 | >100 |
| 82 | >100 | >100 |
| 83 | >100 | >100 |
| 84 | >100 | >100 |
| 85 | >100 | >100 |
| 86 | <5 | <5 |
| 87 | <5 | <5 |
| 88 | <5 | <5 |
| Ripasudil | 22.02 | 29.62 |
| Netarsudil | 6.13 | 7.75 |
| Belumosudil | >1000 | 192 |

It can be seen from the data in the table that the enzymatic activity of the compounds in the embodiments of the present disclosure is better than that of the marketed ROCK inhibitors Ripasudil, Netarsudil, and Belumosudil. Some compounds exhibit strong activity against the ROCK 1/2 kinase and can be used to regulate the treatment of diseases mediated by the ROCK kinase,

Wherein Ripasudil has the following structural formula:

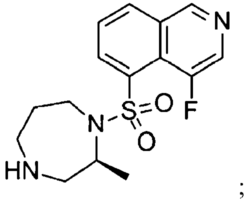

Netarsudil has the following structural formula:

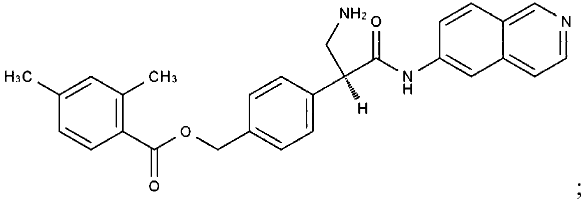

Belumosudil has the following structural formula:

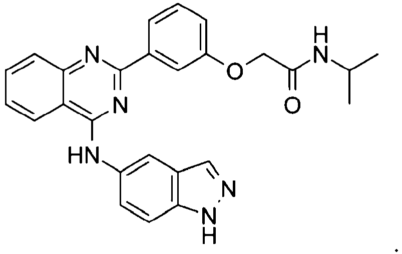

### 2. Cell Activity (IC₅₀) Detection of the compounds (In Cell Western for detecting phosphorylation of myosin light chain )

ROCK resulted in changes in the cytoskeleton by phosphorylating two amino acid sites of myosin light chain T18/S19. The rat smooth muscle cell line A7r5 was used, and the A7r5 cell culture conditions were DMEM containing 10% FBS. Phspho-MLC-T18/S19 specific antibody and a second detection antibody were used to detect the phosphorylation level of myosin light chain by In Cell Western. Cells treated with positive compounds were used as positive controls, while the group without compounds but with compound solvents was used as negative controls. DRAQ5 stained cell nuclei were used as internal references. GraphPad Prism 7.0 software was used, and fitting was carried out by using a non-linear regression curve with varying slopes to determine the absolute IC₅₀ value.

On the first day, A7r5 cells were resuspended in serum-free medium and seeded into 384 wells of PDL coated transparent black plates at a density of 5000 cells per well. Serum starvation culture was performed for 4 hours, and the cells were incubated with the compound in serum-free medium for 1 hour. 50 µL of 8% PFA (polyformaldehyde) was added to each well and fixed at room temperature for 1 hour. The liquid in the wells was discard and 90 µL of ice methanol was added to each well to permeabilize the cells at 4 °C for 1 hour. Then, PBST (0.1% Tween20-PBS) was added using an automatic separator to wash three times. After patting the plate for drying, 50 µL blocking solution was added to each well for sealing at room temperature for 1 hour. The phspho-MLC-T18/S19 specific antibody was diluted with the blocking solution at a ratio of 1:200. After adding 20 µL to each well, incubation was carried out overnight at 4 °C with a sealed film.

On the second day, the liquid in the wells was discarded and PBST was added using an automatic separator to wash the plate 5 times. After patting the plate for drying, the second detection antibody was diluted with blocking solution at a ratio of 1:800 and DRAQ5 at a ratio of 1:1000. After adding 20 µL to each well, incubation was carried out at room temperature for 1 hour. Then, the liquid in the wells was discarded and PBST was added using an automatic separator to wash the plate 3 times, followed by adding ddH2O to wash the plate 3 times. After patting the plate for drying, it was scaned using a LICOR Odyssey near-infrared imaging scanner.

The specific test results are shown in Table 2 below:

**Table 2: cellular activity of compounds (IC₅₀)**

| Examples | IC₅₀(µM) | Examples | IC₅₀(µM) | Examples | IC₅₀(µM) |
|---|---|---|---|---|---|
| 1 | 5.014 | 19 | >10 | 50 | 0.098 |
| 2 | 0.011 | 31 | 0.381 | 55 | >10 |
| 3 | 0.178 | 34 | 0.551 | 61 | >10 |
| 5 | 0.951 | 37 | 1.594 | 62 | >10 |
| 6 | 0.181 | 38 | 3.131 | 69 | >10 |
| 7 | 0.132 | 40 | 1.566 | 82 | >10 |
| 8 | 0.152 | 41 | 0.091 | 87 | 0.678 |
| 18 | 4.110 | 45 | 0.579 | 88 | 0.826 |
| Ripasudil | 1.685 | Netarsudil | 0.288 | Belumosudil | 3.941 |

The compounds in the embodiments of the present disclosure have better cellular activity compared to the marketed ROCK inhibitors Ripasudil, Netarsudil, and Belumosudil. They have a stronger ability to induce changes in the cytoskeleton by phosphorylating two amino acid sites of myosin light chain T18/S19. Therefore, the application value of the compound of the present disclosure will be more extensive.

### 3. The role of compounds in SD rat glaucoma model induced by episcleral veins cauterization

Experimental method: after passing the quarantine, male SD rats were taken and subjected to general anesthesia with isoflurane. The experimental eye (right eye) was given surface anesthesia with Propantheline Hydrochloride eye drops. The eyelids were opened, and the bulbar conjunctiva was cut along the upper corneoscleral edge. The fascia was bluntly separated, and the episcleral vein was exposed. The two episcleral veins adjacent to the superior rectus and one episcleral vein on the temporal side of the rat were cauterized. The hallmark of a successful cauterization is the dilation of blood vessels in the proximal segment of the corneoscleral, disappearance of blood flow in the distal segment of the corneoscleral, and no bleeding. After surgery, the conjunctiva was intermittently sutured with 9-0 non absorbable surgical sutures, and levofloxacin eye drops were administered after suturing. After the rats regained consciousness, they were placed back in the cage.

The wound was disinfected with iodine before and after surgery; postoperative levofloxacin eye drops were applied twice a day for one week; Carprofen was administered once 30 minutes before surgery and twice after surgery, with a 24-hour interval between each dose (subcutaneous injection, 10mg/kg, 1mL/kg).

On the third day after modeling, relatively uniform animals were selected for random grouping based on intraocular pressure and body weight. Randomly, it is divided into a normal control group, a model control group, a positive control group, and a test substance group, with 5 animals in each group. After modeling, the positive control substance (commercially available Rhopress eye drops: 0.02% betarsudil ophthalmic solution) or the test substance (0.01% Example 2) was administered via conjunctival sac eye drops once a day, with one drop each time, for a total of 14 days (14 times in total). The day of the first administration was defined as the first day of the experiment (D1).

During the experiment, the general condition of each group of animals was observed daily; body weight was measured at least once a week after administration, and intraocular pressure was measured within 24 hours on days 1, 3, 7, 10, and 14 of administration; at the end of drug administration, ophthalmic examination was conducted on the animals dissected according to the plan. After euthanasia, gross anatomical observation and histopathological examination were performed (BRN3A immunofluorescence staining: counting the number of RGCs in the retina of one field of view under a 20x objective lens at a distance of 2 mm from the optic nerve, and counting the number of RGCs in a circle of retina under a slice).

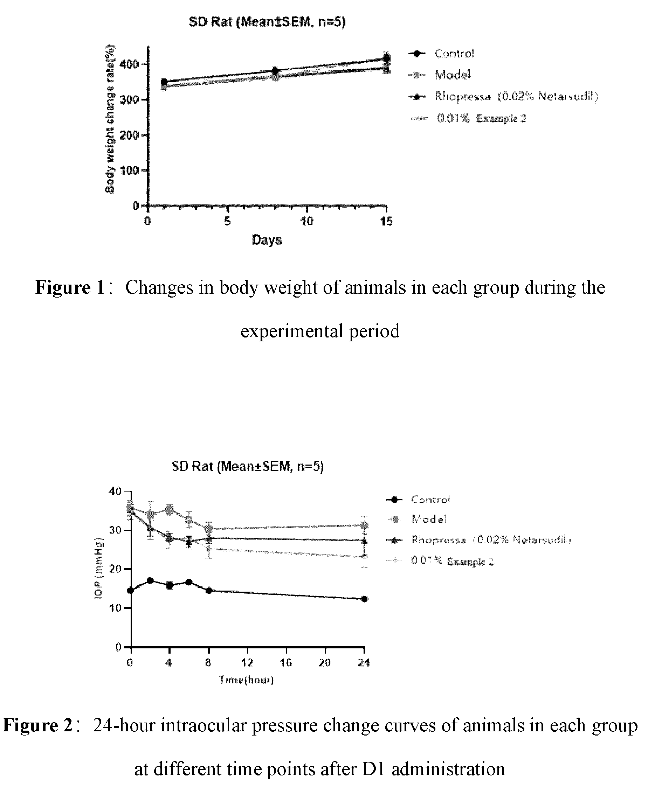

Experimental results:
Weight: at the end of the experiment, the body weight of each group of rats showed a stable upward trend, and there was no significant difference in body weight and weight gain among the groups of rats (P>0.05). As shown in Figure 1.

Intraocular pressure: the data of intraocular pressure changes at different time points of 24 hours on the 1st, 3rd, 7th, 10th, and 14th days of administration showed that the intraocular pressure of animals in each treatment group showed a decreasing trend until 8 hours after administration. Compared with the model control group, the intraocular pressure significantly decreased at 8 hours; at 24 hours, the intraocular pressure tended to stabilize, and the intraocular pressure of the model group animals remained higher than that of each treatment group. The intraocular pressure data is shown in Figure 2.

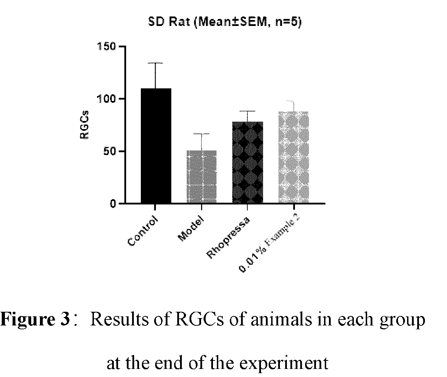

Histopathology: compared with the model control group, the number of RGCs in the positive control group and the test substance group increased to a certain extent. As shown in Figure 3.

The experimental results showed that through the SD rat glaucoma model induced by episcleral veins cauterization, the compounds of the present disclosure have a certain degree of improvement effect on glaucoma and a certain protective effect on rat RGCs after daily eye drops administration on unilateral conjunctival sac for 14 consecutive days.

The above description combines preferred embodiments to explain the present application, but these embodiments are only exemplary and serve only illustrative purposes. On this basis, various substitutions and modifications can be made to the present application, all of which fall within the scope of protection of the present application.

## Claims

1. An azacycloalkane compound shown in formula (I), or pharmaceutically acceptable salts, solvates, active metabolites, polymorphs, isotopic markers, isomers or prodrugs thereof: 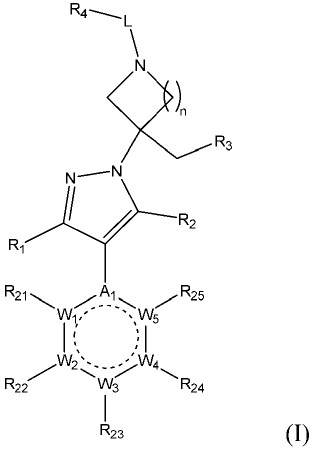
wherein R₁ is -NHR₁₁,-OR₁₁, -SR₁₁ or -C(=O)NHR₁₁; wherein R₁₁ is each independently selected from the group consisting of hydrogen, substituted or unsubstituted C_{1~6} alkyl, substituted or unsubstituted C₂₋₆ alkenyl, substituted or unsubstituted C₂₋₆ alkynyl, substituted or unsubstituted C_{3~6} cycloalkyl, substituted or unsubstituted C_{3~8} heterocyclyl, substituted or unsubstituted C_{6~20} aryl, substituted or unsubstituted C_{5~20} heteroaryl, substituted or unsubstituted C_{1~6} alkylacyl, substituted or unsubstituted C_{1~6} alkylsulfonyl, substituted or unsubstituted C_{5~20} heteroarylacyl, and substituted or unsubstituted C_{1~6} alkoxy; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, -C(=O)R₃₃, hydroxyl, mercapto, substituted C_{3~8} cycloalkyl, substituted C_{3~8} heterocyclyl, substituted C_{6~20} aryl, and substituted C_{5~20} heteroaryl;
R₂ is selected from the group consisting of hydrogen, halogen, substituted or unsubstituted C_{1~6} alkyl, substituted or unsubstituted C₂₋₆ alkenyl, substituted or unsubstituted C₂₋₆ alkynyl, substituted or unsubstituted C_{1~6} haloalkyl, substituted or unsubstituted C_{1~6} alkoxy, cyano, - NR₃₁R₃₂, hydroxyl, carboxyl, and mercapto; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₃ is selected from the group consisting of cyano, -CONH₂, and carboxyl;
L is selected from the group consisting of -S(=O)₂-, -C(=O)-, -CH₂-, and -S(=O)(=N)R_{L}-, wherein R_{L} is selected from the group consisting of hydrogen, and substituted or unsubstituted C_{1~8} alkyl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, - NR₃₁R₃₂, hydroxyl, carboxyl, and mercapto;
R₄ is selected from the group consisting of halogen, substituted or unsubstituted C_{1~8} alkyl, -NR₃₁R₃₂, substituted or unsubstituted C_{1~8} alkoxy, substituted or unsubstituted C₂₋₈ alkenyl, substituted or unsubstituted C₂₋₈ alkynyl, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, hydrazide, C_{1~8} alkylsulfonyl, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, -COR₃₃, carboxyl, and mercapto;
A₁ is selected from C atom;
W₁, W₂, W₃, W₄ and W₅ are each independently selected from the group consisting of C atom and N atom, and one, two, or three of W₁, W₂, W₃, W₄ and W₅ are N atom; the dashed circle represents the bonds that form aromatic ring;
R₂₁ is selected from the group consisting of hydrogen, halogen, -NR₃₁R₃₂, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₂₂ is selected from the group consisting of hydrogen, halogen, -NR₃₁R₃₂, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₂₃ is selected from the group consisting of hydrogen, halogen, -NR₃₁R₃₂, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₂₄ is selected from the group consisting of hydrogen, halogen, -NR₃₁R₃₂, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₂₅ is selected from the group consisting of hydrogen, halogen, -NR₃₁R₃₂, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
or, R₂₄, and W₄ connected to R₂₄ together with R₂₅ and W₅ connected to R₂₅ form a 5-membered aryl, cycloalkyl, heteroaryl, or heterocycloalkyl ring fused with a 6-membered ring formed by A₁, W₁, W₂, W₃, W₄ and W₅;
R₃₁ and R₃₂ are each independently selected from the group consisting of hydrogen, sulfonamido, substituted or unsubstituted C_{1~6} alkyl, substituted or unsubstituted C₂₋₆ alkenyl, substituted or unsubstituted C₂₋₆ alkynyl, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, -C(=O)OR₃₄, and mercapto;
R₃₃ is selected from the group consisting of hydrogen, substituted or unsubstituted C_{1~8} alkyl, substituted or unsubstituted C_{1~8} alkoxy, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, or mercapto;
R₃₄ is selected from the group consisting of hydrogen, substituted or unsubstituted C_{1~8} alkyl, substituted or unsubstituted C_{1~8} haloalkyl, substituted or unsubstituted C_{1~8} alkoxy, and substituted or unsubstituted C_{3~8} cycloalkyl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, carboxyl, and mercapto;
n is 1, 2 or 3;
and, the compound is not the following compounds:

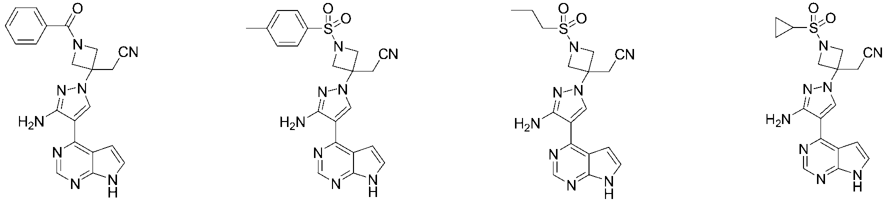

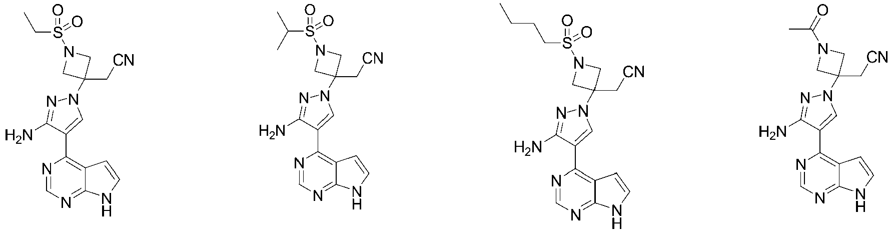

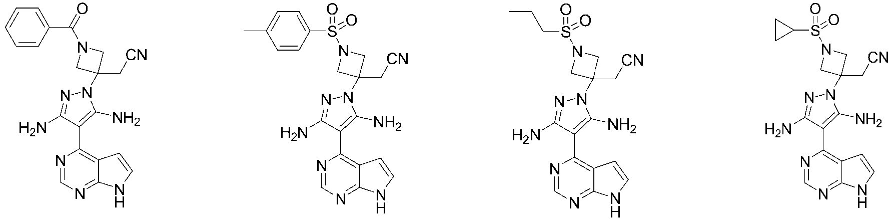

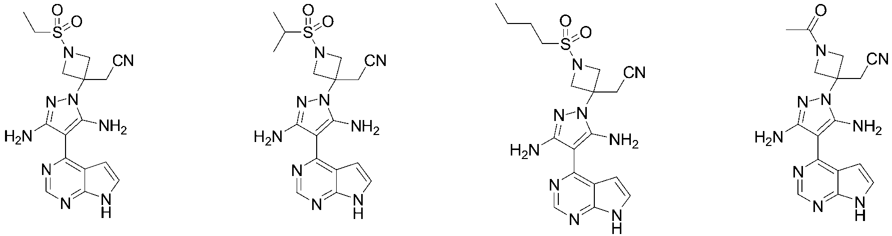

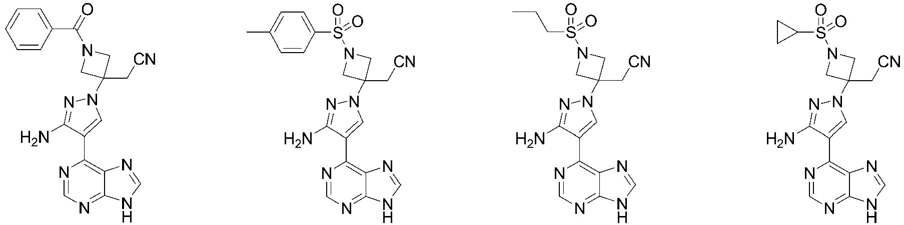

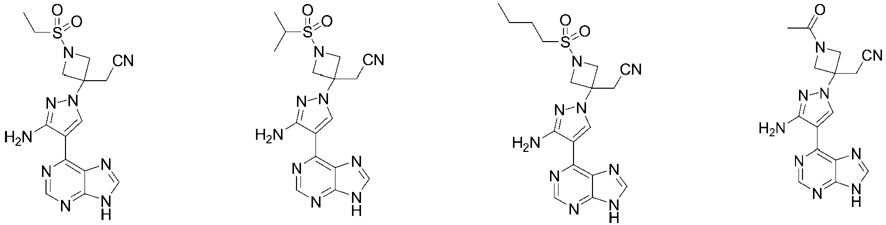

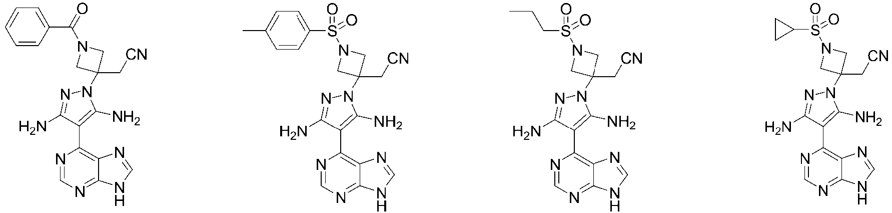

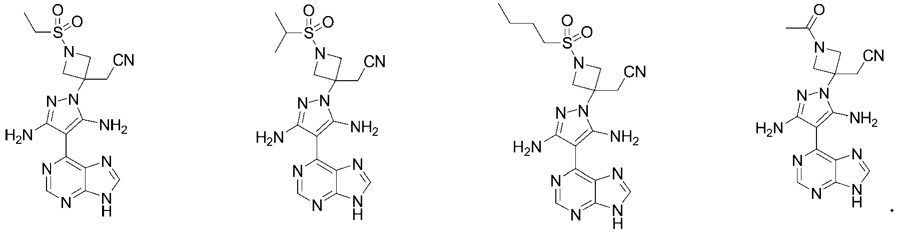

2. The azacycloalkane compound or pharmaceutically acceptable salts, solvates, active metabolites, polymorphs, isotopic markers, isomers or prodrugs thereof according to claim 1, wherein
R₁ is selected from -NHR₁₁, wherein R₁₁ is each independently selected from the group consisting of hydrogen, substituted or unsubstituted C_{1~6} alkyl, substituted or unsubstituted C₂₋₆ alkenyl, substituted or unsubstituted C₂₋₆ alkynyl, substituted or unsubstituted C_{3~6} cycloalkyl, substituted or unsubstituted C_{3~8} heterocyclyl, substituted or unsubstituted C_{6~20} aryl, substituted or unsubstituted C_{5~20} heteroaryl, substituted or unsubstituted C_{1~6} alkylacyl, substituted or unsubstituted C_{1~6} alkylsulfonyl, substituted or unsubstituted C_{5~20} heteroarylacyl, and substituted or unsubstituted C_{1~6} alkoxy; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, -C(=O)R₃₃, hydroxyl, mercapto, substituted C_{3~8} cycloalkyl, substituted C_{3~8} heterocyclyl, substituted C_{6~20} aryl, and substituted C_{5~20} heteroaryl;
wherein R₃₁ and R₃₂ are each independently selected from the group consisting of hydrogen, substituted or unsubstituted C_{1~6} alkyl, substituted or unsubstituted C_{1~6} alkyl, substituted or unsubstituted C₂₋₆ alkenyl, substituted or unsubstituted C₂₋₆ alkynyl, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, -C(=O)OR₃₄, and mercapto;
R₃₃ is selected from the group consisting of hydrogen, substituted or unsubstituted C_{1~8} alkyl, substituted or unsubstituted C_{1~8} alkoxy, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₃₄ is selected from the group consisting of hydrogen, substituted or unsubstituted C_{1~8} alkyl, substituted or unsubstituted C_{1~8} haloalkyl, substituted or unsubstituted C_{1~8} alkoxy, and substituted or unsubstituted C_{3~8} cycloalkyl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, carboxyl, and mercapto.

3. The azacycloalkane compound or pharmaceutically acceptable salts, solvates, active metabolites, polymorphs, isotopic markers, isomers or prodrugs thereof according to claim 2, wherein R₁₁ is selected from the group consisting of hydrogen and substituted or unsubstituted C_{1~6} alkyl, and the substituent is selected from the group consisting of -NR₃₁R₃₂, -C(=O)R₃₃ and substituted or unsubstituted C_{3~8} heterocyclyl.

4. The azacycloalkane compound or pharmaceutically acceptable salts, solvates, active metabolites, polymorphs, isotopic markers, isomers or prodrugs thereof according to claim 2, wherein R₁₁ is selected from substituted or unsubstituted C_{1~6} alkyl, and the substituent is selected from the group consisting of -NR₃₁R₃₂, wherein R₃₁ and R₃₂ are each independently hydrogen, substituted or unsubstituted C_{1~6} alkyl, or substituted or unsubstituted C_{3~8} heterocyclyl.

5. The azacycloalkane compound or pharmaceutically acceptable salts, solvates, active metabolites, polymorphs, isotopic markers, isomers or prodrugs thereof according to claim 2, wherein R₁₁ is selected from substituted or unsubstituted C_{1~6} alkyl, and the substituent is selected from the group consisting of -C(=O)R₃₃, wherein R₃₃ is independently substituted or unsubstituted C_{1~6} alkyl, or substituted or unsubstituted C_{3~8} heterocyclyl.

6. The azacycloalkane compound or pharmaceutically acceptable salts, solvates, active metabolites, polymorphs, isotopic markers, isomers or prodrugs thereof according to claim 2, wherein R₁ is selected from the group consisting of -NH₂, -NHC(=O)CH₃, 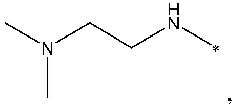 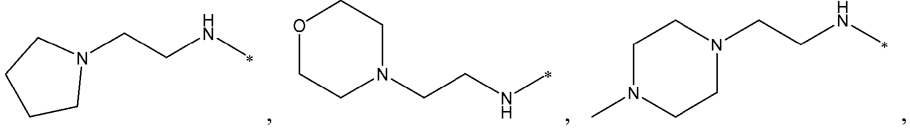 and

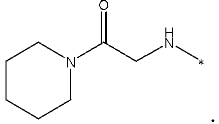

7. The azacycloalkane compound or pharmaceutically acceptable salts, solvates, active metabolites, polymorphs, isotopic markers, isomers or prodrugs thereof according to claim 1, wherein
R₁ is selected from the group consisting of -OR₁₁, wherein R₁₁ is each independently selected from the group consisting of hydrogen, substituted or unsubstituted C_{1~6} alkyl, substituted or unsubstituted C₂₋₆ alkenyl, substituted or unsubstituted C₂₋₆ alkynyl, substituted or unsubstituted C_{3~6} cycloalkyl, substituted or unsubstituted C_{3~8} heterocyclyl, substituted or unsubstituted C_{6~20} aryl, substituted or unsubstituted C_{5~20} heteroaryl, substituted or unsubstituted C_{1~6} alkylacyl, substituted or unsubstituted C_{1~6} alkylsulfonyl, substituted or unsubstituted C_{5~20} heteroarylacyl, and substituted or unsubstituted C_{1~6} alkoxy; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, -C(=O)R₃₃, hydroxyl, and mercapto ;
preferably, R₁₁ is selected from the group consisting of hydrogen and substituted or unsubstituted C_{1~6} alkyl, and the substituent is selected from the group consisting of -NR₃₁R₃₂, - C(=O)R₃₃, and substituted or unsubstituted C_{3~8} heterocyclyl;
wherein R₃₁ and R₃₂ are each independently selected from the group consisting of hydrogen, substituted or unsubstituted C_{1~6} alkyl, substituted or unsubstituted C_{1~6} alkyl, substituted or unsubstituted C₂₋₆ alkenyl, substituted or unsubstituted C₂₋₆ alkynyl, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, -C(=O)OR₃₄, and mercapto;
R₃₃ is selected from the group consisting of hydrogen, substituted or unsubstituted C_{1~8} alkyl, substituted or unsubstituted C_{1~8} alkoxy, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₃₄ is selected from the group consisting of hydrogen, substituted or unsubstituted C_{1~8} alkyl, substituted or unsubstituted C_{1~8} haloalkyl, substituted or unsubstituted C_{1~8} alkoxy, and substituted or unsubstituted C_{3~8} cycloalkyl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, carboxyl, and mercapto.

8. The azacycloalkane compound or pharmaceutically acceptable salts, solvates, active metabolites, polymorphs, isotopic markers, isomers or prodrugs thereof according to claim 1, wherein
R₁ is selected from -C(=O)NHR₁₁, wherein R₁₁ is each independently selected from the group consisting of hydrogen, substituted or unsubstituted C_{1~6} alkyl, substituted or unsubstituted C₂₋₆ alkenyl, substituted or unsubstituted C₂₋₆ alkynyl, substituted or unsubstituted C_{3~6} cycloalkyl, substituted or unsubstituted C_{3~8} heterocyclyl, substituted or unsubstituted C_{6~20} aryl, substituted or unsubstituted C_{5~20} heteroaryl, substituted or unsubstituted C_{1~6} alkylacyl, substituted or unsubstituted C_{1~6} alkylsulfonyl, substituted or unsubstituted C_{5~20} heteroarylacyl, and substituted or unsubstituted C_{1~6} alkoxy; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, -C(=O)R₃₃, hydroxyl, and mercapto;
preferably, R₁₁ is selected from the group consisting of hydrogen and substituted or unsubstituted C_{1~6} alkyl, and the substituent is selected from the group consisting of -NR₃₁R₃₂, - C(=O)R₃₃, and substituted or unsubstituted C_{3~8} heterocyclyl;
wherein R₃₁ and R₃₂ are each independently selected from the group consisting of hydrogen, substituted or unsubstituted C_{1~6} alkyl, substituted or unsubstituted C_{1~6} alkyl, substituted or unsubstituted C₂₋₆ alkenyl, substituted or unsubstituted C₂₋₆ alkynyl, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, -C(=O)OR₃₄, and mercapto;
R₃₃ is selected from the group consisting of hydrogen, substituted or unsubstituted C_{1~8} alkyl, substituted or unsubstituted C_{1~8} alkoxy, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₃₄ is selected from the group consisting of hydrogen, substituted or unsubstituted C_{1~8} alkyl, substituted or unsubstituted C_{1~8} haloalkyl, substituted or unsubstituted C_{1~8} alkoxy, and substituted or unsubstituted C_{3~8} cycloalkyl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, carboxyl, and mercapto.

9. The azacycloalkane compound or pharmaceutically acceptable salts, solvates, active metabolites, polymorphs, isotopic markers, isomers or prodrugs thereof according to claim 1, wherein
R₂ is selected from hydrogen, R₃ is selected from cyano.

10. The azacycloalkane compound or pharmaceutically acceptable salts, solvates, active metabolites, polymorphs, isotopic markers, isomers or prodrugs thereof according to claim 1, wherein 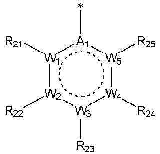 is selected from the group consisting of the following structures A1-A7: 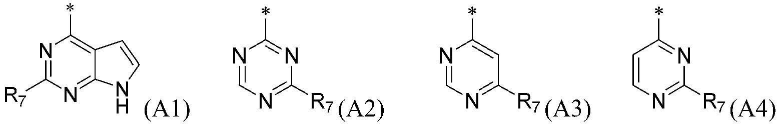 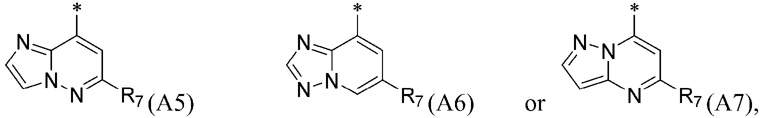
wherein, in the structures A1-A7, R₇ is each independently selected from the group consisting of hydrogen, halogen, -NR₃₁R₃₂, and substituted or unsubstituted C_{5~20} heteroaryl,
wherein R₃₁ and R₃₂ are each independently selected from the group consisting of hydrogen, substituted or unsubstituted C_{1~6} alkyl, substituted or unsubstituted C_{6~20} aryl, substituted or unsubstituted C_{5~20} heteroaryl, and substituted or unsubstituted C_{3~8} heterocyclyl;
wherein the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, carboxyl, and mercapto.

11. The azacycloalkane compound or pharmaceutically acceptable salts, solvates, active metabolites, polymorphs, isotopic markers, isomers or prodrugs thereof according to claim 10, wherein
R₇ is each independently selected from the group consisting of hydrogen, -NR₃₁R₃₂, and substituted or unsubstituted C_{5~20} heteroaryl,
wherein R₃₁ and R₃₂ are each independently selected from the group consisting of hydrogen, substituted or unsubstituted C_{1~6} alkyl, and substituted or unsubstituted C_{5~20} heteroaryl;
wherein the substituent is selected from C_{1~8} alkyl.

12. The azacycloalkane compound or pharmaceutically acceptable salts, solvates, active metabolites, polymorphs, isotopic markers, isomers or prodrugs thereof according to claim 10, wherein 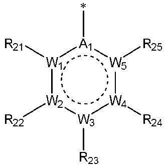 is the following structure A1: 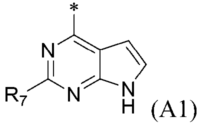 wherein, in the structure A1, R₇ is hydrogen.

13. The azacycloalkane compound or pharmaceutically acceptable salts, solvates, active metabolites, polymorphs, isotopic markers, isomers or prodrugs thereof according to claim 10, wherein 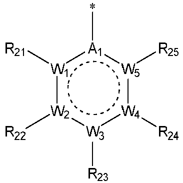 is the following structure A2: 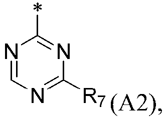
wherein, in the structure A2, R₇ is -NR₃₁R₃₂, or substituted or unsubstituted C_{5~20} heteroaryl,
wherein R₃₁ and R₃₂ are each independently selected from the group consisting of hydrogen, substituted or unsubstituted C_{1~6} alkyl, substituted or unsubstituted C_{5~20} heteroaryl, and substituted or unsubstituted C_{3~8} heterocyclyl;
wherein the substituent is selected from the group consisting of C_{1~8} alkyl and hydroxyl; or, 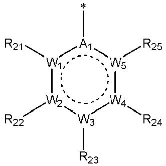 is the following structure A3: 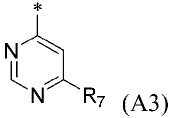
wherein, in the structure A3, R₇ is -NR₃₁R₃₂, or substituted or unsubstituted C_{5~20} heteroaryl,
wherein R₃₁ and R₃₂ are each independently selected from the group consisting of hydrogen, substituted or unsubstituted C_{1~6} alkyl, substituted or unsubstituted C_{5~20} heteroaryl, and substituted or unsubstituted C_{3~8} heterocyclyl;
wherein the substituent is selected from the group consisting of C_{1~8} alkyl, and hydroxyl; or, 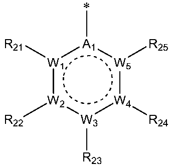 is the following structure A4: 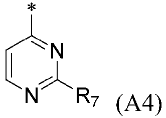
wherein, in the structure A4, R₇ is -NR₃₁R₃₂, or substituted or unsubstituted C_{5~20} heteroaryl,
wherein R₃₁ and R₃₂ are each independently selected from the group consisting of hydrogen, substituted or unsubstituted C_{1~6} alkyl, substituted or unsubstituted C_{5~20} heteroaryl, and substituted or unsubstituted C_{3~8} heterocyclyl;
wherein the substituent is selected from the group consisting of C_{1~8} alkyl, and hydroxyl.

14. The azacycloalkane compound or pharmaceutically acceptable salts, solvates, active metabolites, polymorphs, isotopic markers, isomers or prodrugs thereof according to claim 10, wherein 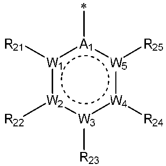 is the following structure A5: 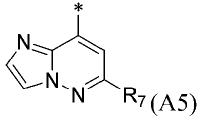
wherein, in the structure A5, R₇ is -NR₃₁R₃₂, and substituted or unsubstituted C_{5~20} heteroaryl,
wherein R₃₁ and R₃₂ are each independently selected from the group consisting of hydrogen, substituted or unsubstituted C_{1~6} alkyl, and substituted or unsubstituted C_{5~20} heteroaryl;
wherein the substituent is selected from the group consisting of C_{1~8} alkyl, and hydroxyl; or, 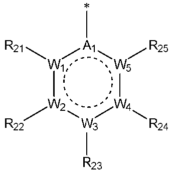 is the following structure A6: 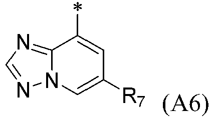
wherein, in the structure A6, R₇ is halogen, -NR₃₁R₃₂, or substituted or unsubstituted C_{5~20} heteroaryl,
wherein R₃₁ and R₃₂ are each independently selected from the group consisting of hydrogen, substituted or unsubstituted C_{1~6} alkyl, and substituted or unsubstituted C_{5~20} heteroaryl;
wherein the substituent is selected from the group consisting of C_{1~8} alkyl, and hydroxyl; or, 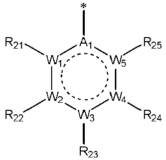 is the following structure A7: 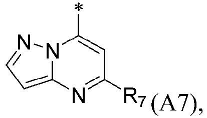
wherein, in the structure A7, R₇ is -NR₃₁R₃₂, or substituted or unsubstituted C_{5~20} heteroaryl,
wherein R₃₁ and R₃₂ are each independently selected from the group consisting of hydrogen, substituted or unsubstituted C_{1~6} alkyl, and substituted or unsubstituted C_{5~20} heteroaryl;
wherein the substituent is selected from the group consisting of C_{1~8} alkyl, and hydroxyl.

15. The azacycloalkane compound or pharmaceutically acceptable salts, solvates, active metabolites, polymorphs, isotopic markers, isomers or prodrugs thereof according to any one of claims 10 to 11 or 13 to 14, wherein R₇ is selected from the group consisting of -NHCH₃, - NHCH₂CH₂OH, Cl,

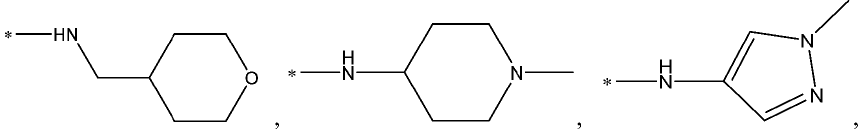

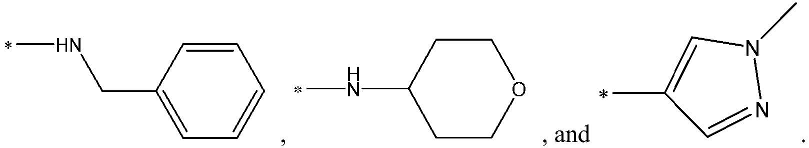

16. The azacycloalkane compound or pharmaceutically acceptable salts, solvates, active metabolites, polymorphs, isotopic markers, isomers or prodrugs thereof according to claim 1, wherein L is selected from the group consisting of -S(=O)₂-, -C(=O)-, and -S(=O)(=N)R_{L}-, wherein R_{L} is selected from the group consisting of hydrogen, and C_{1~6} alkyl.

17. The azacycloalkane compound or pharmaceutically acceptable salts, solvates, active metabolites, polymorphs, isotopic markers, isomers or prodrugs thereof according to claim 1, wherein
R₄ is selected from the group consisting of substituted or unsubstituted C_{1~8} alkyl, - NR₃₁R₃₂, substituted or unsubstituted C_{1~8} alkoxy, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, hydrazide, C_{1~8} alkylsulfonyl, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, -COR₃₃, carboxyl, and mercapto;
wherein R₃₁ and R₃₂ are each independently selected from the group consisting of hydrogen, sulfonamido, substituted or unsubstituted C_{1~6} alkyl, substituted or unsubstituted C_{1~6} alkyl, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} alkoxy, C_{3~8} cycloalkyl, cyano, hydroxyl, -C(=O)OR₃₄, and mercapto;
R₃₃ is selected from the group consisting of hydrogen, substituted or unsubstituted C_{1~8} alkyl, substituted or unsubstituted C_{1~8} alkoxy, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₃₄ is selected from the group consisting of hydrogen, substituted or unsubstituted C_{1~8} alkyl, substituted or unsubstituted C_{1~8} haloalkyl, substituted or unsubstituted C_{1~8} alkoxy, and substituted or unsubstituted C_{3~8} cycloalkyl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, carboxyl, and mercapto.

18. The azacycloalkane compound or pharmaceutically acceptable salts, solvates, active metabolites, polymorphs, isotopic markers, isomers or prodrugs thereof according to claim 1, wherein the azacycloalkane compound is selected from the following compounds:

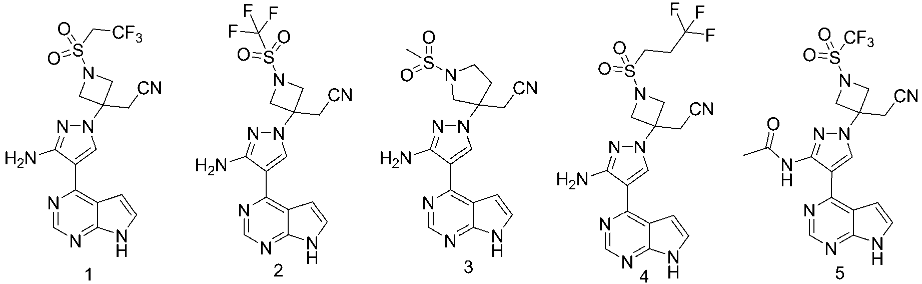

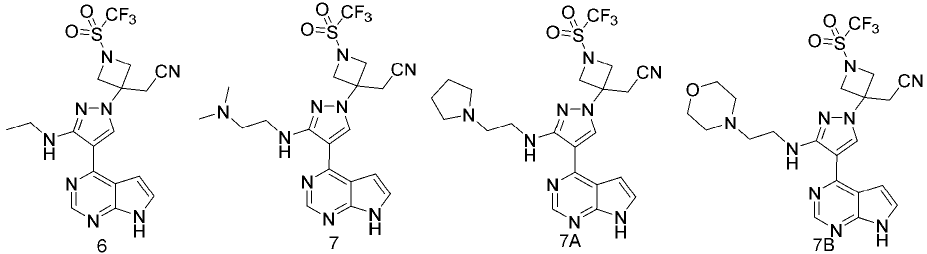

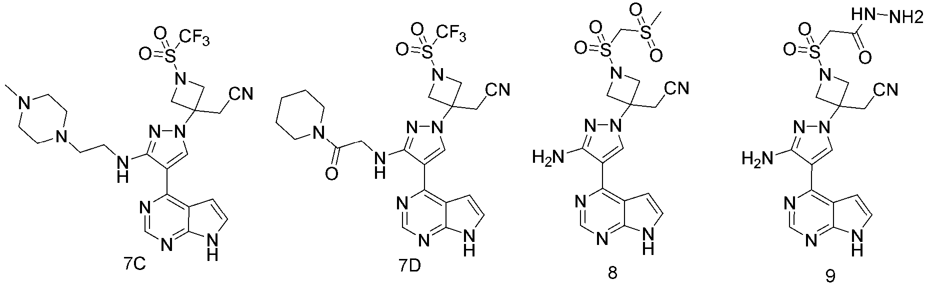

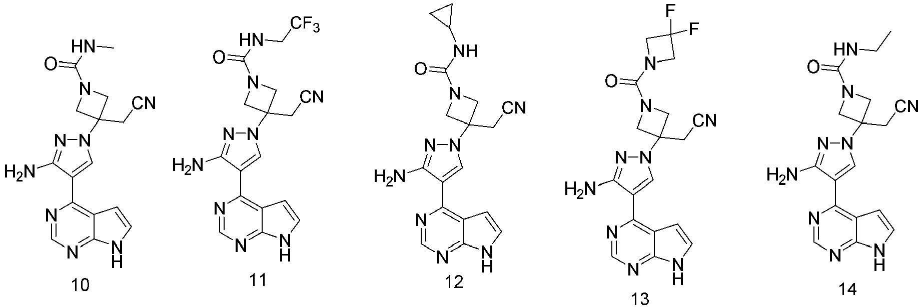

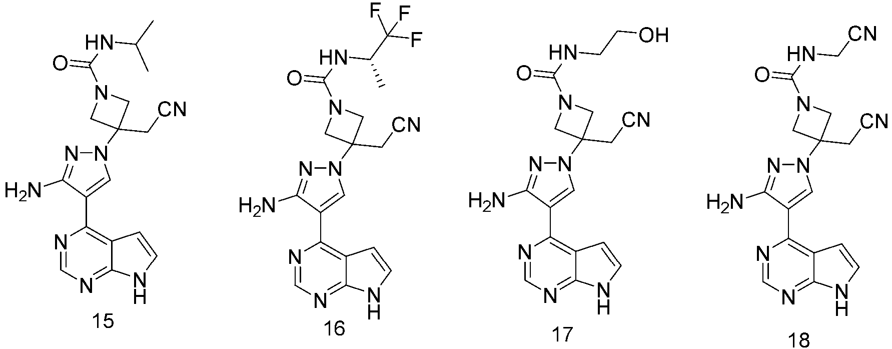

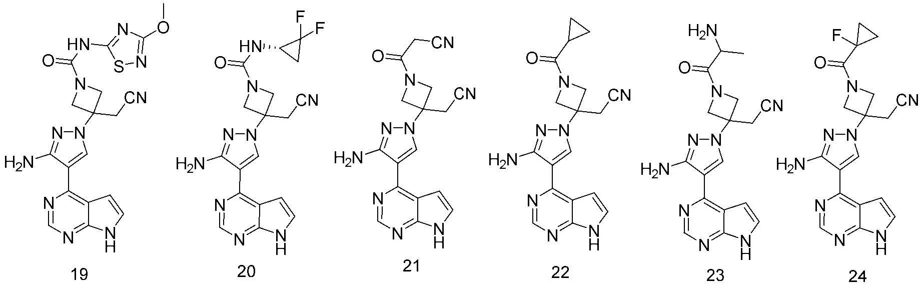

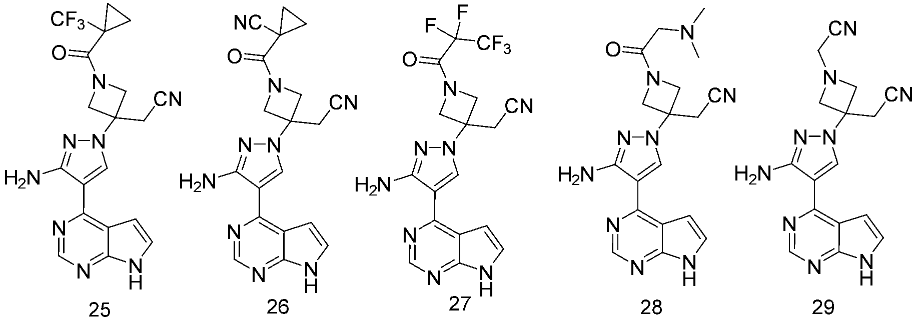

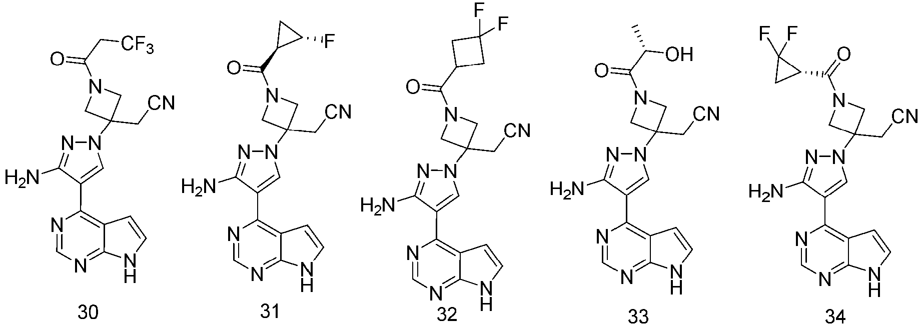

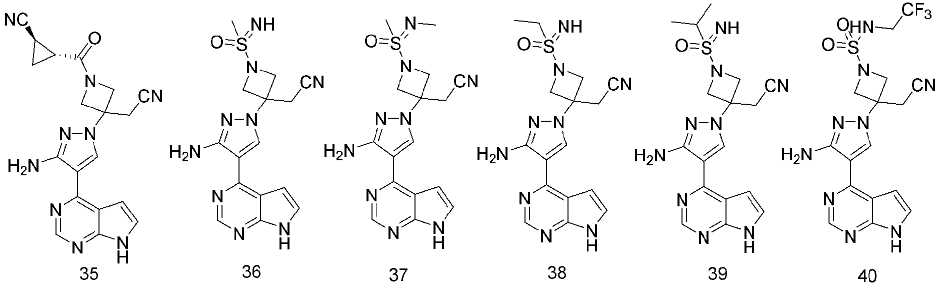

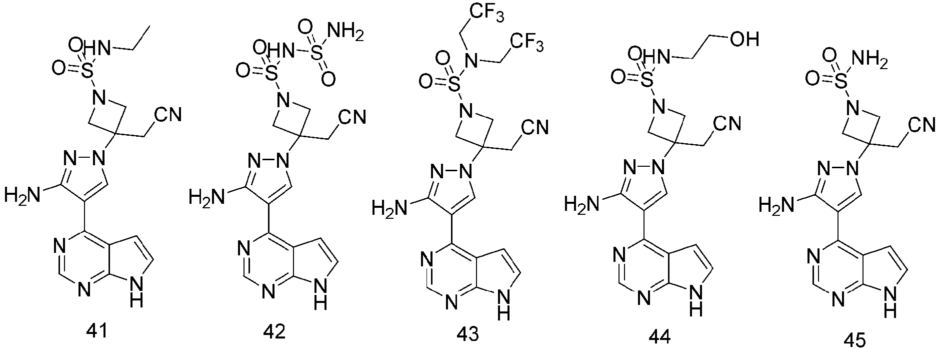

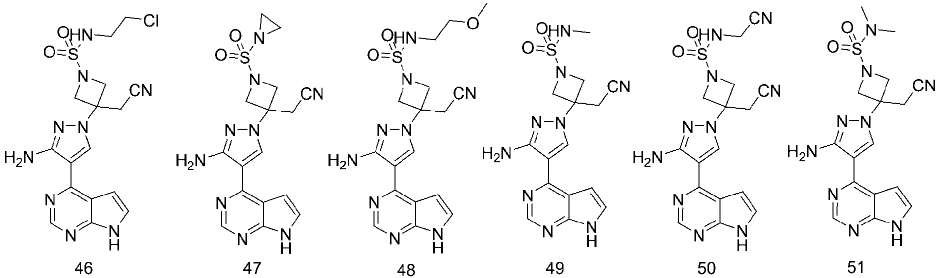

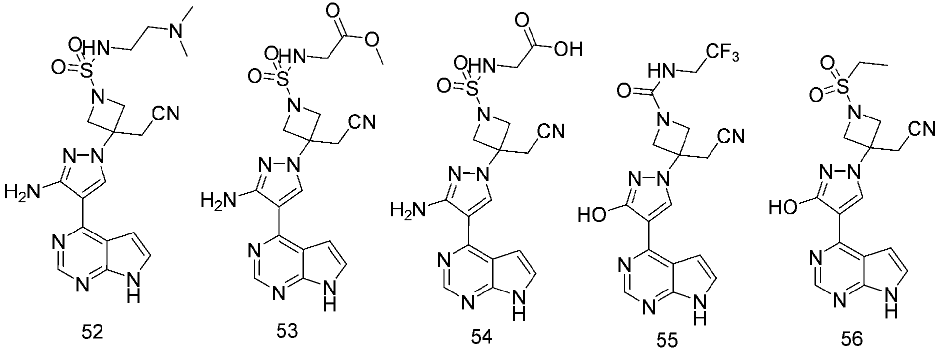

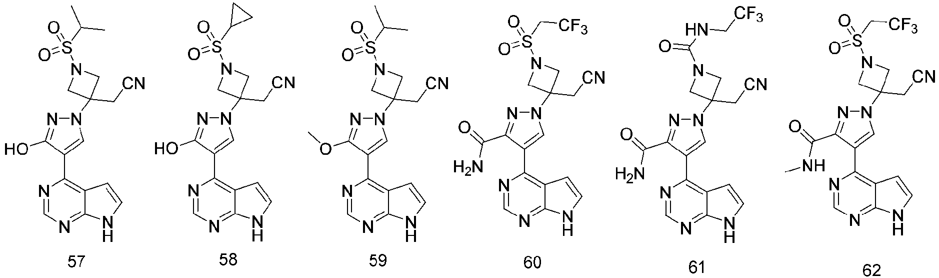

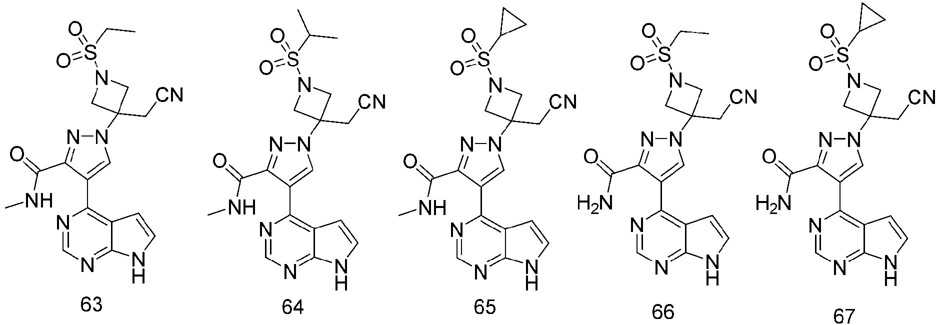

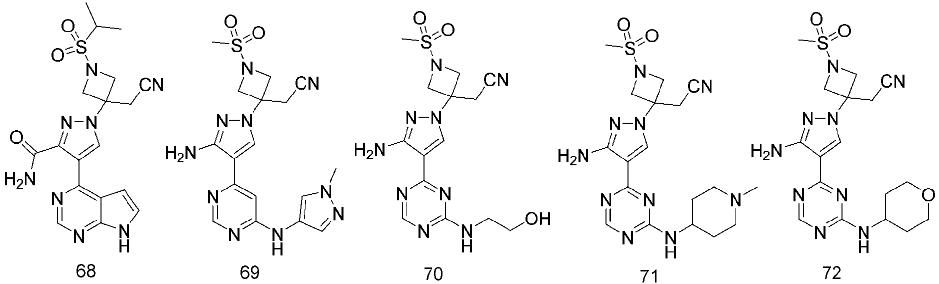

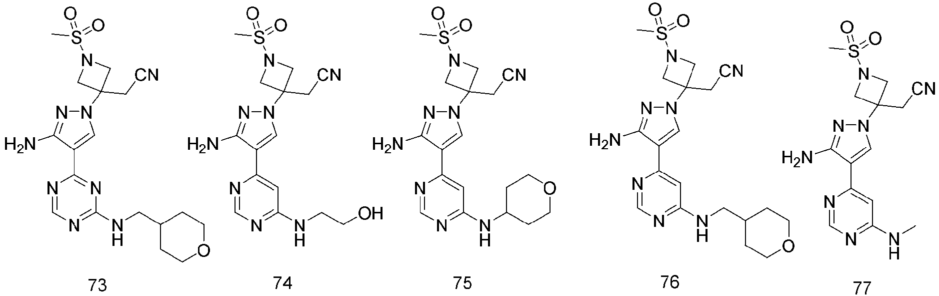

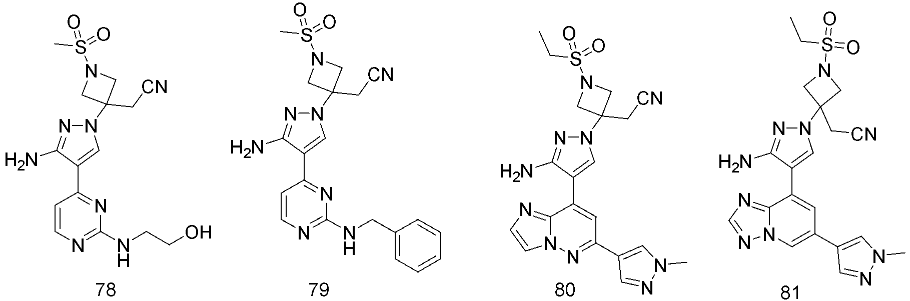

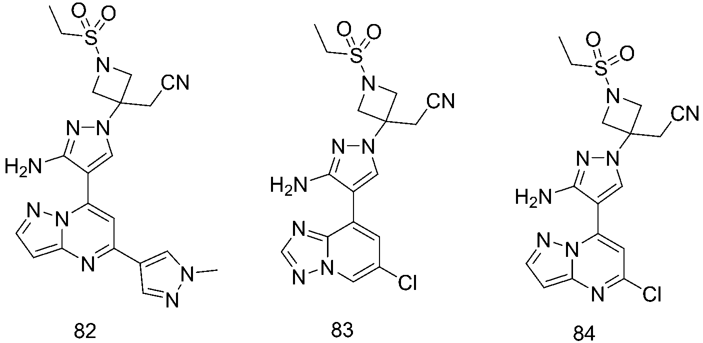

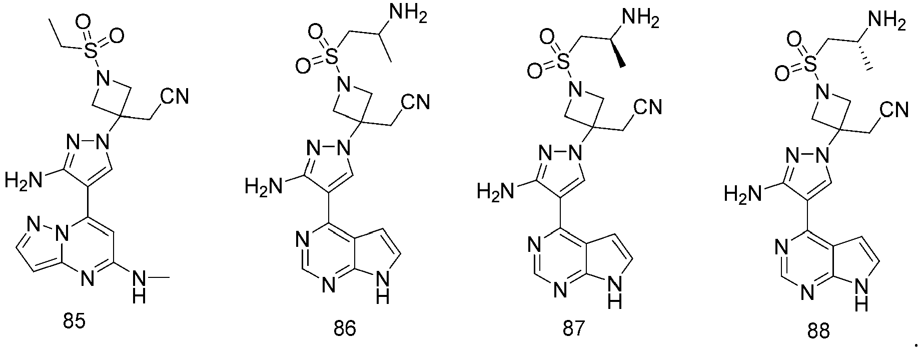

19. The azacycloalkane compound or pharmaceutically acceptable salts, solvates, active metabolites, polymorphs, isotopic markers, isomers or prodrugs thereof according to claim 1, wherein the azacycloalkane compound is selected from the following compounds:

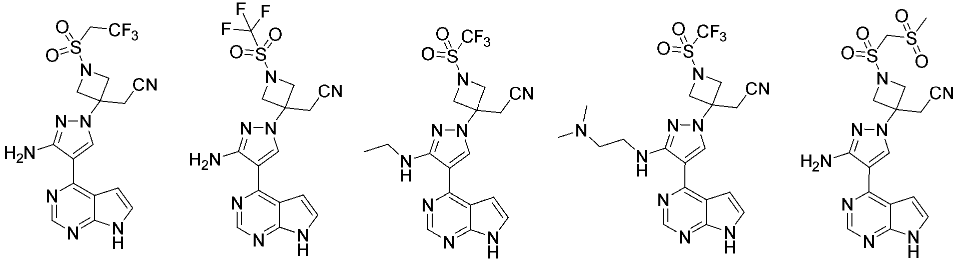

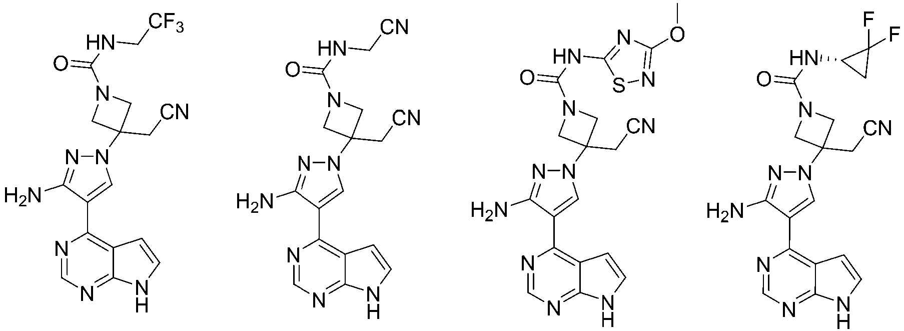

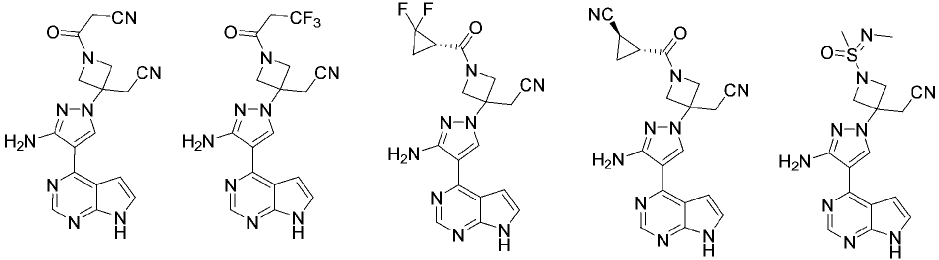

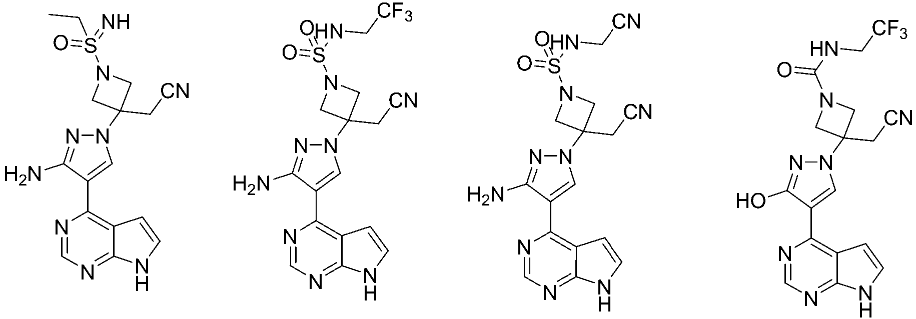

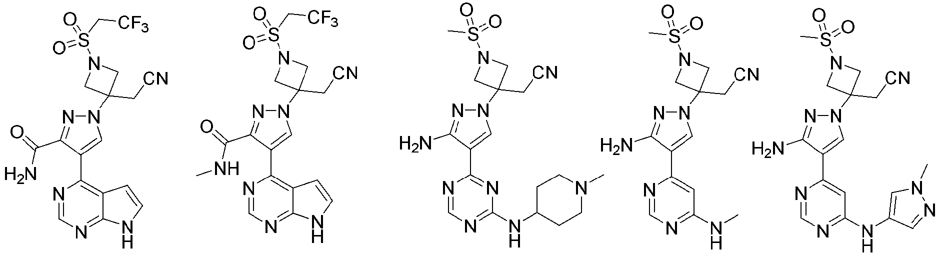

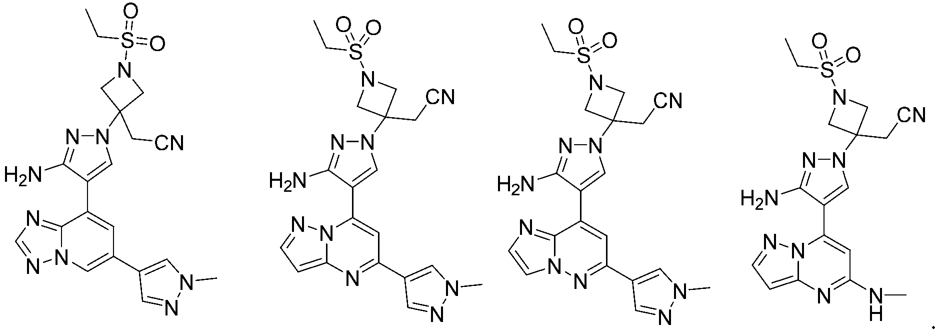

20. A pharmaceutical composition, comprising the azacycloalkane compound or pharmaceutically acceptable salts, solvates, active metabolites, polymorphs, isotopic markers, isomers or prodrugs thereof according to any one of claims 1 to 16, and a pharmaceutically acceptable carrier.

21. Use of the azacycloalkane compound or pharmaceutically acceptable salts, solvates, active metabolites, polymorphs, isotopic markers, isomers or prodrugs thereof according to any one of claims 1 to 19, and the pharmaceutical composition according to claim 20 in manufacture of pharmaceuticals for treating Rho kinase mediated diseases.

22. Use according to claim 21, wherein the Rho kinase mediated diseases are asthma, cancer, glaucoma, insulin resistance, kidney failure, neuronal degeneration, or osteoporosis.

23. A method for treating Rho kinase mediated diseases, comprising administering a therapeutically effective amount of the azacycloalkane compound or pharmaceutically acceptable salts, solvates, active metabolites, polymorphs, isotopic markers, isomers or prodrugs thereof according to any one of claims 1 to 19, or the pharmaceutical composition according to claim 20 to a patient in need of administration.

24. The method according to claim 23, wherein the Rho kinase mediated diseases are asthma, cancer, glaucoma, insulin resistance, kidney failure, neuronal degeneration, or osteoporosis.
